# DEMANDE DE BREVET EUROPEEN

(11) **EP 1 559 405 A1**
(43) Date de publication de la demande: **03.08.2005**
(21) Numéro de dépôt: 05290063.6
(22) Date de dépôt: 11.01.2005
(51) Int. Cl.: A61K 7/13

(54) **Composition de teinture des fibres kératiniques contenant une alcool oxydase et un colorant direct cationique azoique, méthinique ou azométhinique**

(30) Priorité: 28.01.2004 FR 0400776
(71) Demandeur: L'OREAL, 75008 Paris (FR)
(72) Inventeur: Plos, Grégory, Tokyo 158-0097 (JP)
(74) Mandataire: Casalonga, Axel

(57) **Abrégé**

La présente demande concerne une composition pour la teinture des fibres kératiniques, en particulier des fibres kératiniques humaines telles que les cheveux, contenant, dans un milieu approprié pour la teinture, au moins un précurseur de colorant d'oxydation, au moins une enzyme alcool oxydase, au moins un substrat pour ladite enzyme et au moins un colorant direct cationique azoïque, méthinique ou azométhinique. Cette demande concerne encore un procédé de teinture des fibres kératiniques qui consiste à appliquer cette composition ainsi qu'un "kit" de teinture.

## Description

La présente invention a pour objet une composition pour la teinture des fibres kératiniques, en particulier des fibres kératiniques humaines telles que les cheveux, contenant dans un milieu approprié pour la teinture, au moins un précurseur de colorant d'oxydation, au moins une enzyme alcool oxydase, au moins un substrat pour ladite enzyme, ainsi qu'un colorant direct cationique azoïque, méthinique ou azométhinique.

Il est connu de teindre les fibres kératiniques et en particulier les cheveux humains avec des compositions tinctoriales contenant des précurseurs de colorant d'oxydation, appelés généralement bases d'oxydation, tels que des ortho ou paraphénylènediamines, des ortho ou paraaminophénols et des composés hétérocycliques. Ces bases d'oxydation sont des composés incolores ou faiblement colorés qui, associés à des produits oxydants, peuvent donner naissance par un processus de condensation oxydative à des composés colorés.

On sait également que l'on peut faire varier les nuances obtenues avec ces bases d'oxydation en les associant à des coupleurs ou modificateurs de coloration, ces derniers étant choisis notamment parmi les métadiamines aromatiques, les métaaminophénols, les métadiphénols et certains composés hétérocycliques tels que des composés indoliques.

La variété des molécules mises en jeu au niveau des bases d'oxydation et des coupleurs permet l'obtention d'une riche palette de couleurs.

La coloration dite "permanente" obtenue grâce à ces colorants d'oxydation, doit par ailleurs satisfaire un certain nombre d'exigences. Ainsi, elle doit être sans inconvénient sur le plan toxicologique, elle doit permettre d'obtenir des nuances dans l'intensité souhaitée et présenter une bonne tenue face aux agents extérieurs tels que la lumière, les intempéries, le lavage, les ondulations permanentes, la transpiration et les frottements.

Les colorants doivent également permettre de couvrir les cheveux blancs, et être enfin les moins sélectifs possibles, c'est-à-dire permettre d'obtenir des écarts de coloration les plus faibles possibles tout au long d'une même fibre kératinique, qui est en général différemment sensibilisée (c'est-à-dire abîmée) entre sa pointe et sa racine.

La coloration est généralement réalisée en milieu fortement alcalin, en présence de peroxyde d'hydrogène. Toutefois, l'utilisation des milieux alcalins en présence de peroxyde d'hydrogène présente pour inconvénient d'entraîner une dégradation non négligeable des fibres, ainsi qu'une décoloration des fibres kératiniques qui n'est pas toujours souhaitable.

De plus, ce type de composition présente l'inconvénient de réaliser le mélange entre l'eau oxygénée et le support de coloration au moment de l'application de ladite composition sur les fibres kératiniques.

La coloration d'oxydation des fibres kératiniques peut également être réalisée à l'aide de systèmes oxydants différents du peroxyde d'hydrogène tels que des systèmes enzymatiques, en particulier avec des enzymes de type oxydase à 2 électrons. Ainsi la demande de brevet FR 2 769 219 décrit l'utilisation d'une enzyme uricase et de son substrat l'acide urique en coloration d'oxydation pour la teinture de fibres kératiniques. Ces enzymes catalysent l'oxydation d'un substrat par l'oxygénation de l'air pour générer un ou plusieurs produits d'oxydation, ainsi que de l'eau oxygénée. L'eau oxygénée générée peut être utilisée pour oxyder des précurseurs de colorants d'oxydation et par conséquent, produire de la couleur sur le cheveu. Ce système permet d'envisager une coloration d'oxydation sans mélange au moment de l'emploi. Plus récemment, la demande de brevet FR 2 833 492 décrit l'utilisation de l'enzyme alcool oxydase en tant que seule enzyme dans une composition de coloration d'oxydation pour la teinture des fibres kératiniques. Cependant, les formulations de teintures utilisant l'alcool oxydase, bien qu'étant mises en oeuvre dans des conditions n'entraînant pas une dégradation du cheveu comparable à celle engendrée par les formulations utilisant de l'eau oxygénée et bien qu'offrant la possibilité d'être formulées en tout en un, conduisent à des colorations encore insuffisantes à la fois sur le plan de l'homogénéité de la couleur, de la puissance tinctoriale et de la chromaticité.

La demande EP-A-0 310 675 décrit l'utilisation de précurseur de colorant d'oxydation de type benzénique en association avec des enzymes telles que la pyranose-oxydase, la glucose-oxydase. Les compositions décrites dans ce brevet peuvent aussi comprendre des colorants directs. Toutefois, les colorations obtenues en utilisant ces compositions restent encore insuffisantes.

Le but de la présente invention est de fournir de nouvelles compositions pour la teinture des fibres kératiniques par coloration d'oxydation qui respectent la nature de la fibre kératinique, qui offrent la possibilité d'être formulées en tout en un et qui conduisent à des couleurs homogènes, puissantes et de forte chromaticité.

La demanderesse a découvert de nouvelles compositions contenant au moins un précurseur de colorant d'oxydation, au moins une enzyme de type alcool oxydase, au moins un substrat pour ladite enzyme et au moins un colorant direct cationique azoïque, méthinique ou azométhinique. Les compositions selon la présente invention permettent l'obtention de teintures de couleurs chromatiques, puissantes, peu sélectives et résistantes. Ces compositions peuvent donner des nuances significatives variées de couleur intense et uniforme, sans dégradation signifiante du cheveu.

D'autres caractéristiques, aspects, objets et avantages de la présente invention apparaîtront encore plus clairement à la lecture de la description et des exemples qui suivent.

Au sens de la présente invention, on entend :
- par colorant azoïque, une molécule ou un reste de molécule absorbant les radiations lumineuses dans le domaine visible (400-750 nm) et comportant dans sa structure au moins un enchaînement (A) non inclus dans un cycle

   -N=N- (A)
- par colorant méthinique, une molécule ou un reste de molécule absorbant les radiations lumineuses dans le domaine visible (400-750 nm) et comportant dans sa structure au moins un enchaînement (B) non inclus dans un cycle

   -C=C- (B)
- par colorant azométhinique, une molécule ou un reste de molécule absorbant les radiations lumineuses dans le domaine visible (400-750 nm) et comportant dans sa structure au moins un enchaînement (C) non inclus dans un cycle

   -N=C- (C)

Les colorants directs cationiques azoïques, méthiniques ou azométhiniques utilisables pour l'invention sont, de préférence les composés suivants :
- Les composés de formule (I) : 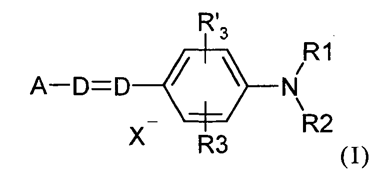 dans laquelle D représente un atome d'azote ou le groupement CH,
   R₁ et R₂, identiques ou différents entre eux, représentent un atome d'hydrogène, un radical alkyle en C₁-C₄ pouvant être substitué par un radical CN, OH, ou NH₂ ou forment avec un atome de carbone du cycle benzènique un hétérocycle azoté pouvant comporter en plus un atome d'oxygène ou un atome d'azote, pouvant être substitué par un ou plusieurs radicaux alkyle en C₁-C₄ ; R₁ et R₂ peuvent former un radical 4'-aminophényle ;
   R₃ et R'₃, identiques ou différents, représentent un atome d'hydrogène ou d'halogène choisi parmi le chlore, le brome, l'iode et le fluor, un radical cyano, alcoxy en C₁-C₄ ou acétyloxy.
   X⁻ représente un anion de préférence choisi parmi le chlorure, le méthylsulfate et l'acétate.
   A représente un groupement choisi par les structures A1 à A19 suivantes : 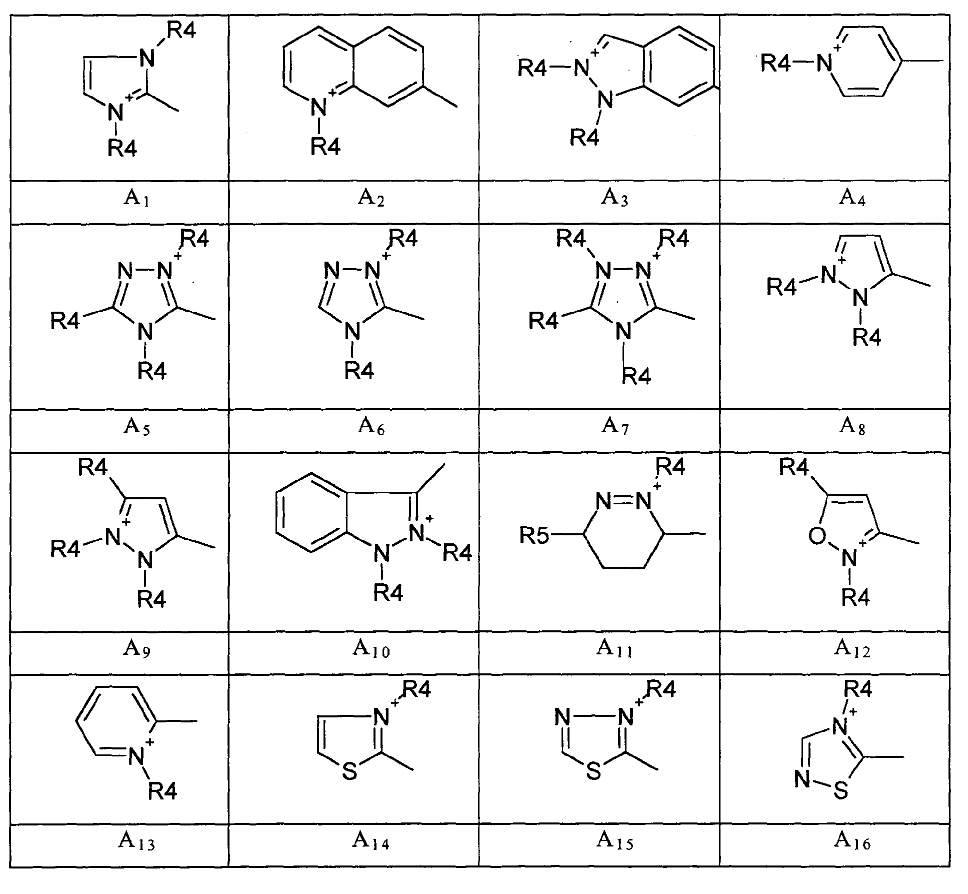 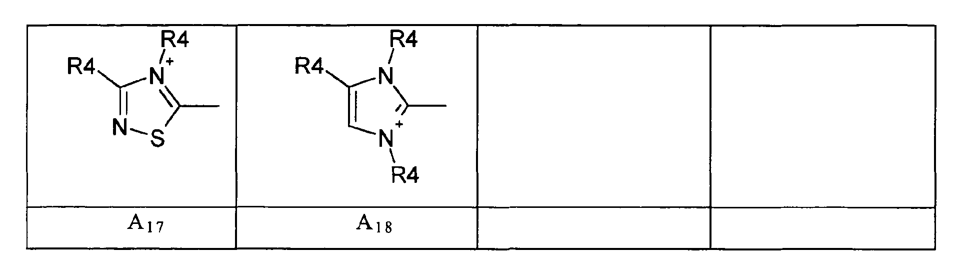
   R₄ représente un radical alkyle en C₁-C₄ pouvant être substitué par un radical hydroxyle et R₅ représente un radical alcoxy en C₁-C₄.
- Les composés de formule (II) : 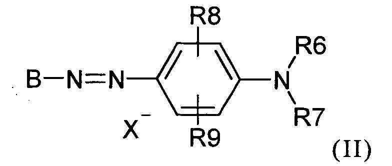 dans laquelle R₆ représente un atome d'hydrogène ou un radical alkyle en C₁-C_{4 ;}
   R₇ représente un atome d'hydrogène, un radicale alkyle pouvant être substitué par un radical CN ou par un groupement amino, un radical 4'-aminophényle ou forme avec R₆ un hétérocycle azoté pouvant comporter en plus un atome d'oxygène et/ou un atome d'azote pouvant être substitué par un radical en C₁-C₄ ;
   R₈ et R₉, identiques ou différents, représentent un atome d'hydrogène, un atome d'halogène tel que le brome, le chlore, l'iode ou le fluor, un radical alkyle en C₁-C₄ ou alcoxy en C₁-C₄, un radical CN ;
   X⁻ représente un anion de préférence choisi parmi le chlorure, le méthylsulfate et l'acétate ;
   B représente un groupement choisi parmi les structures B₁ à B₆ suivantes : 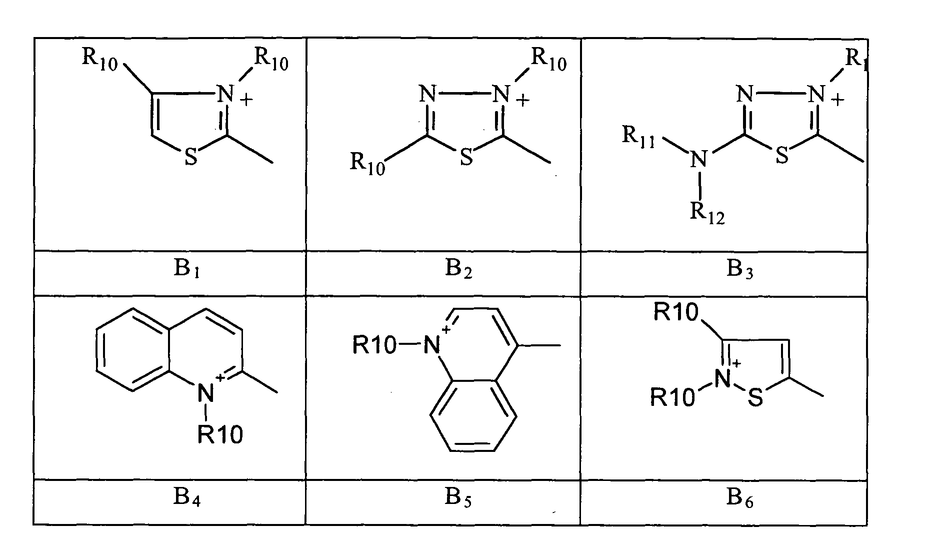 dans lesquelles R₁₀ représente un radical alkyle en C₁-C₄ ;
   R₁₁ et R₁₂, identiques ou différents, représentent un atome d'hydrogène ou un radical alkyle en C₁-C₄.
- Les composés de formule (III) et (III') : 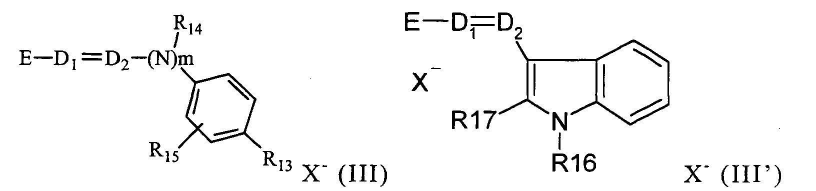 dans lesquelles, R₁₃ représente un atome d'hydrogène, un radical alcoxy en C₁-C₄, un atome d'halogène tel que le brome, le chlore, l'iode ou le fluor ou un radical amino ;
   R₁₄ représente un atome d'hydrogène, un radical alkyle en C₁-C₄ ou forme avec un atome de carbone du cycle benzènique un hétérocycle éventuellement oxygéné et/ou azoté substitué par un ou plusieurs groupements en C₁-C₄ ;
   R₁₅ représente un atome d'hydrogène ou d'halogène tel que le brome, le chlore, l'iode ou le fluor ;
   R₁₆ et R₁₇, identiques ou différents, représentent un atome d'hydrogène ou un radical alkyle en C₁-C₄ ;
   D₁ et D₂, identiques ou différents, représentent un atome d'azote ou le groupement CH ;
   m est égal à 0 ou 1 ;
      étant entendu que lorsque R₁₃ représente un groupement amino non substitué alors D₁ et D₂ représentent simultanément un groupement -CH et m vaut 0 ;
   X- représente un anion de préférence choisi parmi le chlorure, le méthyl sulfate et l'acétate ;
   E représente un groupement choisi parmi les structures E₁ à E₈ suivantes : 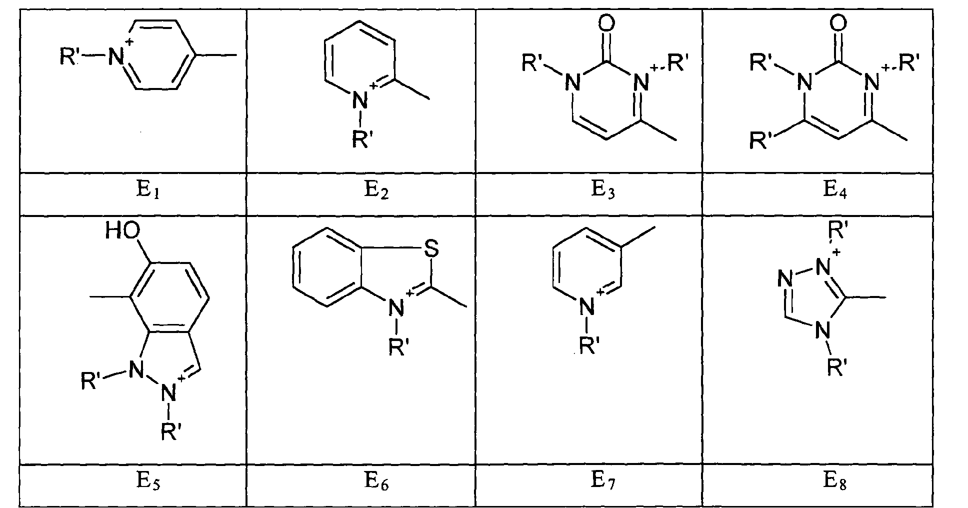 dans lesquelles R' représente un radical alkyle en C₁-C₄.
      Lorsque m vaut 0 et que D₁ représente un atome d'azote, alors E peut également désigner un groupement de structure E₉ suivante : 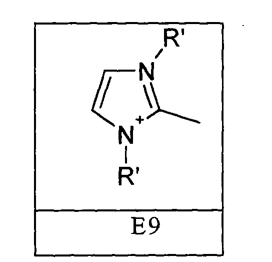 dans laquelle R' représente un radical alkyle en C₁-C₄.
      Les composés de formule (I), (II), (III), (III') sont notamment décrits dans la demande FR 2 807 650, dont le contenu fait intégralement partie de l'invention.
- Les composés de formule (IV) :

   W₁-W₂-N=N-W₃-L-W₄-N=N-W₅-W₆ (IV)

   Dans laquelle W₁ et W₆ représentent, indépendamment l'un de l'autre, un radical NR'₁R'₂ ;
   W₂ et W₅ représentent indépendamment l'un de l'autre, un groupement aromatique carboné, pyridinique ou pyridazinyle de formule (IVa) : 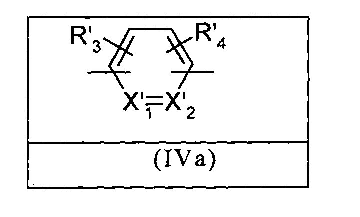
   W₃ et W₄ représentent, indépendamment l'un de l'autre, un radical hétéroaromatique représenté par les formules (IVb) et (IVc) suivantes : 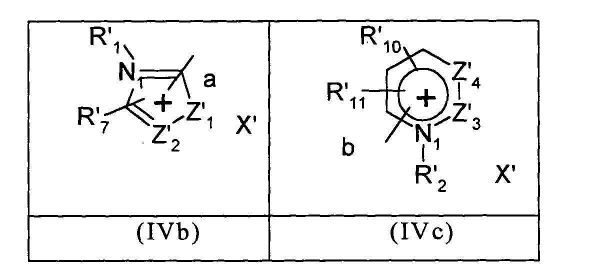 dans lesquelles X'₁ représente un atome d'azote ou un radical CR'₅
   X'₂ représente un atome d'azote ou un radical CR'₆
   Z'₁ représente un atome d'oxygène, de soufre ou un radical NR'₈
   Z'₂ représente un atome d'azote ou un radical CR'₉
   Z'₃ représente un atome d'azote ou un radical CR'₁₂
   Z'₄ représente un atome d'azote ou un radical CR'₁₃
   N₁ du cycle à 5 chaînons de la formule (IVb) est relié au groupement L de la formule (IV) via le groupe R'₁ et la liaison *a* du même cycle à 5 chaînons est reliée au groupement azoïque de la formule (IV) ;
      la liaison *b* du cycle à 6 chaînons de la formule (IVc) est reliée au groupement azoïque de la formule (IV) et N₁ du cycle à 6 chaînons de la formule (IVc) est reliée au groupe L de la formule (IV) via le groupe R'₂ ;
   L, R'₁, R'₂, R'₃, R'₄, R'₅, R'₆, R'₇, R'₉, R'₁₀, R'₁₁, R'₁₂ et R'₁₃ représentent ensemble ou indépendamment les uns des autres une chaîne hydrocarbonée en C₁-C₁₆ linéaire ou ramifiée pouvant former un ou plusieurs cycles carbonés comportant de 3 à 6 chaînons et pouvant être saturés ou insaturés, dont un ou plusieurs carbones de la chaîne carbonée peuvent être remplacés par un atome d'oxygène, d'azote, de soufre ou par un groupement SO₂ et dont les atomes de carbone peuvent être indépendamment les uns des autres substitués par un ou plusieurs atomes d'halogène ;
   R'₁, R'₂, R'₃, R'₄, R'₅, R'₆, R'₇, R'₉, R'₁₀, R'₁₁, R'₁₂ et R'₁₃ peuvent représenter l'atome d'hydrogène. L, R'₁, R'₂, R'₃, R'₄, R'₅, R'₆, R'₇, R'₉, R'₁₀, R'₁₁, R'₁₂ et R'₁₃ ne comportent pas de liaison peroxyde, ni de radicaux diazo ou nitroso et L est un radical divalent ;
   R'₈ représente un radical alkyle en C₁-C₈ linéaire ou ramifié, éventuellement substitué par un ou plusieurs radicaux choisis parmi les radicaux hydroxy, alcoxy en C₁-C₂, (poly)-hydroxyalcoxy en C₂-C₄, amino, (di)alkylamino en C₁-C₂, carboxy ou sulfonique, un radical phényle éventuellement substitué ;
   R'₇ avec R'₉, R'₁₀ avec R'₁₁, R'₁₂ avec R'₁₃ peuvent former un cycle aromatique carboné tel qu'un phényle ;
   X' représente un anion organique ou minéral.
   Les composés de formule (IV) sont notamment décrits dans la demande FR 2 822 696, dont le contenu fait intégralement partie de l'invention.
- Les composés de formule (V) :

   W₇-N=N-W₈-N=N-W₉ (V)

   dans laquelle :
   W₇ et W₉ représentent indépendamment l'un de l'autre un radical hétéroaromatique représenté par les formules Va et Vb suivantes : 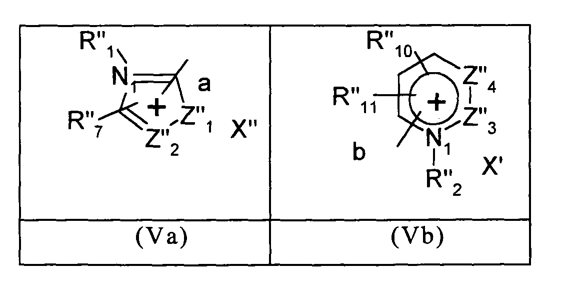
   W₈ représente un groupement aromatique carboné pyridinique ou pyridazinyle de formule (Vc) : 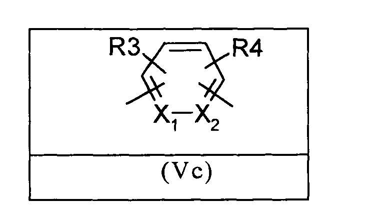 dans lesquelles :
      X₁ représente un atome d'azote ou un radical CR''₅
      X₂ représente un atome d'azote ou un radical CR''₆
      Z''₁ représente un atome d'oxygène, de soufre ou un radical NR''₈
      Z''₂ représente un atome d'azote ou un radical CR''₉,
      Z''₃ représente un atome d'azote ou un radical CR''₁₂
      Z''₄ représente un atome d'azote ou un radical CR''₁₃
      la liaison a du cycle cationique à 5 chaînons de la formule (Va) est reliée au groupement azoïque de la formule (V) ;
      la liaison b du cycle cationique à 6 chaînons de la formule (Vb) est relié au groupement azoïque de la formule (V) ;
   R₃, R₄, R''₁, R''₂, R''₅, R''₆, R''₇, R''₈, R''₉, R''₁₀, R''₁₁, R''₁₂, R''₁₃ représentent, ensemble ou indépendamment l'un de l'autre, un atome d'hydrogène, une chaîne hydrocarbonée en C₁-C₁₆, linéaire ou ramifiée, saturée ou insaturée, pouvant former un ou plusieurs cycles carbonés comportant de 3 à 6 chaînons, dont un ou plusieurs atomes de carbone de la chaîne carbonée peuvent être remplacés par un atome d'oxygène, d'azote ou de soufre ou par un groupement SO₂ et dont les atomes de carbone peuvent être indépendamment les uns des autres substitués par un ou plusieurs atomes d'halogène ;
   R₃, R₄, R''₁, R''₂, R''₅, R''₆, R''₇, R''₈, R''₉, R''₁₀, R''₁₁, R''₁₂, R''₁₃ ne comportent pas de liaison peroxyde, ni de radicaux diazo ou nitroso ;
   R''₇ et R''₉, R''₁₀ et R''₁₁, R''₁₂ et R''₁₃ peuvent former un cycle carboné tel un phényle ;
   X" est un anion organique ou minéral par exemple choisi parmi un halogénure, un hydroxyde, un sulfate, un hydrogénosulfate, un alkylC₁-C₆sulfate, un acétate, un tartrate, un oxalate, un alkylC₁-C₆sulfonate, un arylsulfonate substitué ou non substitué par un radical alkyl en C₁-C₄. Les composés de formule (V) sont notamment décrits dans la demande FR 2 825 702, dont le contenu fait intégralement partie de l'invention.
- Les composés de formule (VIa) et (VIb) : 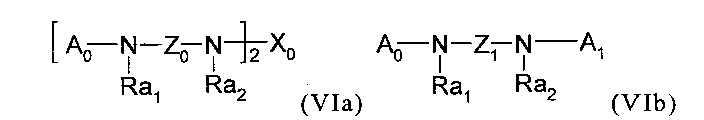 où A₀ et A₁, indépendamment l'un de l'autre désignent : 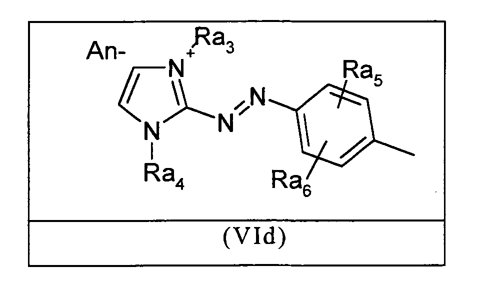
   Z₀ désigne un radical aliphatique
   Z₁ désigne un radical alkyle
   Rₐ₁ et Rₐ₂, indépendamment l'un de l'autre désignent un atome d'hydrogène ou un radical (C₁-C₄)alkyl ou (C₁-C₄)alkyl substitué par un ou plusieurs atomes d'halogène, un radical hydroxyl, carboxyl, cyano, un radical (C₁-C₄)alcoxy, un radical (C₁-C₄)alcoxy substitué par un ou plusieurs radicaux hydroxyl, ou (C₁-C₄)alcoxy, un radical amino, alkylamino, dialkylamino, aminocarbonyl, phényl, phénoxy, ou phénylaminocarbonyl dans lequel le radical phényl est non substitué ou substitué par un radical (C₁-C₄)alkyl, (C₁-C₄)alcoxy ou phénoxy ;
   Rₐ₁ et Rₐ₂ peuvent aussi former ensemble avec les deux atomes d'azote qui les portent et le radical Z₀ un radical pipérazinique ;
   X₀ est un radical de pontage choisi parmi -CO-, -COCH₂-, -CH₂CO-, -COCO-, 1,4-dicarbonylphényl, -CH₂-CH₂-, ou une triazine de formules suivantes : 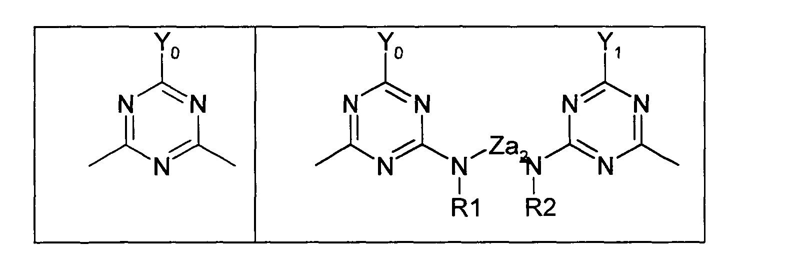 Dans lesquelles Y₀ et Y₁, indépendamment l'un de l'autre, désignent un atome d'halogène ou un radical hydroxyl, ou amino, ou monoalkylamino, ou dialkylamino, ou 1-pipéridino, ou morpholino, ou 1-pipérazino, le radical pipérazino étant non substitué ou substitué sur l'atome d'azote non attaché au cycle triazine par un radical (C₁-C₄)alkyl, lesdits radicaux alkyls étant non substitués ou substitués par hydroxyl, amino, mono-(C₁-C₄)alkylamino ou di(C₁-C₄)alkylamino ;
   Zₐ₂ désigne un radical C₂-C₈alkylène ou forme avec les deux atomes d'azote adjacents et les radicaux R₁ et R₂ un cycle pipérazinique ;
   Dans le radical de formule (VId) :
   Rₐ₃ et Rₐ₄, indépendamment l'un de l'autre, désignent un atome d'hydrogène, ou un radical (C₁-C₄)alkyl ou C₁-C₄alkyl substitué par un ou plusieurs atomes d'halogène, un radical hydroxyl, carboxyl, cyano, un radical (C₁-C₄)alcoxy, un radical (C₁-C₄)alcoxy substitué par un radical hydroxyl ou (C₁-C₄)alcoxy, un radical amino, alkylamino, dialkylamino, aminocarbonyl, phényle, phénoxy ou phénylaminocarbonyl dans lequel le radical phényl est non substitué ou substitué par un radical (C₁-C₄)alkyl, (C₁-C₄)alcoxy ou phénoxy ;
   Rₐ₅ et Rₐ₆, indépendamment l'un de l'autre, désignent un atome d'hydrogène, un radical C₁-C₄alkyl ou C₁-C₄alcoxy, éventuellement substitués par un radical hydroxyl, carboxyl, halogène, cyano, (C₁-C₄)alcoxy éventuellement substitué par un radical hydroxyl, ou (C₁-C₄)alcoxy, un radical amino, alkylamino, dialkylamino, aminocarbonyl, phényl, phénoxy, ou phénylaminocarbonyl, dans lequel le radical phényl est non substitué ou substitué par un radical (C₁-C₄)alkyl, (C₁-C₄)alcoxy ou phénoxy ;
   An⁻ désigne un anion.
- Les composés de formule (VIc) : 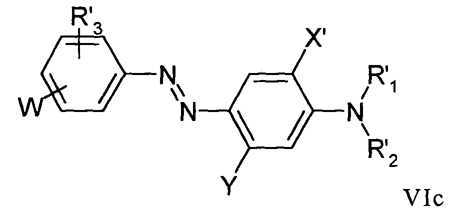 dans laquelle le nombre de charge cationique est de 2 ;
   X'et Y, indépendamment l'un de l'autre, désignent un atome d'hydrogène, halogène, (C₁-C₄)alkyl, (C₁-C₄)alcoxy, (C₁-C₄)alkylcarbonylamino, arylcarbonylamino, uréido, ou aryluréido ;
   R'₁ désigne un atome d'hydrogène, un radical alkyl ou aryl substitués, un radical alkyl ou aryl non substitués ou la même désignation que R'₂ ;
   R'₂ est un radical du type : 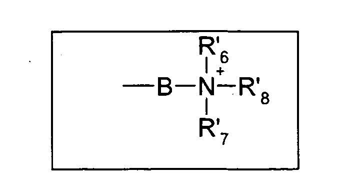 Dans laquelle B désigne un radical alkylène linéaire ou ramifié ;
   R'₆ désigne un atome d'hydrogène ou alkyl substitué ou non substitué ;
   R'₇ et R'₈, indépendamment l'un de l'autre désignent un radical alkyl substitué ou non subtitué ;
   R'₆ et R'₇ ensemble avec l'azote, forment un cycle à 5, 6 ou 7 chaînons substitués ou non substitués, pouvant contenir d'autres hétéroatomes ou bien R'₆ et R'₇ et R'₈ forment ensemble un cycle pyridinium ;
   R'₃ désigne un atome d'hydrogène, d'halogène (C₁-C₄)alkyl, (C₁-C₄)alcoxy ;
   W est un radical de formule : 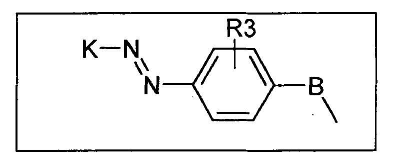 où K désigne un composé de couplage choisi parmi ceux de formule (f), (g) ou (h) suivantes : 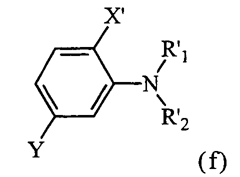 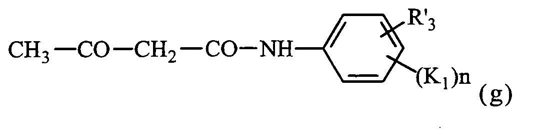 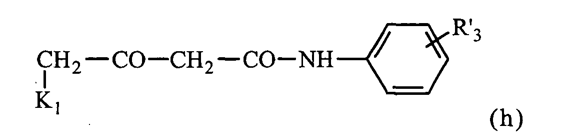 dans lesquelles,
      - X' et Y, indépendamment l'un de l'autre, désignent un atome d'hydrogène, un atome d'halogène, un groupe alkyl en C₁-C₄, un alcoxy en C₁-C₄, un alkyl(C₁-C₄)carbonylamino, un arylcarbonylamino, un uréido ou un aryluréido,
      - R'₁ désigne un atome d'hydrogène, un radical alkyl substitué, un radical aryl substitué, un radical alkyl non substitué, un radical aryl non substitué, ou la même signification que le substituant R'₂,
      - R'₂ est un radical de formule suivante : 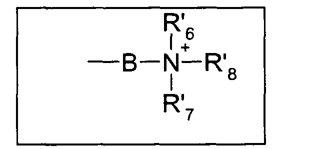 dans laquelle B désigne un radical alkylène linéaire ou ramifié ;
         R'₆ désigne un atome d'hydrogène ou alkyl substitué ou non substitué ;
         R'₇ et R'₈, indépendamment l'un de l'autre désignent un radical alkyl substitué ou non substitué ;
         R'₆ et R'₇ ensemble avec l'azote, forment un cycle à 5, 6 ou 7 chaînons substitués ou non substitués, pouvant contenir d'autres hétéroatomes ou bien R'₆ et R'₇ et R'₈ forment ensemble un cycle pyridinium ;
         R'₃ désigne un atome d'hydrogène, d'halogène, (C₁-C₄)alkyl, (C₁-C₄)alcoxy ;
            - n est égal à 1 ou 2,
            - K₁ désigne le radical de formule -N⁺R'₆R'₇R'₈ ;
         R3 a les mêmes significations que Rₐ₃.
         B désigne un radical de pontage choisi parmi les radicaux suivants :

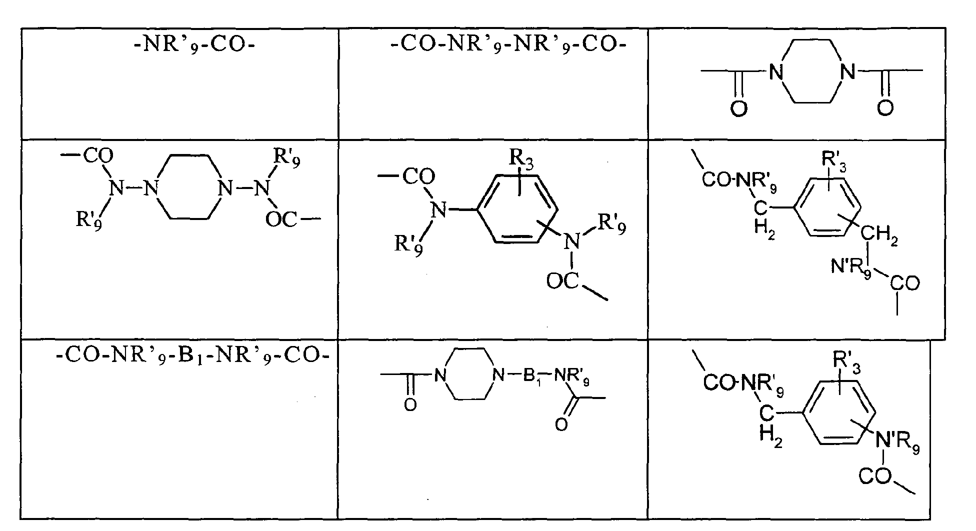

         Dans lesquelles R'₉ désigne un atome d'hydrogène, ou un radical alkylène en C₁-C₂ ; B₁ désigne un radical (C₂-C₄)alkylène non substitué ou substitué, le radical alkylène étant linéaire ou ramifié et pouvant être interrompu par un ou plusieurs groupements choisis parmi -NR'₉-, -O-, -S-.
         Les composés de formule (VIa), (VIb) et (VIc) sont notamment décrits dans la demande FR 2 825 625, dont le contenu fait intégralement partie de l'invention.
- Les composés de formule (VIIIa) :

   Z₁-N=N-A₁-(A₃)ₙ-Z₂ (VIIIa) ou Z₁-N=N-A₂ (VIIIb)

   Dans lesquelles n est égal à 0 ou 1.
   Z₁ représente un radical hétéroaromatique cationique à 5 ou 6 chaînons de formules suivantes : 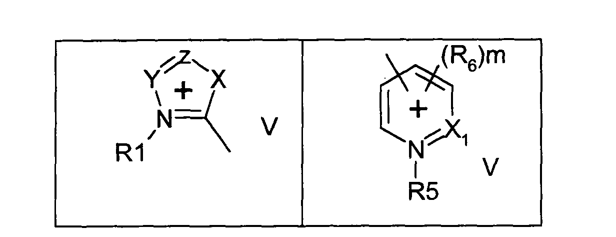 où X représente NR₃, S, ou O, Z représente CR₂ ou N et Y représente CR₄ ou N avec les conditions suivantes :
- lorsque X représente NR₃ ou O et Z représente CR₂ alors Y représente CR₄ ou N.
- lorsque X représente S alors Z représente N ou Y représente N.
- lorsque X représente S et Z représente N alors Y représente CR₄.
   X₁ représente CR₆ ou N.
   m est un nombre entier égal à 0, 1, 2 ou 3.
   R₁, R₃, R₅ représentent, indépendamment l'un de l'autre, une chaîne hydrocarbonée en C₁-C₁₀, saturée ou insaturée, linéaire ou ramifiée pouvant former un cycle carboné ayant de 5 à 7 chaînons éventuellement aromatique, un ou plusieurs atomes de carbone pouvant être remplacée par un atome d'oxygène, d'azote, d'halogène de soufre ou par un groupement SO₂, à l'exception du carbone relié à l'atome d'azote des 2 cycles précédents. Les radicaux R₁ et R₃ ou R₅ ne comportant pas de liaison peroxyde, ni de radicaux diazo, nitro ou nitroso.
   R₂, R₄ et R₆ représentent, indépendamment l'un de l'autre, un atome d'hydrogène, une chaîne hydrocarbonée en C₁ -C₁₀, saturée ou insaturée, linéaire ou ramifiée pouvant former un cycle carboné ayant de 5 à 7 chaînons, éventuellement aromatique ; un ou plusieurs atomes de carbone pouvant être remplacés par un ou plusieurs atomes d'oxygène, d'azote, de soufre, ou par un groupement SO₂, les radicaux R₂, R₄ ou R₆ ne comportant pas de liaison peroxyde, ni de radicaux diazo, nitro ou nitroso, les radicaux R₂ et R₄ peuvent former ensemble un cycle aromatique carboné.
   V représente un anion organique ou minéral.
   A₁ et A₃ représentent, indépendamment l'un de l'autre, un radical divalent de formule (VIIIc) ou (VIIId) 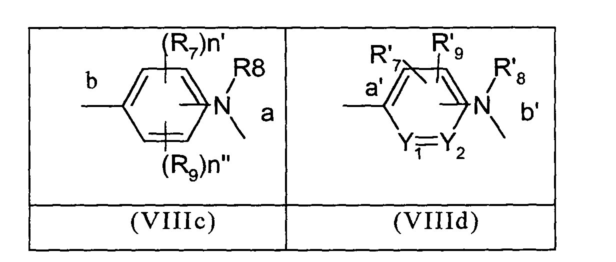
   n' est un nombre entier égal à 0, 1, 2 ou 3.
   n" est un nombre entier égal à 0 ou 1.
   Y₁=Y₂ représente C=N ou N=N ;
      lorsque n vaut 0, alors soit la liaison a du groupement A₁ de la formule (VIIIc) est reliée à la fonction Z₂ de la formule (VIIIa), soit la liaison b' du groupement A₁ de la formule (VIIId) est reliée à la fonction Z₂ de la formule (VIIIa)
      lorsque n vaut 1, les composés de formule (VIIIa) correspondent aux quatre sous structures :

      Z₁-N=N-(VIIIc)-(VIIId)-Z₂

      Z₁-N=N-(VIIIc)-(VIIIc)-Z₂

      Z₁-N=N-(VIIId)-(VIIIc)-Z₂

      Z₁-N=N-(VIIId)-(VIIId)-Z₂

      Les radicaux divalents (VIIIc) et (VIIId) étant reliés entre eux ou à Z₂ ou à l'atome d'azote du groupement azoïque via les bras de liaison a, a', b, b'.
   R₈ et R'₈ représentent indépendamment l'un de l'autre un groupe non cationique choisi parmi un atome hydrogène, une chaîne hydrocarbonée en C₁-C₁₀, linéaire ou ramifiée pouvant former un cycle carboné ayant de 5 à 7 chaînons, éventuellement aromatique, un ou plusieurs atomes de carbone de la chaîne hydrocarbonée pouvant être remplacés par un ou plusieurs atomes d'oxygène, d'azote, de soufre, ou par un groupement SO₂ à l'exception du carbone relié à l'atome d'azote. Les radicaux R₈ ou R'₈ ne comportant pas de liaison peroxyde, ni de radicaux diazo, nitro ou nitroso ;
   R₇, R₉, R'₇ et R'₉ représentent indépendamment l'un de l'autre un groupe non cationique tel que défini pour R₂ ou un groupe cationique Z₃, à la condition qu'un seul des groupes R₇, R₉, R'₇ et R'₉ soit cationique ;
   R₇ avec R₈, respectivement R'₇ avec R_{'8} peuvent former ensemble un hétérocycle à 5 ou 6 chaînons saturé ;
   Z₃ est un groupe cationique représenté par la formule (VIIIe) suivante :

      -(B)_{n'''}-D (VIIIe)

      dans laquelle :
   B représente une chaîne hydrocarbonée comportant de 1 à 15 atomes de carbone, linéaire ou ramifiée, pouvant former un ou plusieurs cycles comportant de 3 à 7 chaînons éventuellement aromatiques, et dont un ou plusieurs atomes de carbone peuvent être remplacés par un atome d'oxygène, d'azote, de soufre, ou par un radical SO₂, à l'exception du carbone relié à l'atome d'azote. B ne comportant pas de liaison peroxyde ni de radicaux diazo, nitro ou nitroso ;
      le radical B est relié à D par l'un quelconque des atomes du radical D ;
   n"' peut prendre la valeur 0 ou 1 ;
   D est choisi parmi les groupes cationiques de formules (VIIIf) et (VIIIg) suivantes : 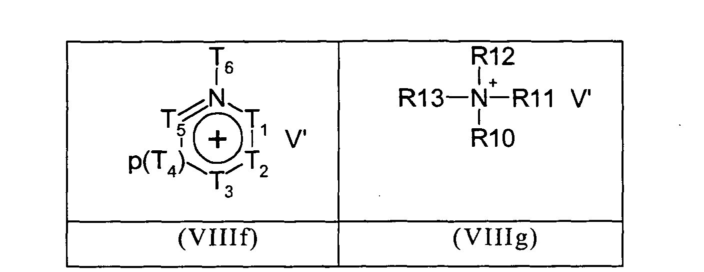 dans lesquelles :
   p peut prendre la valeur 0 ou 1 ;
   T₁, T₂, T₃ et T₄, indépendamment les uns des autres, représentent un atome d'oxygène, un atome de soufre, un atome d'azote non substitué ou substitué par un radical R₁₄, ou un atome de carbone non substitué ou substitué par un ou deux radicaux R₁₄, identiques ou différents ;
   T₅ représente un atome d'azote ou un atome de carbone non substitué ou substitué par un radical R₁₄ ;
   T₆ peut prendre les mêmes significations que celles indiquées ci-dessous pour le radical R₁₄, étant entendu que T₆ est différent d'un atome d'hydrogène ;
   T₁ ou T₅ peuvent, en outre, former avec T₆ un cycle saturé ou insaturé comportant de 5 à 7 chaînons, chaque chaînon étant non substitué ou substitué par un ou deux radicaux R₁₄ identiques ou différents ;
      deux des radicaux adjacents T₁, T₂, T₃, T₄ et T₅ peuvent en outre former un cycle comportant de 5 à 7 chaînons, chaque chaînon étant indépendamment représenté par un atome de carbone non substitué ou substitué par un ou deux radicaux R14 identiques ou différents, un atome d'azote substitué ou non substitué par un radical R₁₄, un atome d'oxygène ou un atome de soufre ;
   R₁₀, R₁₁, R₁₂, R₁₃ et R₁₄, identiques ou différents, représentent un atome d'hydrogène, une chaîne hydrocarbonée comportant de 1 à 10 atomes de carbone, linéaire ou ramifiée, éventuellement aromatiques, et dont un ou plusieurs atomes de carbone peuvent être remplacés par un atome d'oxygène, d'azote, de soufre, ou par un groupe SO₂, et dont un ou plusieurs atomes de carbone peuvent, indépendamment les uns des autres, être substitués par un ou plusieurs atomes d'halogène. Ledit radical ne comportant pas de liaison peroxyde ni de radicaux diazo, nitro ou nitroso ;
   R₁₀, R₁₁ et R₁₂ peuvent également former, deux à deux avec l'atome d'azote quaternaire auquel ils sont rattachés, un ou plusieurs cycles saturés comportant de 5 à 7 chaînons, chaque chaînon étant indépendamment représenté par un atome de carbone non substitué ou substitué par un ou deux radicaux R₁₄ identiques ou différents, un atome d'azote non substitué ou substitué par un radical R₁₄, un atome d'oxygène ,ou un atome de soufre ;
      lorsque n"' vaut 0, alors le groupement de formule (VIIIg) peut être relié au composé de formule (VIIIc) et (VIIId) directement par l'atome d'azote de l'ammonium quaternaire, R₁₃ représentant dans ce cas une simple liaison ;
   V' représente un anion organique ou minéral ;
   Z₂ représente une chaîne hydrocarbonée en C₁-C₁₀, linéaire ou ramifiée pouvant former un cycle carboné ayant de 5 à 7 chaînons, éventuellement aromatique, un ou plusieurs atomes de carbone pouvant être remplacés par un ou plusieurs atomes d'oxygène, d'azote, de soufre ou par un groupement SO₂ ; ledit radical Z₂ ne comportant pas de liaison peroxyde, ni de radicaux diazo, nitro ou nitroso, un groupe cationique Z₃ tel que défini ci-dessus, avec la réserve que Z₂ n'est pas cationique lorsque R₇, R₉, R_{7'} ou R_{9'} est cationique ;
   A₂ représente un radical de formule (VIIIh) correspondant à un radical aromatique carboné, pyridinique ou pyridazinique substitué par un radical hétéroaromatique cationique à 5 chaînons, éventuellement substitué par un ou plusieurs radicaux R₁₉ de même définition que R₂, un radical de formule (VIIIi) : 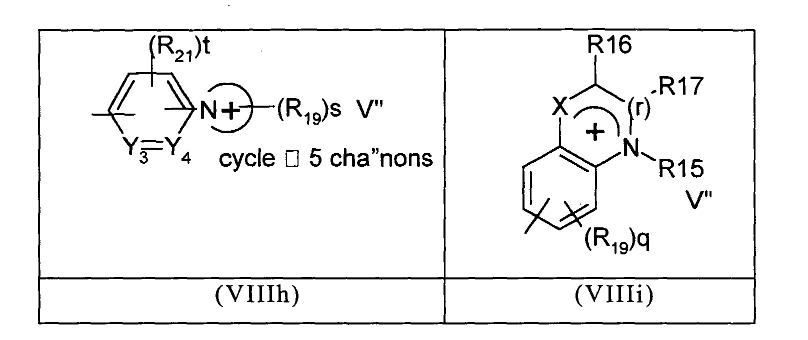 dans lesquelles :
      r est un entier égal à 0 ou 1.
      q est un entier égal à 0, 1, 2 ou 3.
      s est un entier égal à 0, 1, 2, 3 , 4 ou 5.
      t est un entier égal à 0, 1 ou 2.
      Y₃=Y₄ représente C=C, C=N ou N=N.
      si r=0 alors X représente O, S, NR₁₈, CR₂₀.
      si r=1 alors X représente CR₂₀.
      R₁₅ et R₁₈ ont la même définition que R₁ définie ci-dessus.
      R₁₆, R₁₇, R₁₉, R₂₀ et R₂₁ ont la même définition que R₂ définie ci-dessus.
      V" représente un anion organique ou minéral avec la condition que dans la formule (VIIIa) un des groupes A₁, Z₂ et A₃ est un groupe cationique. Les composés de formule (VIIIa) ou (VIIIb) sont notamment décrits dans la demande FR 2 822 698, dont le contenu fait partie intégrante de l'invention.
- Les composés de formule (IX)

   W₁-N=N-W₂-W₃ (IX)

   Dans laquelle W₁ représente un hétérocycle aromatique cationique à 5 chaînons de formule (IXa) suivante : 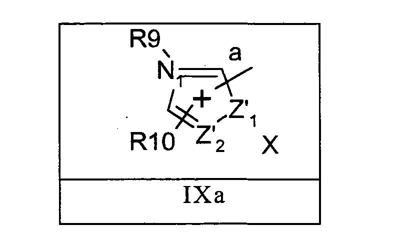
   W₂ représente un groupe divalent aromatique carboné ou pyridinique de formules (IXb) ou (IXc) suivantes : 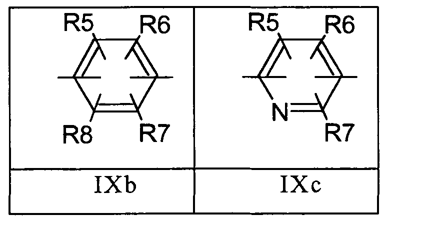
   W₃ représente un hétérocycle à 5 ou 6 chaînons de formule (Xd) suivante : 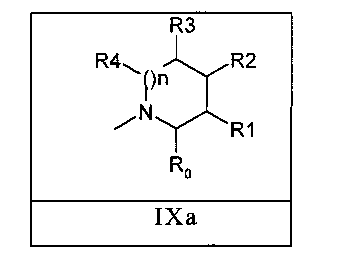 formules dans lesquelles :
   Z'₁ représente un atome d'oxygène, de soufre ou un radical NR₁₂.
   Z'₂ représente un atome d'azote ou un radical CR_{11.}
   R₉ et R₁₂ représentent, indépendamment l'un de l'autre un, radical alkyle en C₁-C₈, éventuellement substitué par un ou plusieurs radicaux choisis parmi un hydroxy, un alcoxy en C₁-C₂, un radical (poly)-hydroxyalcoxy en C₂-C₄, un amino, un (di)alkylamino en C₁-C_{2,} un carboxy, un sulfonique ,un radical phényle éventuellement substitué ;
   R₁₀ et R₁₁ représentent, indépendamment l'un de l'autre, un atome d'hydrogène, un radical alkyle en C₁-C₄, éventuellement substitué par un ou plusieurs radicaux choisis parmi un hydroxy, un alcoxy en C₁-C₂, un radical (poly)-hydroxyalcoxy en C₂-C₄, un amino, un (di)alkylamino en C₁-C₂, un carboxy, un sulfonique, un radical phényle éventuellement substitué, un radical carboxy, un radical sulfonylamino.
   R₅, R₆, R₇ et R₈ représentent, indépendamment les uns des autres un atome d'hydrogène, un atome de chlore, un atome de brome, une chaîne hydrocarbonée en C₁-C₆ linéaire ou ramifiée, pouvant former un ou plusieurs cycles carbonés comportant de 3 à 6 chaînons, et pouvant être saturée ou insaturée, dont un ou plusieurs atomes de carbone de la chaîne carbonée peuvent être remplacés par un atome d'oxygène, d'azote ou de soufre ou par un groupement SO₂, et dont les atomes de carbone peuvent être, indépendamment les uns des autres, substitués par un ou plusieurs atomes d'halogène ; R₅, R₆, R₇ et R₈ ne comportent pas de liaison peroxyde, ni de radicaux diazo ou nitroso ;
   n est un nombre entier égale à 0 ou 1 ;
   R₀, R₁, R₂, R₃ et R₄ représentent, indépendamment les uns des autres, un atome d'hydrogène, un radical hydroxy, amino, acétoxy, un groupement -NR₁₃R₁₄, R₁₃ et R₁₄ représentant indépendamment l'un de l'autre un atome d'hydrogène, un radical alkyle en C₁-C₄ substitué par un ou plusieurs radicaux choisis parmi un atome d'halogène, un radical hydroxy, alcoxy en C₁-C₂, amino ou amino(di)alkyle en C₁-C₂, un radical sulfonylamino, un radical carboxy, un radical carboxamido, un radical amido, un radical mono ou di alkylamido, un halogène, un radical alkyle en C₁-C₆ substitué par un ou plusieurs radicaux choisis parmi un radical hydroxy, alcoxy en C₁-C₂, (poly)-hydroxyalcoxy en C₂-C₄, amino, (di)alkylamino en C₁-C₂, étant entendu qu'au moins un des groupes R₀, R₁, R₂, R₃ et R₄ est différent de l'hydrogène ;
   X est un anion organique ou minéral.
      Les composés de formule (IX) sont notamment décrits dans la demande FR 2 822 693, dont le contenu fait intégralement partie de l'invention.
- Les composés de formule (X)

   W'₁-N=N-W'₂-W'₃ (X)

   Dans laquelle W'₁ représente un hétérocycle aromatique cationique à 5 chaînons de formule (Xa) suivante : 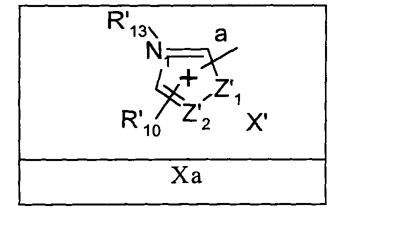
   W'₂ représente un groupe divalent aromatique carboné ou pyridinique de formules (Xb) ou (Xc) suivantes : 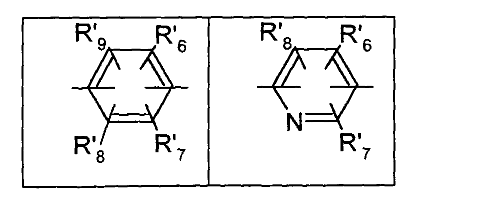 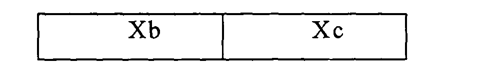
   W'₃ représente un hétérocycle à 7 ou 8 chaînons de formule (Xd) suivante : 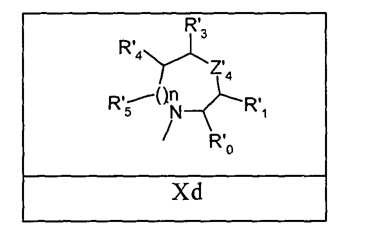 formules dans lesquelles :
      Z'₁ représente un atome d'oxygène, de soufre ou un radical NR'₁₂ ;
      Z'₂ représente un atome d'azote ou un radical CR'₁₁ ;
      R'₁₂ et R'₁₃ représentent, indépendamment l'un de l'autre, un radical alkyle en C₁-C₈, éventuellement substitué par un ou plusieurs radicaux choisis parmi un hydroxy, un alcoxy en C₁-C₂, un radical (poly)-hydroxyalcoxy en C₂-C₄, un amino, un (di)alkylamino en C₁-C₂, un carboxy ou un sulfonique, un radical phényle éventuellement substitué ;
      R'₁₀ et R'₁₁ représentent, indépendamment l'un de l'autre, un atome d'hydrogène, un radical alkyle en C₁-C₄, éventuellement substitué par un ou plusieurs radicaux choisis parmi un hydroxy, un alcoxy en C₁-C₂, un radical (poly)-hydroxyalcoxy en C₂-C₄, un amino, un (di)alkylamino en C₁-C₂, un carboxy ou un sulfonique, un radical phényle éventuellement substitué, un radical carboxy, un radical sulfonylamino ;
      R'₆, R'₇, R'₈ et R'₉ représentent, indépendamment les uns des autres un atome d'hydrogène, un atome de chlore, un atome de brome, une chaîne hydrocarbonée en C₁-C₆ linéaire ou ramifiée, pouvant former un ou plusieurs cycles carbonés comportant de 3 à 6 chaînons, et pouvant être saturées ou insaturées, dont un ou plusieurs atomes de carbone de la chaîne carbonée peuvent être remplacés par un atome d'oxygène, d'azote ou de soufre ou par un groupement SO₂ et dont les atomes de carbone peuvent être, indépendamment les uns des autres, substitués par un ou plusieurs atomes d'halogène ; R'₆, R'₇, R'₈ et R'₉ ne comportent pas de liaison peroxyde, ni de radicaux diazo ou nitroso ;
      n est un nombre entier égale à 1 ou 2 ;
      Z'₄ représente un atome d'oxygène, de soufre, un radical NR'₂ ou un radical CR'₂R"₂ ;
      R'₀, R'₁, R'₂, R"₂, R'₃, R'₄ et R'₅ représentent, indépendamment les uns des autres, un atome d'hydrogène, un radical alkyle, un radical alcoxy, un radical hydroxy, amino, acétoxy, un groupement -NR₁₄R₁₅, R₁₄ et R₁₅ représentant indépendamment l'un de l'autre un atome d'hydrogène, un radical alkyle en C₁-C₄ substitué par un ou plusieurs radicaux choisis parmi un atome d'halogène, un radical hydroxy, alcoxy en C₁-C₂, amino ou amino(di)alkyle en C₁-C₂, un radical sulfonylamino, un radical carboxy, un radical carboxamido, un radical amido, un radical mono ou di alkylamido, un halogène, un radical alkyle en C₁-C₆ substitué par un ou plusieurs radicaux choisis parmi un radical hydroxy, alcoxy en C₁-C₂, (poly)-hydroxyalcoxy en C₂-C₄, amino, (di)alkylamino en C₁-C₂ ;
      X' est un anion organique ou minéral.
         Les composés de formule (X) sont notamment décrits dans la demande FR 2 822 694, dont le contenu fait intégralement partie de l'invention.
- Les composés de formule (XI) 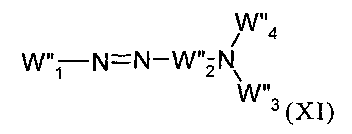 dans laquelle W"₁ représente un hétérocycle aromatique cationique à 5 chaînons de formule (XIa) suivante : 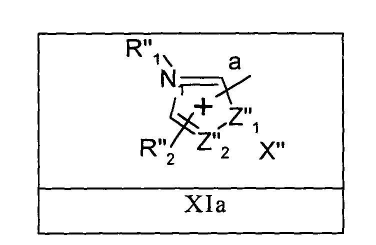
   W"₂ représente un groupe divalent aromatique carboné ou pyridinique de formules (XIb) ou (XIc) suivantes : 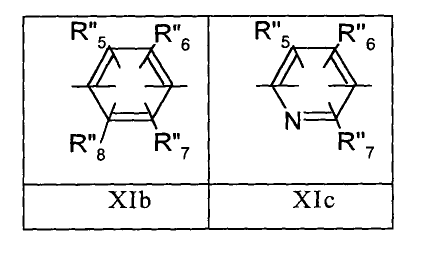 formules dans lesquelles :
      Z"₁ représente un atome d'oxygène, de soufre ou un radical NR"_{4.}
      Z"₂ représente un atome d'azote ou un radical CR"_{3.}
      R"₁ et R"₄ représentent, indépendamment l'un de l'autre, un radical alkyle en C₁-C₈, éventuellement substitué par un ou plusieurs radicaux choisis parmi un hydroxy, un alcoxy en C₁-C₂, un radical (poly)-hydroxyalcoxy en C₂-C₄, un amino, un (di)alkylamino en C₁-C₂, un carboxy, un sulfonique, un radical phényle éventuellement substitué ;
      R"₂ et R"₃ représentent, indépendamment l'un de l'autre, un atome d'hydrogène, un radical alkyle en C₁-C₄, éventuellement substitué par un ou plusieurs radicaux choisis parmi un hydroxy, un alcoxy en C₁-C₂, un (poly)-hydroxyalcoxy en C₂-C₄, un amino, un (di)alkylamino en C₁-C₂, un carboxy ou un sulfonique, un radical phényle éventuellement substitué, un radical carboxy, un radical sulfonylamino ;
      R"₅, R"₆, R"₇, R"₈ et W"₄ représentent, indépendamment les uns des autres un atome d'hydrogène, un atome de chlore, un atome de brome, une chaîne hydrocarbonée en C₁-C₆ linéaire ou ramifiée, pouvant former un ou plusieurs cycles carbonés comportant de 3 à 6 chaînons, et pouvant être saturées ou insaturées, dont un ou plusieurs atomes de carbone de la chaîne hydrocarbonée peuvent être remplacés par un atome d'oxygène, d'azote ou de soufre ou par un groupement SO₂, et dont les atomes de carbone peuvent être, indépendamment les uns des autres, substitués par un ou plusieurs atomes d'halogènes ; R"₅, R"₆, R"₇, R"₈ et W"₄ ne comportant pas de liaison peroxyde, ni de radicaux diazo ou nitroso, et W"₄ étant un substituant non aromatique ;
      W"₃ représente un radical thiényle, pyrazolyle, pyrrolyle, imidazolyle, furyle, triazolyle, thiadiazolyle, isoxazolyle, 5-isothiazolyle, thiazolyle, oxazolyle, pyridyle, pyrimidinyle, triazinyle, pyridazinyle, pyrazinyle, chacun de ces cycles hétéroaromatiques pouvant être substitué par au moins un radical alkyle en C₁-C₄, éventuellement substitué par un ou plusieurs hydroxy, alcoxy en C₁-C₄, (poly)-hydroxyalcoxy, amino, (di )alkylamino en C₁ -C₄, (poly)hydroxyalkylamino en C₂-C₄, carboxy, sulfonyle, alcoxycarbonyle ou thioéther en C₁-C₄, un radical phényle éventuellement substitué par un ou plusieurs radicaux choisis parmi les radicaux alcoxy en C₁-C₂, amino, (di)-alkylamino en C₁-C₂, carboxy, sulfonyle, alkyle en C₁-C₄, halogène ou thioéther en C₁-C₂, un halogène tel qu'un atome de chlore, de fluor ou de brome, un radical amino, un radical alkylamino en C₁-C₄, un radical (poly)hydroxyalkylamino en C₂-C₄, un radical (di)alkylamino en C₁-C₄, un radical alcoxy en C₁-C₂, un radical carboxyle, un radical sulfonylamino ;
      X" est un anion organique ou minéral.
      Les composés de formule (XI) sont notamment décrits dans la demande FR 2 822 695, dont le contenu fait intégralement partie de l'invention. ¥
- Les composés de formule (XII)

   W⁰ ₁-W⁰ ₂-N=N-W⁰ ₃ (XII)

   Dans laquelle W⁰₁ représente un hétérocycle à 5, 6, 7 ou 8 chaînons de formule (XIIa) suivante : 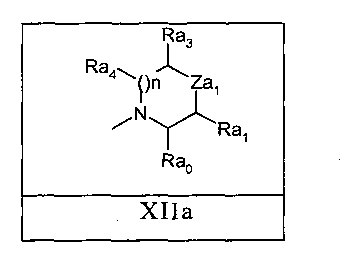
   W⁰₂ représente un groupe divalent aromatique carboné, pyridinique ou pyridazinique de formule (XIIb) suivante : 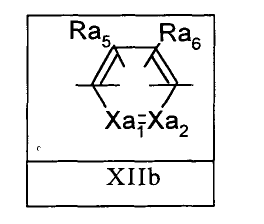
   W⁰₃ représente un radical hétéroaromatique cationique représenté par la formule (XIIc) suivante : 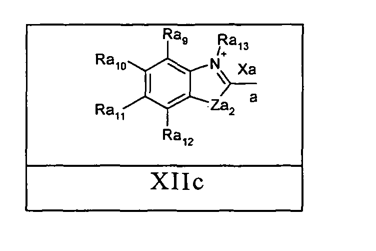 formules (XIIa), (XIIb), (XIIc) dans lesquelles :
      n=0, 1, 2 ou 3, étant entendu que lorsque n est supérieur ou égal à 2, alors les radicaux Ra₄ peuvent être identiques ou différents ;
      Xa₁ représente un atome d'azote ou un radical CRa₇ ;
      Xa₂ représente un atome d'azote ou un radical CRa₈ ;
      Za₁ représente un radical CHRa₂, un atome d'oxygène, de soufre ou un radical NRa₁₄ ;
      Za₂ représente un atome d'oxygène, de soufre ou un radical NRa₁₅ ;
      Ra₀, Ra₁, Ra₂, Ra₃, Ra₄, Ra₅, Ra₆, Ra₇, Ra₈,.Ra₉, Ra₁₀, Ra₁₁, et Ra₁₂ représentent, identiques ou différents, un atome d'hydrogène, une chaîne hydrocarbonée en C₁-C₁₀ linéaire ou ramifiée, pouvant former un ou plusieurs cycles carbonés comportant de 3 à 6 chaînons, et pouvant être saturée ou insaturée, dont un ou plusieurs atomes de carbone de la chaîne carbonée peuvent être remplacés par un atome d'oxygène, d'azote ou de soufre ou par un groupement SO₂, et dont les atomes de carbone peuvent être, indépendamment les uns des autres, substitués par un ou plusieurs atomes d'halogène, Ra₀, Ra₁, Ra₂, Ra₃, Ra₄, Ra₅, Ra₆, Ra₇, Ra₈,.Ra₉, Ra₁₀, Ra₁₁, et Ra₁₂ ne comportant pas de liaison peroxyde, ni de radicaux diazo ou nitroso ;
      Ra₁₄ représente un atome d'hydrogène, une chaîne hydrocarbonée en C₁ -C₁₀ linéaire ou ramifiée, pouvant former un ou plusieurs cycles carbonés comportant de 3 à 6 chaînons, et pouvant être saturée ou insaturée, dont un ou plusieurs atomes de carbone de la chaîne carbonée peuvent être remplacés par un atome d'oxygène, d'azote ou de soufre ou par un groupement SO₂, et dont les atomes de carbone peuvent être, indépendamment les uns des autres, substitués par un ou plusieurs atomes d'halogène, Ra₁₄ ne comportant pas de liaison peroxyde, ni de radicaux diazo ou nitroso, étant entendu que lesdits atomes d'oxygène, d'azote et de soufre ne sont pas reliés directement à l'atome d'azote porteur du radical Ra₁₄ ;
      Ra₅ et Ra₆ peuvent former un cycle aromatique carboné, tel qu'un phényle ;
      Ra13 et Ra15 représentent, identiques ou différents, un radical alkyle en C1-C8,éventuellement substitué par un ou plusieurs radicaux choisis dans le groupe constitué par un hydroxy, un alcoxy en C1-C2, un radical (poly)hydroxyalcoxy en C2-C4, un amino, un (di)-alkylamino en C1-C2, un carboxyle, un sulfonique, un phényl éventuellement substitué ;
         la liaison a du cycle cationique de la formule (XIIc) est reliée au groupement azoïque de la formule (XIIa) ;
      Xa est un anion organique ou minéral.
      Les composés de formule (XII) sont notamment décrits dans la demande FR 2 829 926, dont le contenu fait partie intégrante de l'invention.
- Les composés de formule (XIII)

   A-N=N-B (XIII)

   Dans laquelle le symbole A représente un groupement choisi parmi les structures A₁ à A₃ suivantes : 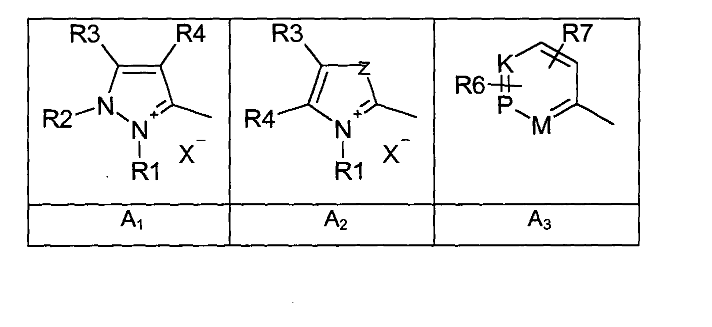 Dans lesquelles, R₁ désigne un radical alkyle en C₁-C₄, un radical phényle pouvant être substitué par un radical alkyle en C₁-C₄ ou un atome d'halogène choisi parmi le chlore, le brome, l'iode et le fluor ;
   R₂ désigne un radical alkyle en C₁-C₄ ou un radical phényle ;
   R₃ et R₄, identiques ou différents, représentent un radical alkyle en C₁-C₄, un radical phényle, ou bien, dans le cas de la structure A₁, peuvent former ensemble un cycle benzénique substitué, et dans le cas de la structure A₂, peuvent former ensemble un cycle benzénique éventuellement substitué par un ou plusieurs radicaux alkyle en C₁-C₄, alcoxy en CI-C4, ou NO₂ ;
   R₃ peut en outre désigner un atome d'hydrogène ;
   Z désigne un atome d'oxygène, de soufre ou un groupement -NR₂ ;
   M représente un groupement -CH, -CR (R désignant alkyle en C₁-C₄) ou -NR₅ (X⁻)ᵣ ;
   K représente un groupement -CH, -CR (R désignant alkyle en C₁-C₄) ou -NR₅ (X⁻)ᵣ ;
   P représente un groupement -CH, -CR (R désignant alkyle en C1-C4) ou -NR₅ (X⁻)ᵣ. r désignant 0 ou 1 ;
   R₅ représente un atome O⁻, un radical alcoxy en C₁-C₄, ou un radical alkyle en C₁-C₄ ;
   R₆ et R₇, identiques ou différents, représentent un atome d'hydrogène ou d'halogène choisi parmi le chlore, le brome, l'iode et le fluor, un radical alkyle en C₁-C₄, alcoxy en C₁-C₄, un radical NO₂ ;
   X⁻ représente un anion de préférence choisi parmi le chlorure, l'iodure, le méthyl sulfate, l'éthyl sulfate, l'acétate et le perchlorate, sous réserve que,
- si R₄ désigne un radical alkyle en C₁-C₄ et Z désigne un atome de soufre, R₃ ne désigne pas un atome d'hydrogène.
   - si R₅ désigne O⁻, alors r désigne zéro.
   - si K ou P ou M désignent -N-alkyle C₁-C₄ X⁻, alors R₆ ou R₇ est différent d'un atome d'hydrogène.
   - si. K désigne -NR₅(X⁻)ᵣ, alors M=P=-CH ou -CR
   - si M désigne -NR₅(X⁻)ᵣ, alors K=M= -CH ou -CR
   - si P désigne -NR₅(X⁻)ᵣ. alors K=M et désignent -CH ou -CR
   - si Z désigne -NR₂ et R₂ désigne un radical alkyle en C₁-C₄, alors au moins un des radicaux R₁, R₃ ou R₄ de A₂ est différent d'un radical alkyle en C₁-C_{4.}
   Le symbole B représente :
   a- Un groupement de structure B₁ suivante : 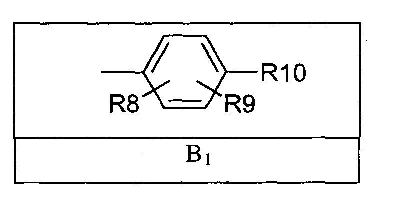 Dans laquelle, R₈ représente un atome d'hydrogène, un atome d'halogène choisi parmi le chlore, le brome, l'iode et le fluor, un radical alkyle en C₁-C₄, alcoxy en C₁-C₄, un radical -OH, -NO₂, -NHR₁₁, -NR₁₂R₁₃, -NHCOalkyle en C₁-C₄, ou forme avec R₉ un cycle à 5 ou 6 chaînons, contenant ou non un ou plusieurs hétéroatomes choisis parmi l'azote, l'oxygène ou le soufre ;
      R₉ représente un atome d'hydrogène, un atome d'halogène choisi parmi le chlore, le brome, l'iode et le fluor, un radical alkyle en C₁-C₄, alcoxy en C₁-C₄, ou forme avec R₁₀ ou R₁₁ un cycle à 5 ou 6 chaînons contenant ou non un ou plusieurs hétéroatomes choisis parmi l'azote, l'oxygène ou le soufre ;
      R₁₀ représente un atome d'hydrogène, un radical -OH, un radical -NHR₁₁, un radical -NR₁₂R₁₃ ;
      R₁₁ représente un atome d'hydrogène, un radical alkyle en C₁-C₄, un radical monohydroxyalkyle en C₁-C₄, polyhydroxyalkyle en C₂-C₄, un radical phényle.
      R₁₂ et R₁₃, identiques ou différents, représentent un radical alkyle en C₁-C₄, un radical monohydroxyalkyle en C₁-C₄, polyhydroxyalkyle en C₂-C₄ ;
   b- un groupement hétérocyclique azoté à 5 ou 6 chaînons susceptible de renfermer d'autres hétéroatomes et/ou des groupements carbonylés et pouvant être substitué par un ou plusieurs radicaux alkyle en C₁-C₄, amino ou phényle et notamment un groupement de structure B₂ suivante : 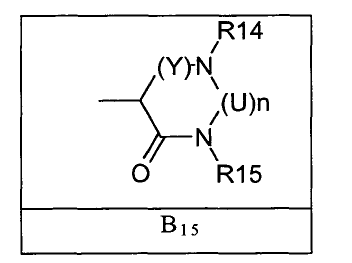 Dans laquelle, R₁₄ et R₁₅ identiques ou différents, représentent un atome d'hydrogéne, un radical alkyle en C₁-C₄, un radical phényle ;
      Y désigne le radical -CO- ou le radical 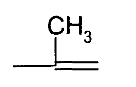
      N vaut 0 ou 1, avec, lorsque n désigne 1, U désigne le radical -CO. Les composés de formule (XIII) sont notamment décrits dans la demande FR 2 807 650, dont le contenu fait intégralement partie de l'invention.
- Les composés de formule (XIV) :

   W₁-N=N-W₂-W₃ (XIV)

   dans laquelle
   - W1 représente un hétérocycle aromatique cationique à 5 chaînons de formule(II) suivante : 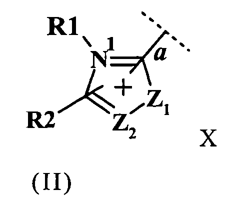
   - W2 représente un groupe divalent aromatique carboné ou pyridinique de formules (IV) ou (V) suivantes : 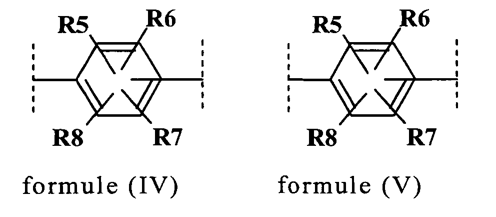
   - W3 représente un radical hétéroaromatique azoté à 5 ou 6 chaînons relié à W2 par l'atome d'azote du cycle du radical hétéroaromatique, le radical héréroaromatique étant choisi parmi les radicaux pyrazolyle, pyrrolyle, imidazolyle, triazolyle, thiadiazolyle, pyridazinyle, pyrazinyle, chacun de ces hétéroaromatiques pouvant être éventuellement substitué par un ou plusieurs radicaux choisi parmi un atome d'hydrogène ; un radical alkyle en C1-C6, éventuellement substitué par un ou plusieurs radicaux hydroxy, alcoxy en C1-C4, (poly)-hydroxyalcoxy, amino, (di)alkylamino en C1-C4, (poly)hydroxyalkylamino en C2-C4, carboxy, sulfonyle, alcoxycarbonyle, thioether en C1-C4 ; un radical phényle éventuellement substitué par un ou plusieurs radicaux choisis parmi les radicaux alcoxy en C1-C2, amino, (di)-alkylamino en C1-C2, carboxy, sulfonyle, alkyle en C1-C4, halogène, thioéther en C1-C2,
   - Z1 représente un atome d'oxygène, de soufre ou un radical NR4,
   - Z2 représente un atome d'azote ou un radical CR3,
   - R1 et R4 représentent, indépendamment l'un de l'autre, un radical alkyle en C1-C8, éventuellement substitué par un ou plusieurs radicaux choisis parmi un hydroxy, un alcoxy en C1-C2, un radical (poly)-hydroxyalcoxy en C2-C4, un amino, un (di)alkylamino en C1-C2 , un carboxy, un sulfonique ; un radical phényle éventuellement substitué,
   - R2 et R3 représentent, indépendamment l'un de l'autre, un atome d'hydrogène ; un radical alkyle en C1-C6, éventuellement substitué par un ou plusieurs radicaux choisis parmi un hydroxy, un alcoxy en C1-C2, un radical (poly)-hydroxyalcoxy en C2-C4, un amino, un (di)alkylamino en C1-C2 , un carboxy, un sulfonique; un radical phényle éventuellement substitué ; un radical carboxy ; un radical sulfonylamino ;
   - R5, R6, R7 et R8 représentent, ensemble ou indépendamment l'un de l'autre un atome d'hydrogène ; un atome de chlore ; un atome de brome ; une chaîne hydrocarbonée en C1-C6 linéaire ou ramifiée, pouvant former un ou plusieurs cycles carbonés comportant de 3 à 6 chaînons, et pouvant être saturées ou insaturées, dont un ou plusieurs atomes de carbone de la chaîne carbonée peuvent être remplacés par un atome d'oxygène, d'azote ou de soufre ou par un groupement SO2, et dont les atomes de carbone peuvent être, indépendamment les uns des autres, substitués par un ou plusieurs atomes d'halogènes ; R5, R6, R7 et R8 ne comportant pas de liaison peroxyde, ni de radicaux diazo ou nitroso,
   - X représente un anion organique ou minéral.
   Les composés de formule (XIV) sont notamment décrits dans la demande WO 02/078660, dont le contenu fait intégralement partie de l'invention.
- Les composés de formule (XV) :

   W₁-N=N-W₂-(W₃)n-W₄-N=N-W₅ (XV)

   dans laquelle
   n représente 0 ou 1,
      - W1 et W5, indépendamment l'un de l'autre, représentent un radical hétéroaromatique de formules (II) et (III) suivantes : 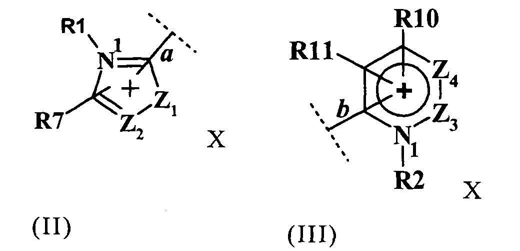
      - W3 représente un atome d'oxygène, un radical NR14, un groupe -NR15-W6-NR16-, un groupe -NR16-W6-O-, un groupe -O-W6-O-, un radical W6, un radical carbonyle,
      - W2 et W4 représentent, indépendamment l'un de l'autre, un groupement aromatique carboné, pyridinique ou pyridazinyle de formule (IV) 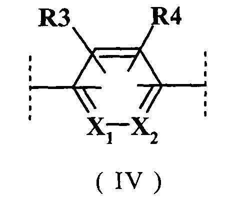
      - W6 représente un groupement aromatique ou hétéroaromatique à 5 ou 6 chaînons de formule (V) : 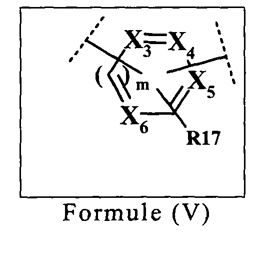 dans lesquelles
         m représente 0 ou 1,
         X1 représente un atome d'azote ou un radical CR5,
         X2 représente un atome d'azote ou un radical CR6,
         X3 représente un atome d'azote, de carbone ou un radical CR18,
         X4 représente un atome d'azote, de carbone ou un radical CR19,
         X5 représente un atome d'azote, de carbone ou un radical CR20,
         X6 représente un atome d'azote, de carbone ou un radical CR21,
         Z1 représente un atome d'oxygène, de soufre ou un radical NR8,
         Z2 représente un atome d'azote ou un radical CR9,
         Z3 représente un atome d'azote ou un radical CR12,
         Z4 représente un atome d'azote ou un radical CR13,
            la liaison *a* du cycle cationique à 5 chaînons de la formule (II) est reliée au groupement azoïque de la formule (XV),
            la liaison *b* du cycle cationique à 6 chaînons de la formule (III) est reliée au groupement azoïque de la formule (XV),
            étant entendu que lorsque X3 à X6 représentent un atome de carbone, alors ils sont reliés à W2 ou W4,
            étant entendu que la formule (V) ne contient pas plus de trois atomes d'azote,
            étant entendu que lorsque la formule (V) contient trois atomes d'azote, ils sont non contigus,
         R1, R2 et R8 représentent indépendamment l'un de l'autre un radical alkyle en C1-C8, linéaire ou ramifié, éventuellement substitué par un ou plusieurs radicaux choisis parmi les radicaux hydroxy, alcoxy en C1-C2, (poly)-hydroxyalcoxy en C2-C4, amino, (di)alkylamino en C1-C2 , carboxy ou sulfonique ; un radical phényle éventuellement substitué par un ou plusieurs radicaux choisis parmi les radicaux hydroxy, alcoxy en C1-C2, (poly)-hydroxyalcoxy en C2-C4, amino, (di)alkylamino en C1-C2, carboxy, sulfonique ou un atome d'halogène tel que chlore, fluor ou brome,
         R3, R4, R5, R6, R7, R9, R10, R11, R12, R13 , R14, R15 , R16, R17, R18 , R19 , R20 et R21 représentent, indépendamment l'un de l'autre, un atome d'hydrogène, une chaîne hydrocarbonée en C1-C16 linéaire ou ramifiée, pouvant être saturée ou insaturée, dont un ou plusieurs atomes de carbone de la chaîne carbonée peuvent être remplacés par un atome d'oxygène, d'azote ou de soufre ou par un groupement SO2, et dont les atomes de carbone peuvent être, indépendamment les uns des autres, substitués par un ou plusieurs atomes d'halogènes ; R3, R4, R5, R6, R7, R9, R10, R11, R12, R13 , R14, R15, R16, R17, R18 , R19 , R20 et R21 ne comportant pas de liaison peroxyde, ni de radicaux diazo ou nitroso,
         R7 avec R9, R10 avec R11 et R12 avec R13 peuvent former un cycle aromatique carboné, tel qu'un phényle,
         X est un anion organique ou minéral.
         Les composés de formule (XI) sont notamment décrits dans la demande WO 02/100834, dont le contenu fait intégralement partie de l'invention.
- Les composés de formule (XVI) :

   [W₁-N=N-W₂-W₃-(W₄-W₅)ₚ]₂-L (XVI)

   dans laquelle
   p vaut 0 ou 1.
   W1 représente un radical hétéroaromatique de formules (II) et (III) suivantes : 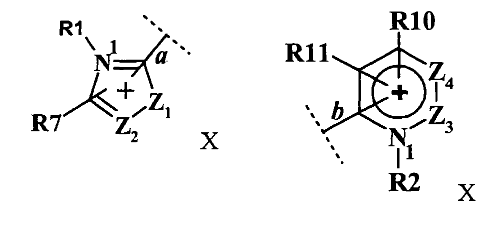 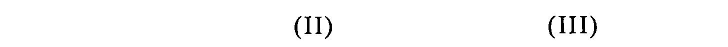
   W2 représente un groupement aromatique carboné, pyridinique ou pyridazinyle de formule (IV) suivante : 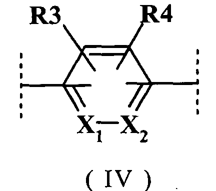
      - W3 et W5 représentent indépendamment l'un de l'autre un radical -NR14- ou un atome d'oxygène -O- , ou un atome d'azote,
   W4 représente une chaîne hydrocarbonée en C1-C16 linéaire ou ramifiée, pouvant former un ou plusieurs cycles carbonés comportant de 3 à 6 chaînons, et pouvant être saturée ou insaturée, dont un ou plusieurs atomes de carbone de la chaîne carbonée peuvent être remplacés par un atome d'oxygène, d'azote ou de soufre ou par un groupement SO2, et dont les atomes de carbone peuvent être, indépendamment les uns des autres, substitués par un ou plusieurs atomes d'halogènes, W4 ne comportant pas de liaison peroxyde, ni de radicaux diazo ou nitroso; un cycle pipérazine ; un groupe de formule (V) suivante : 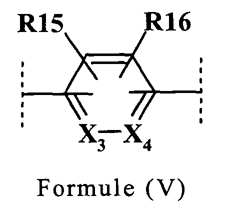 Formules (II), (III), (IV), (V) dans lesquelles :
      X1 représente un atome d'azote ou un radical CR5
      X2 représente un atome d'azote ou un radical CR6
      X3 représente un atome d'azote ou un radical CR17
      X4 représente un atome d'azote ou un radical CR18
      Z1 représente un atome d'oxygène, de soufre ou un radical NR8,
      Z2 représente un atome d'azote ou un radical CR9,
      étant entendu que (Z1, Z2) est différent de (NR8, CR9),
      Z3 représente un atome d'azote ou un radical CR12,
      Z4 représente un atome d'azote ou un radical CR13,
         la liaison *a* du cycle cationique à 5 chaînons de la formule (II) est reliée au groupement azoïque de la formule (XVI),
         la liaison *b* du cycle cationique à 6 chaînons de la formule (III) est reliée au groupement azoïque de la formule (XVI),
         lorsque p vaut 0, alors L représente une chaîne hydrocarbonée en C1-C16 linéaire ou ramifiée, pouvant former un ou plusieurs cycles carbonés comportant de 3 à 6 chaînons dont un ou plusieurs atomes de carbone de la chaîne carbonée peuvent être remplacés par un atome d'oxygène, d'azote ou de soufre ou par un groupement SO2, et dont les atomes de carbone peuvent être, indépendamment les uns des autres, substitués par un ou plusieurs atomes d'halogènes ; L ne comportant pas de liaison peroxyde, ni de radicaux diazo ou nitroso,
         lorsque p vaut 1, alors L représente une chaîne hydrocarbonée en C1-C16 linéaire ou ramifiée, pouvant former un ou plusieurs cycles carbonés comportant de 3 à 6 chaînons, et pouvant être saturée ou insaturée, dont un ou plusieurs atomes de carbone de la chaîne carbonée peuvent être remplacés par un atome d'oxygène, d'azote ou de soufre ou par un groupement SO2 et ne comportant pas de liaison peroxyde, ni de radicaux diazo ou nitroso,
      R3, R4, R5, R6, R7, R9, R10, R11, R12, R13, R14, R15, R16, R17 et R18 représentent, ensemble ou indépendamment l'un de l'autre , un atome d'hydrogène, une chaîne hydrocarbonée en C1-C16 linéaire ou ramifiée, pouvant former un ou plusieurs cycles carbonés comportant de 3 à 6 chaînons, et pouvant être saturée ou insaturée, dont un ou plusieurs atomes de carbone de la chaîne carbonée peuvent être remplacés par un atome d'oxygène, d'azote ou de soufre ou par un groupement SO2, et dont les atomes de carbone peuvent être, indépendamment les uns des autres, substitués par un ou plusieurs atomes d'halogènes ; R3, R4, R5, R6, R7, R9, R10, R11, R12 , R13 , R14, R15, R16, R17 et R18 comportant pas de liaison peroxyde, ni de radicaux diazo ou nitroso,
      R3 avec R4, R7 avec R9, R10 avec R11,R12 avec R13 et R15 avec R16 peuvent former un cycle aromatique carboné, tel qu'un phényle,
      X est un anion organique ou minéral.
      Les composés de formule (XI) sont notamment décrits dans la demande WO 02/100369, dont le contenu fait intégralement partie de l'invention.
- Les composés de formule (XVI) :

   W₁-N=N-W₂-NW₃-W₄-W₅ (XVI)

   dans laquelle
   - W1 représente un hétérocycle aromatique cationique à 5 ou 6 chaînons de formules (II) et (III) suivantes : 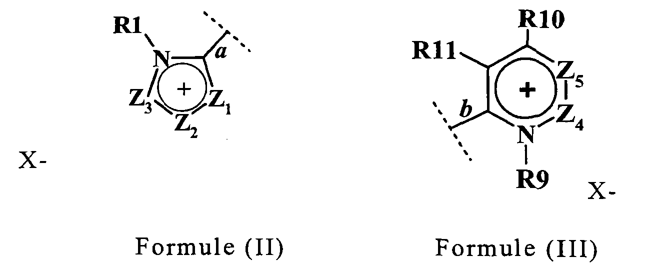 dans lesquelles :
      Z1 représente un atome d'oxygène, un atome de soufre, un radical NR2, ou un radical CR3,
      Z2 représente un atome d'azote ou un radical CR4,
      Z3 représente un radical NR12 ou un radical CR13,
      Z4 représente un atome d'azote ou un radical CR14,
      Z5 représente un atome d'azote ou un radical CR15,
         la liaison *a* relie le cycle cationique à 5 chaînons de formule (II) à la fonction azoïque de la formule (XVI).
         la liaison *b* du cycle cationique à 6 chaînons de la formule (III) est reliée à la fonction azoïque de la formule (XVI).
      X- est un anion organique ou minéral ;
      W2 et W4 (formule I) représentent, identiques ou différents, un groupe divalent aromatique carboné ou pyridinique de formules (IV) ou (V) suivantes : 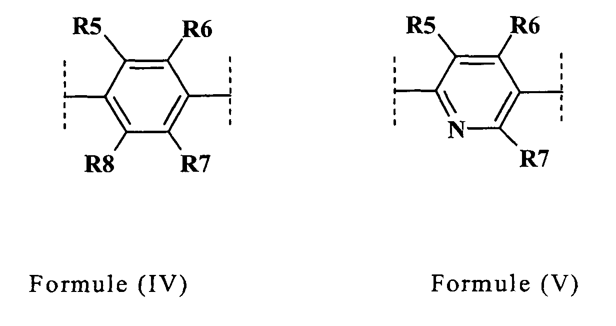
   - W3 représente un atome d'hydrogène ou un radical alkyle en C1-C6 éventuellement substitué par un ou plusieurs radicaux hydroxy, un ou plusieurs radicaux alcoxy, un ou plusieurs radicaux amino, un ou plusieurs radicaux monoalkylamino, un ou plusieurs radicaux dialkylamino.
   - W5 représente un radical hétéroaromatique azoté à 5 chaînons relié à W4 par l'atome d'azote du cycle dudit radical hétéroaromatique, ce radical héréroaromatique étant choisi parmi les radicaux pyrazolyle, pyrrolyle, imidazolyle, triazolyle, thiadiazolyle, chacun de ces hétéroaromatiques pouvant être substitué par un ou plusieurs atomes d'hydrogène, de chlore ou de fluor, ou par un ou plusieurs radicaux alkyle en C1-C6, éventuellement substitués par un ou plusieurs radicaux hydroxy, alcoxy en C1-C4, (poly)-hydroxyalcoxy, amino, (di)alkylamino en C1-C4, (poly)hydroxyalkylamino en C2-C4, carboxyle, sulfonyle, alcoxycarbonyle, thioether en C1-C4 ; ou par un ou plusieurs radicaux phényle éventuellement substitués par un ou plusieurs radicaux choisis parmi les radicaux hydroxy, alcoxy en C1-C2, amino, (di)-alkylamino en C1-C2, carboxy, sulfonyle, alkyle en C1-C4, halogène, thioéther en C1-C2,
      R1, R2, R9, R12 représentent, identiques ou différents, un radical phényle éventuellement substitué ou un radical alkyle en C1-C8, éventuellement substitué par un ou plusieurs radicaux choisis dans le groupe formé par les radicaux hydroxy, alcoxy en C1-C2, (poly)-hydroxyalcoxy en C2-C4, amino, (di)alkylamino en C1-C2 ,
   - R5, R6, R7 et R8 représentent, identiques ou différents, un atome d'hydrogène ; un atome de chlore ; un atome de brome ; une chaîne hydrocarbonée en C1-C8 linéaire ou ramifiée, pouvant former un ou plusieurs cycles carbonés comportant de 3 à 6 chaînons, et pouvant être saturée ou insaturée, dont un ou plusieurs atomes de carbone de la chaîne carbonée peuvent être remplacés par un atome d'oxygène, d'azote ou de soufre ou par un groupement SO2, et dont les atomes de carbone peuvent être, indépendamment les uns des autres, substitués par un ou plusieurs atomes d'halogène ; R5, R6, R7 et R8 ne comportant pas de liaison peroxyde, ni de radicaux diazo ou nitroso.
   - R3, R4, R10, R11, R13, R14 et R15 représentent, identiques ou différents, un atome d'hydrogène, une chaîne hydrocarbonée en C1-C16 linéaire ou ramifiée, pouvant former un ou plusieurs cycles carbonés comportant de 3 à 6 chaînons, et pouvant être saturée ou insaturée, dont un ou plusieurs atomes de carbone de la chaîne carbonée peuvent être remplacés par un atome d'oxygène, d'azote ou de soufre ou par un groupement SO2, et dont les atomes de carbone peuvent être, indépendamment les uns des autres, substitués par un ou plusieurs atomes d'halogène ; R3, R4, R10, R11, R13, R14 et R15 ne comportant pas de liaison peroxyde, ni de radicaux diazo ou nitroso.
      R4 avec R13 et R14 avec R15 peuvent former un cycle aromatique carboné, tel qu'un phényle.
   Les composés de formule (XI) sont notamment décrits dans la demande FR 2 844 269, dont le contenu fait intégralement partie de l'invention.

Parmi les colorants azoïques ou azométhiniques cationiques de l'invention, on peut aussi citer le Basic Blue 41, le Basic Blue 67, le Basic Brown 1, le Basic Brown 4, le Basic Brown 16, le Basic Brown 17, le Basic Red 18, le Basic Red 22, le Basic Red 46, le Basic Red 76, le Basic Red 104, le Basic Red 118, le Basic Violet 35, le Basic Yellow 45, le Basic Yellow 57, le Basic Yellow 67.

Parmi les colorants directs méthiniques cationiques, on peut aussi citer le Basic Red 14, le Basic Yellow 13 et le Basic Yellow 29.

Les colorants directs cationiques azoïques, méthiniques ou azométhiniques préférés sont les composés de formule I, II, III et III'.

Le ou les colorants directs cationiques de l'invention représentent de préférence de 0,0005 à 15% en poids environ du poids total de la composition et encore plus préférentiellement, de 0,005 à 10% en poids environ.

Dans le cadre de la présente invention, les enzymes alcool oxydases pouvant être utilisées dans la composition tinctoriale conforme à l'invention appartiennent à la classe E.C.1.1.3 de la nomenclature des enzymes, (voir Enzyme Nomenclature, Academic Press Inc ; 1992).

Lesdites enzymes peuvent notamment être choisies parmi les alcool primaire oxydases ( EC1.1.3.13), les alcool secondaire oxydases (EC 1.1.3.18), les alcool à longue chaîne hydrocarbonée oxydases (EC 1.1.3.20), les alcool polyvinylique oxydases ( EC 1.1.3.30), l'alcool vanillique oxydase (EC 1.1.3.38), les alcool aromatique oxydases (EC 1.1.3.7) encore appelées aryl alcool oxydases.

Les enzymes alcool oxydases forment une classe particulière d'enzymes oxydo-réductase à 2 électrons.

L'enzyme alcool oxydase utilisée dans la composition tinctoriale selon l'invention peut être issue d'un extrait de végétaux, d'animaux, de microorganismes (bactérie, champignon, levure, microalgue ou virus), de cellules différenciées ou dédifférenciées, obtenues *in vivo* ou *in vitro,* modifiées ou non modifiées génétiquement, ou synthétiques (obtenues par synthèse chimique ou biotechnologique).

A titre d'exemples, on peut citer en particulier les enzymes extraites des espèces suivantes : *Pinus, Gastropode, Manduca, Pichia, Candida, Pleurotus, Pseudomonas, Rhodococcus, Aspergillus, Kamagataella, Phanerochaete, Polyporus, Hansenula, Poria, Penicillium,* et de façon encore plus particulière les espèces suivantes *: Pinus strobus,* qui est une espèce d'origine végétale, *Gastropode mollusc, Manduca sexta,* qui sont d'origine animale, *Pichia sp. (pastoris, methanolica, angusta)* et *Candida sp. (boidinii, albicans, tropicalis*), qui sont des levures, *Pleurotus pulmonarius Aspergillus niger, Kamagataella pastoris, Phanerochaete chrysosporium, Polyporus obtusus, Hansenula polymorpha, Poria contigua, Penicillium simplicissimum,* qui sont des champignons, et *Pseudomonas pseudoalcaligenes, Rhodococcus erythropolis,* qui sont des bactéries.

De préférence, l'alcool oxydase utilisée provient de la souche *Pichia pastoris*.

Généralement, la concentration en enzyme alcool oxydase utilisée dans la composition tinctorial est comprise entre 0,05 et 20% en poids par rapport au poids total de ladite composition, de préférence entre 0,1 et 10%, et plus particulièrement entre 0,5 et 8% en poids par rapport au poids total de la composition tinctoriale.

L'activité enzymatique des enzymes alcool oxydases utilisées conformément à l'invention peut être définie à partir de l'oxydation du donneur en condition aérobie. L'unité U correspond à la quantité d'enzyme conduisant à la génération de 1 µmole de peroxyde d'hydrogène par minute à un pH donné et à une température de 25°C.

De façon préférentielle, la quantité en enzyme alcool oxydase utilisée dans la composition tinctoriale est comprise entre 10³U et 10⁵U, et de préférence entre 2 10³ et 5 10⁴U pour 100g de composition tinctoriale.

Le ou les substrats pour ladite enzyme sont encore appelés donneurs pour l'enzyme. La nature de ce substrat varie en fonction de la nature de l'enzyme alcool oxydase qui est utilisée. Le substrat pour l'enzyme dans les compositions de l'invention sera de préférence un alcool choisi parmi les alcools primaires, secondaires, ramifiés ou non, saturés ou non, substitués ou non, les alcools à longue chaîne hydrocarbonée et les alcools aromatiques. Par exemple à titre de donneur pour les alcool primaire oxydases, on peut citer les alcools primaires contenant de 1 à 6 atomes de carbone ; à titre de donneur pour les aryl alcool oxydases : l'alcool benzylique, le 4-terbutyl benzylique alcool, le 3-hydroxy-4- méthoxybenzyl alcool, le vératryl alcool, le 4-méthoxybenzyl alcool, l'alcool cinnamique ; le 2,4 hexadiéne-1-ol peut également être utilisé en tant que donneur pour les aryl alcool oxydases.

Selon une autre variante de l'invention, le substrat pour l'enzyme est un composé portant au moins une fonction alcool aliphatique ou aromatique, approprié pour réagir avec l'enzyme utilisée. Le composé portant au moins une fonction alcool aliphatique ou aromatique peut notamment être un précurseur de colorant d'oxydation ou encore un adjuvant cosmétiquement acceptable par exemple un polymère, un tensioactif, un conservateur porteur d'au moins une fonction alcool. De façon encore plus préférée, le substrat pour l'enzyme est un précurseur de colorant d'oxydation portant au moins une fonction alcool aliphatique ou aromatique. Par exemple, la N-(β-hydroxypropyl)-paraphénylènediamine qui porte une fonction alcool primaire peut avoir la fonction de base d'oxydation et de substrat pour l'alcool oxydase. De même, les coupleurs d'oxydation, tels que le méta- ou le para-aminophénol, peuvent remplir les deux fonctions. De tels précurseurs sont décrits ci-après. Dans cette variante, l'utilisation d'autres substrats de l'enzyme est facultative.

Ainsi la présente demande vise une composition pour la teinture des fibres kératiniques, en particulier des fibres kératiniques humaines telles que les cheveux, contenant, dans un milieu approprié pour la teinture, au moins les composés suivants : un précurseur de colorant d'oxydation ; au moins une enzyme alcool oxydase ; au moins un substrat, portant une fonction alcool, pour ladite enzyme et au moins un colorant direct cationique azoïque, méthinique ou azométhinique, ledit substrat pouvant être substitué (c'est-à-dire remplacé) en tout ou en partie par le précurseur de colorant d'oxydation dans le cas où il porte au moins une fonction alcool aliphatique ou aromatique.

L'utilisation de la composition conforme à l'invention permet de diminuer les risques associés à la manipulation de peroxyde d'hydrogène. De plus, la concentration en conservateurs des compositions selon la présente invention peut être diminuée par l'apport de composés possédant une fonction alcool qui possèdent également des propriétés de conservateur.

Généralement, la concentration du ou des substrats est comprise entre 0,01% et 60% en poids par rapport au poids total de la composition et de préférence comprise entre 0,05% et 30% en poids par rapport au poids total de la composition.

Les bases d'oxydation classiques peuvent notamment être choisies parmi les paraphénylènediamines, les bis-phénylalkylènediamines, les para-aminophénols, les ortho-aminophénols et les bases hétérocycliques et leurs sels d'addition.

Parmi les paraphénylènediamines, on peut plus particulièrement citer à titre d'exemple, la paraphénylènediamine, la paratoluylènediamine, la 2-chloro paraphénylènediamine, la 2,3-diméthyl paraphénylènediamine, la 2,6-diméthyl paraphénylènediamine, la 2,6-diéthyl paraphénylènediamine, la 2,5-diméthyl paraphénylènediamine, la N,N-diméthyl paraphénylènediamine, la N,N-diéthyl paraphénylènediamine, la N,N-dipropyl paraphénylènediamine, la 4-amino N,N-diéthyl 3-méthyl aniline, la N,N-bis-(β-hydroxyéthyl) paraphénylènediamine, la 4-N,N-bis-(β-hydroxyéthyl)amino 2-méthyl aniline, la 4-N,N-bis-(β-hydroxyéthyl)amino 2-chloro aniline, la 2-β-hydroxyéthyl paraphénylènediamine, la 2-fluoro paraphénylènediamine, la 2-isopropyl paraphénylènediamine, la N-(β-hydroxypropyl) paraphénylènediamine, la 2-hydroxyméthyl paraphénylènediamine, la N,N-diméthyl 3-méthyl paraphénylènediamine, la N,N-(éthyl, β-hydroxyéthyl) paraphénylènediamine, la N-(β,γ-dihydroxypropyl) paraphénylènediamine, la N-(4'-aminophényl) paraphénylènediamine, la N-phényl paraphénylènediamine, la 2-β-hydroxyéthyloxy paraphénylènediamine, la 2-β-acétylaminoéthyloxy paraphénylènediamine, la N-(β-méthoxyéthyl) paraphénylènediamine, la 4 aminophenyl pyrrolidine, le 2 thiényl paraphénylène diamine, le 2-β hydroxyéthylamino 5-amino toluène et leurs sels d'addition avec un acide.

Parmi les paraphénylènediamines citées ci-dessus, la paraphénylènediamine, la paratoluylènediamine, la 2-isopropyl paraphénylènediamine, la 2-β-hydroxyéthyl paraphénylènediamine, la 2-β-hydroxyéthyloxy paraphénylènediamine, la 2,6-diméthyl paraphénylènediamine, la 2,6-diéthyl paraphénylènediamine, la 2,3-diméthyl paraphénylènediamine, la N,N-bis-(β-hydroxyéthyl) paraphénylènediamine, la 2-chloro paraphénylènediamine, la 2-β-acétylaminoéthyloxy paraphénylènediamine, et leurs sels d'addition avec un acide sont particulièrement préférées.

Parmi les bis-phénylalkylènediamines, on peut citer à titre d'exemple, le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthyl-aminophényl) tétraméthylènediamine, la N,N'-bis-(éthyl) N,N'-bis-(4'-amino, 3'-méthylphényl) éthylènediamine, le 1,8-bis-(2,5-diamino phénoxy)-3,6-dioxaoctane, et leurs sels d'addition avec un acide.

Parmi les para-aminophénols, on peut citer à titre d'exemple, le para-aminophénol, le 4-amino 3-méthyl phénol, le 4-amino 3-fluoro phénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, le 4-amino 2-fluoro phénol, et leurs sels d'addition avec un acide.

Parmi les ortho-aminophénols, on peut citer à titre d'exemple, le 2-amino phénol, le 2-amino 5-méthyl phénol, le 2-amino 6-méthyl phénol, le 5-acétamido 2-amino phénol, et leurs sels d'addition avec un acide.

Parmi les bases hétérocycliques, on peut citer à titre d'exemple, les dérivés pyridiniques, les dérivés pyrimidiniques et les dérivés pyrazoliques.

Parmi les dérivés pyridiniques, on peut citer les composés décrits par exemple dans les brevets GB 1 026 978 et GB 1 153 196, comme la 2,5-diamino pyridine, la 2-(4-méthoxyphényl)amino 3-amino pyridine, la 3,4-diamino pyridine, et leurs sels d'addition avec un acide. D'autres bases d'oxydation pyridiniques utiles dans la présente invention sont les bases d'oxydation 3-amino pyrazolo-[1,5-a]-pyridines ou leurs sels d'addition décrits par exemple dans la demande de brevet FR 2801308. A titre d'exemple, on peut citer la pyrazolo[1,5-a]pyridin-3-ylamine ; la 2-acétylamino pyrazolo-[1,5-a] pyridin-3-ylamine ; la 2-morpholin-4-yl-pyrazolo[1,5-a]pyridin-3-ylamine ; l'acide 3-amino-pyrazolo[1,5-a]pyridin-2-carboxylique ; la 2-méthoxy-pyrazolo[1,5-a]pyridine-3-ylamino ; le (3-amino-pyrazolo[1,5-a]pyridine-7-yl)-méthanol ; le 2-(3-amino-pyrazolo[1,5-a]pyridine-5-yl)-éthanol ; le 2-(3-amino-pyrazolo[1,5-a]pyridine-7-yl)-éthanol ; le (3-amino-pyrazolo[1,5-a]pyridine-2-yl)-méthanol ; la 3,6-diamino-pyrazolo[1,5-a]pyridine ; la 3,4-diamino-pyrazolo[1,5-a]pyridine ; la pyrazolo[1,5-a]pyridine-3,7-diamine ; la 7-morpholin-4-yl-pyrazolo[1,5-a]pyridin-3-ylamine ; la pyrazolo[1,5-a]pyridine-3,5-diamine ; la 5-morpholin-4-yl-pyrazolo[1,5-a]pyridin-3-ylamine ; le 2-[(3-amino-pyrazolo[1,5-a]pyridin-5-yl)-(2-hydroxyéthyl)-amino]-éthanol ; le 2-[(3-amino-pyrazolo[1,5-a]pyridin-7-yl)-(2-hydroxyéthyl)-amino]-éthanol ; la 3-amino-pyrazolo[1,5-a]pyridine-5-ol ; 3-amino-pyrazolo[1,5-a]pyridine-4-ol ; la 3-amino-pyrazolo[1,5-a]pyridine-6-ol ; la 3-amino-pyrazolo[1,5-a]pyridine-7-ol ; ainsi que leurs sels d'addition avec un acide.

Parmi les dérivés pyrimidiniques, on peut citer les composés décrits par exemple dans les brevets DE 2 359 399 ; JP 88-169 571 ; JP 05 163 124 ; EP 0 770 375 ou demande de brevet WO 96/15765 comme la 2-hydroxy 4,5,6-triaminopyrimidine, la 2,4-dihydroxy 5,6-diaminopyrimidine, et les dérivés pyrazolo-pyrimidiniques tels ceux mentionnés dans la demande de brevet FR-A-2 750 048 et parmi lesquels on peut citer la pyrazolo-[1,5-a]-pyrimidine-3,7-diamine ; la 2,5-diméthyl pyrazolo-[1,5-a]-pyrimidine-3,7-diamine ; la pyrazolo-[1,5-a)-pyrimidine-3,5-diamine ; la 2,7-diméthyl pyrazolo-[1,5-a]-pyrimidine-3,5-diamine ; le 3-amino pyrazolo-[1,5-a]-pyrimidin-7-ol ; le 3-amino pyrazolo-[1,5-a]-pyrimidin-5-ol ; le 2-(3-amino pyrazolo-[1,5-a]-pyrimidin-7-ylamino)-éthanol, le 2-(7-amino pyrazolo-[1,5-a]-pyrimidin-3-ylamino)-éthanol, le 2-[(3-amino-pyrazolo[1,5-a]pyrimidin-7-yl)-(2-hydroxy-éthyl)-amino]-éthanol, le 2-[(7-amino-pyrazolo[1,5-a]pyrimidin-3-yl)-(2-hydroxy-éthyl)-amino]-éthanol, la 5,6-diméthyl pyrazolo-[1,5-a]-pyrimidine-3,7-diamine, la 2,6-diméthyl pyrazolo-[1,5-a]-pyrimidine-3,7-diamine, la 2, 5, N 7, N 7-tetraméthyl pyrazolo-[1,5-a]-pyrimidine-3,7-diamine, la 3-amino-5-méthyl-7-imidazolylpropylamino pyrazolo-[1,5-a]-pyrimidine et leurs sels d'addition avec un acide et leurs formes tautomères, lorsqu'il existe un équilibre tautomérique.

Parmi les dérivés pyrazoliques, on peut citer les composés décrits dans les brevets DE 3 843 892, DE 4 133 957 et demandes de brevet WO 94/08969, WO 94/08970, FR-A-2 733 749 et DE 195 43 988 comme le 4,5-diamino 1-méthyl pyrazole, le 4,5-diamino 1-(β-hydroxyéthyl) pyrazole, le 3,4-diamino pyrazole, le 4,5-diamino 1-(4'-chlorobenzyl) pyrazole, le 4,5-diamino 1,3-diméthyl pyrazole, le 4,5-diamino 3-méthyl 1-phényl pyrazole, le 4,5-diamino 1-méthyl 3-phényl pyrazole, le 4-amino 1,3-diméthyl 5-hydrazino pyrazole, le 1-benzyl 4,5-diamino 3-méthyl pyrazole, le 4,5-diamino 3-tert-butyl 1-méthyl pyrazole, le 4,5-diamino 1-tert-butyl 3-méthyl pyrazole, le 4,5-diamino 1-(β-hydroxyéthyl) 3-méthyl pyrazole, le 4,5-diamino 1-éthyl 3-méthyl pyrazole, le 4,5-diamino 1-éthyl 3-(4'-méthoxyphényl) pyrazole, le 4,5-diamino 1-éthyl 3-hydroxyméthyl pyrazole, le 4,5-diamino 3-hydroxyméthyl 1-méthyl pyrazole, le 4,5-diamino 3-hydroxyméthyl 1-isopropyl pyrazole, le 4,5-diamino 3-méthyl 1-isopropyl pyrazole, le 4-amino 5-(2'-aminoéthyl)amino 1,3-diméthyl pyrazole, le 3,4,5-triamino pyrazole, le 1-méthyl 3,4,5-triamino pyrazole, le 3,5-diamino 1-méthyl 4-méthylamino pyrazole, le 3,5-diamino 4-(β-hydroxyéthyl)amino 1-méthyl pyrazole, et leurs sels d'addition avec un acide.

Généralement la concentration de la ou des bases d'oxydation est comprise entre 0,0001 et 20%, de préférence entre 0,005 et 6% en poids par rapport au poids total de la composition.

Parmi les coupleurs d'oxydation classiques, on peut notamment citer les métaphénylènediamines, les méta-aminophénols, les métadiphénols, les coupleurs naphtaléniques et les coupleurs hétérocycliques ainsi que leurs sels d'addition.

A titre d'exemple, on peut citer le 2-méthyl 5-aminophénol, le 5-N-(β-hydroxyéthyl)amino 2-méthyl phénol, le 6-chloro-2-méthyl-5-aminophénol, le 3-amino phénol, le 1,3-dihydroxy benzène (ou résorcinol), le 1,3-dihydroxy 2-méthyl benzène, le 4-chloro 1,3-dihydroxy benzène, le 2,4-diamino 1-(β-hydroxyéthyloxy) benzène, le 2-amino 4-(β-hydroxyéthylamino) 1-méthoxybenzène, le 1,3-diamino benzène, le 1,3-bis-(2,4-diaminophénoxy) propane, la 3-uréido aniline, le 3-uréido 1-diméthylamino benzène, le sésamol, le 1-β-hydroxyéthylamino-3,4-méthylènedioxybenzène, l'α-naphtol, le 2 méthyl-1-naphtol, le 6-hydroxy indole, le 4-hydroxy indole, le 4-hydroxy N-méthyl indole, la 2-amino-3-hydroxy pyridine, la 6-hydroxy benzomorpholine la 3,5-diamino-2,6-diméthoxypyridine, le 1-N-(β-hydroxyéthyl)amino-3,4-méthylène dioxybenzène, le 2,6-bis-(β-hydroxyéthylamino)toluène et leurs sels d'addition.

Généralement la concentration du ou des coupleurs d'oxydation est comprise entre 0,0001 et 20%, de préférence entre 0,005 et 6% en poids par rapport au poids total de la composition.

D'une manière générale, les sels d'addition avec un acide utilisables pour les bases d'oxydation et les coupleurs sont notamment choisis parmi les chlorhydrates, les bromhydrates, les sulfates, les citrates, les succinates, les tartrates, les lactates, les tosylates, les benzènesulfonates, les phosphates et les acétates.

Les sels d'addition utilisables dans le cadre de l'invention sont par exemple choisis parmi les sels d'addition avec la soude, la potasse, l'ammoniaque, les amines ou les alcanolamines.

La composition tinctoriale conforme à l'invention peut également renfermer divers adjuvants utilisés classiquement dans les compositions pour la teinture des cheveux, tels que des agents antioxydants, des agents de pénétration, des agents séquestrants, des parfums, des tampons, des agents dispersants, des tensioactifs, des polymères épaississants, des agents de conditionnement tels que par exemple des silicones volatiles ou non volatiles, modifiées ou non modifiées, des polymères cationiques, des cations, des agents filmogènes, des céramides, des agents conservateurs, des agents opacifiants, des vitamines ou provitamines, des colorants d'oxydation, des colorants directs autres que des colorants directs cationiques azoïques, méthiniques ou azométhiniques.

Les adjuvants ci-dessus sont en général présents en quantité comprise pour chacun d'eux entre 0,01 et 20 % en poids par rapport au poids de la composition.

Bien entendu, l'homme de l'art veillera à choisir ce ou ces éventuels composés complémentaires de manière telle que les propriétés avantageuses attachées intrinsèquement à la composition de teinture d'oxydation conforme à l'invention ne soient pas, ou substantiellement pas, altérées par la ou les adjonctions envisagées.

Le milieu approprié pour la teinture appelé aussi support de teinture est généralement constitué par de l'eau ou par un mélange d'eau et d'au moins un solvant organique pour solubiliser les composés qui ne seraient pas suffisamment solubles dans l'eau. Ce solvant peut être le cas échéant un substrat de l'enzyme comme l'éthanol, l'isopropanol. Cela peut être également un composé non substrat de l'enzyme tel que les éthers de polyols comme le 2-butoxyéthanol, le propylèneglycol, le monométhyléther de propylèneglycol, le monoéthyléther et le monométhyléther du diéthylèneglycol, le phénoxyéthanol, et leurs mélanges.

Les solvants peuvent être présents dans des proportions de préférence comprises entre 1 et 40 % en poids environ par rapport au poids total de la composition tinctoriale, et encore plus préférentiellement entre 5 et 30 % en poids environ.

Le pH de la composition tinctoriale conforme à l'invention est généralement compris entre 6 et 11 environ, et de préférence entre 7 et 10 environ. Il peut être ajusté à la valeur désirée au moyen d'agents acidifiants ou alcalinisants habituellement utilisés en teinture des fibres kératiniques ou bien encore à l'aide de systèmes tampons classiques.

Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule (III) suivante : 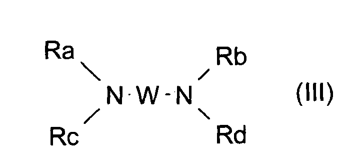 dans laquelle W est un reste propylène éventuellement substitué par un groupement hydroxyle ou un radical alkyle en C₁-C₄ ; Ra, Rb, Rc et Rd, identiques ou différents, représentent un atome d'hydrogène, un radical alkyle en C₁-C₄ ou hydroxyalkyle en C₁-C₄.

La composition tinctoriale peut se présenter sous des formes diverses, telles que sous forme de liquides, de crèmes, de gels, ou sous toute autre forme appropriée pour réaliser une teinture des fibres kératiniques, et notamment des cheveux humains.

Lorsque les colorants d'oxydation et la ou les alcool oxydases sont présents au sein de la même composition prête à l'emploi, ladite composition est de préférence exempte d'oxygène gazeux de manière à éviter toute oxydation prématurée du ou des colorants d'oxydation.

L'invention a également pour objet un procédé de teinture des fibres kératiniques, et en particulier des fibres kératiniques humaines telles que les cheveux, tel qu'on applique sur ces fibres au moins une composition pour la teinture selon l'invention, pendant une durée suffisante pour développer la coloration désirée.

Au contact de l'oxygène de l'air, la couleur est révélée par la mise en présence de l'enzyme alcool oxydase et de son substrat.

La composition est appliquée sur les fibres kératiniques. Après un temps de pose de 3 à 60 minutes environ, de préférence 5 à 40 minutes environ, les fibres kératiniques sont rincées, lavées au shampooing, rincées à nouveau puis séchées.

Lorsque la composition pour la teinture est une composition sous une forme prête à l'emploi, elle comprend, dans un milieu approprié pour la teinture des fibres kératiniques, au moins un précurseur de colorant d'oxydation, au moins une enzyme alcool oxydase, au moins un substrat pour ladite enzyme et au moins un colorant direct cationique azoïque, méthinique ou azométhinique, l'ensemble est alors stocké sous forme anaérobie, exempte d'oxygène gazeux.

Selon une variante, ce procédé comporte une étape préliminaire consistant à stocker sous forme séparée, d'une part une composition (A) comprenant, dans un milieu approprié pour la teinture des fibres kératiniques, au moins un précurseur de colorant d'oxydation, et d'autre part, une composition (B) renfermant, dans un milieu approprié pour la teinture des fibres kératiniques, au moins une enzyme alcool oxydase, la composition (A) et/ou la composition (B) contenant au moins un substrat pour ladite enzyme, et la composition (A) et/ou la composition (B) contenant au moins un colorant direct cationique azoïque, méthinique ou azométhinique puis à procéder au mélange des compositions (A) et (B) au moment de l'emploi avant d'appliquer ce mélange sur les fibres kératiniques.

De préférence, ce procédé comporte une étape préliminaire consistant à stocker sous forme séparée, d'une part une composition (A) comprenant, dans un milieu approprié pour la teinture des fibres kératiniques, au moins un précurseur de colorant d'oxydation, au moins un substrat pour l'enzyme alcool oxydase et au moins un colorant direct cationique azoïque, méthinique ou azométhinique et d'autre part, une composition (B) renfermant, dans un milieu approprié pour la teinture des fibres kératiniques, au moins une enzyme alcool oxydase, puis à procéder au mélange des compositions (A) et (B) au moment de l'emploi avant d'appliquer ce mélange sur les fibres kératiniques.

La couleur peut être révélée à pH acide, neutre ou alcalin. Dans le cas où le procédé est mis en oeuvre au moyen d'une composition (A) comprenant au moins un précurseur de colorant d'oxydation, au moins un substrat pour ladite enzyme et au moins un colorant direct cationique azoïque, méthinique ou azométhinique et d'une composition (B) renfermant au moins une enzyme alcool oxydase, l'enzyme peut être ajoutée à la composition de l'invention juste au moment de l'emploi ou peut être mis en oeuvre à partir d'une composition la contenant, appliquée simultanément ou séquentiellement à la composition de l'invention.

Cette composition (dite composition oxydante) peut également renfermer divers adjuvants utilisés classiquement dans les compositions pour la teinture des cheveux et tels que définis précédemment.

Le pH de la composition B dite oxydante est tel qu'après mélange avec la composition tinctoriale A, le pH de la composition résultante appliquée sur les fibres kératiniques varie de préférence entre 6 et 11 environ, et encore plus préférentiellement entre 7 et 10. Il peut être ajusté à la valeur désirée au moyen d'agents acidifiants ou alcalinisants habituellement utilisés en teinture des fibres kératiniques et tels que définis précédemment.

De préférence, l'application de la composition selon l'invention est effectuée à une température comprise entre la température ambiante et 220°C, de préférence entre la température ambiante et 60°C.

Un autre objet de l'invention est un dispositif à plusieurs compartiments ou "kit" de teinture dans lequel un premier compartiment renferme la composition (A) telle que définie ci-dessus et un deuxième compartiment renferme la composition (B) telle que définie ci-dessus. Ce dispositif peut être équipé d'un moyen permettant de délivrer sur les cheveux le mélange souhaité, tel que les dispositifs décrits dans le brevet FR-2 586 913 au nom de la demanderesse.

Les exemples qui suivent servent à illustrer l'invention sans toutefois présenter un caractère limitatif.

### EXEMPLE

La demanderesse a préparé la composition suivante conformément à l'invention.

| | |
|---|---|
| Acid Black 1 (colorant direct cationique azoïque, méthinique ou azométhinique) | 0,4 g |
| Ethanol (substrat donneur) | 25g |
| Paraphénylènediamine (précuseur de coloration) | 3. 10⁻³ mole |
| Métaaminophénol (coupleur) | 3. 10⁻³ mole |
| Alcool oxydase | 20000unités |
| 2-amino-2-méthyl-1-propanol | Qs pH 7 |
| Eau distillé | qsp 100g |

L'alcool oxydase utilisée est celle commercialisée par la société Biozyme Laboratories sous forme liquide à la concentration de 1980 unités/ml.

L'unité U correspond à la quantité d'enzyme conduisant à la génération de 1 µmole de peroxyde d'hydrogène par minute à pH 7,5 (tampon phosphate 100mM) et à une température de 25°C.

On applique les compositions ci-dessus sur des mèches de cheveux gris naturels et permanentés à 90% de blancs et on laisse poser pendant 30 minutes. Le rapport de bain est fixé à 5. L'alcool oxydase est ajoutée extemporanément. Les cheveux ont ensuite été rincés, lavés avec un shampooing standard, puis séchés.

## Revendications

1. Composition pour la teinture des fibres kératiniques, en particulier des fibres kératiniques humaines telles que les cheveux, contenant, dans un milieu approprié pour la teinture, au moins les composés suivants : un précurseur de colorant d'oxydation ; au moins une enzyme alcool oxydase ; au moins un substrat, portant une fonction alcool, pour ladite enzyme et au moins un colorant direct cationique azoïque, méthinique ou azométhinique, ledit substrat pouvant être substitué en tout ou en partie par le précurseur de colorant d'oxydation dans le cas où ledit précurseur porte au moins une fonction alcool aliphatique ou aromatique.

2. Composition selon la revendication 1, **caractérisée en ce qu'**elle contient des colorants directs cationiques azoïques, méthiniques ou azométhiniques choisis parmi les composés suivants :
• Les composés de formule (I) : 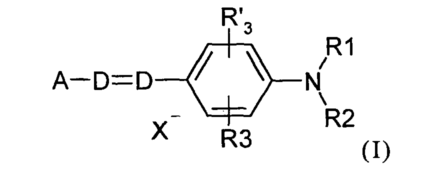 dans laquelle D représente un atome d'azote ou le groupement CH,
R₁ et R₂, identiques ou différents entre eux, représentent un atome d'hydrogène, un radical alkyle en C₁-C₄ pouvant être substitué par un radical CN, OH, ou NH₂ ou forment avec un atome de carbone du cycle benzènique un hétérocycle azoté pouvant comporter en plus un atome d'oxygène ou un atome d'azote, pouvant être substitué par un ou plusieurs radicaux alkyle en C₁-C₄ ; R₁ et R₂ peuvent former un radical 4'-aminophényle ;
R₃ et R'₃, identiques ou différents, représentent un atome d'hydrogène ou d'halogène choisi parmi le chlore, le brome, l'iode et le fluor, un radical cyano, alcoxy en C₁-C₄ ou acétyloxy,
X⁻ représente un anion de préférence choisi parmi le chlorure, le méthylsulfate et l'acétate,
A représente un groupement choisi par les structures A1 à A19 suivantes : 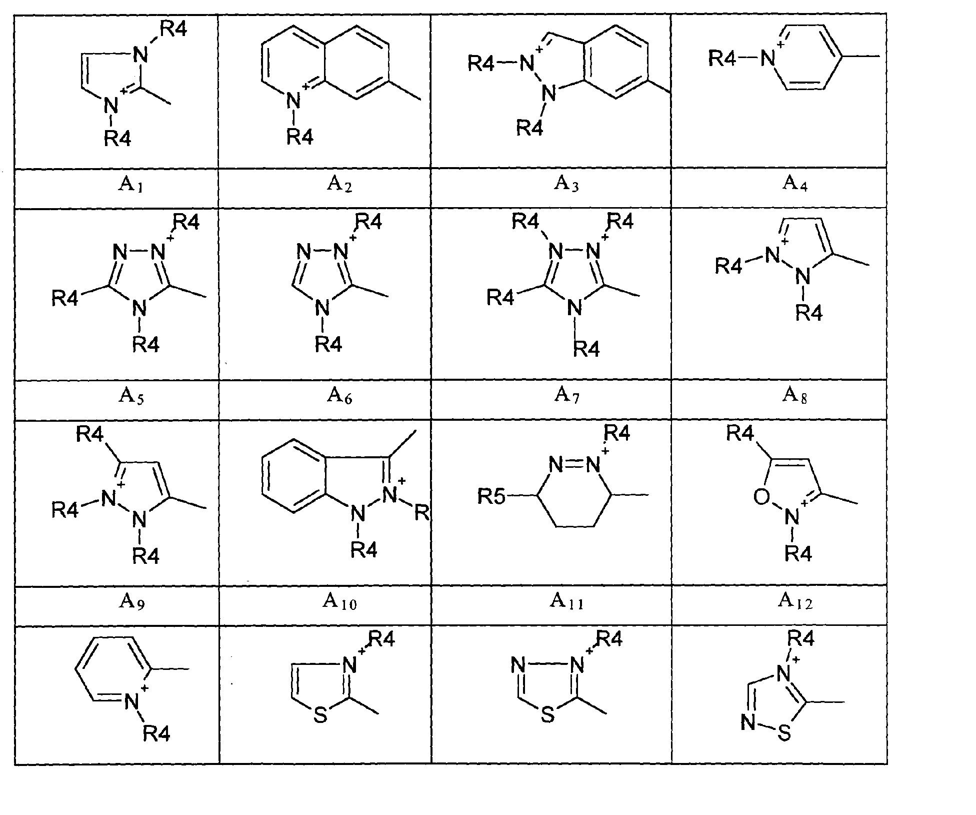 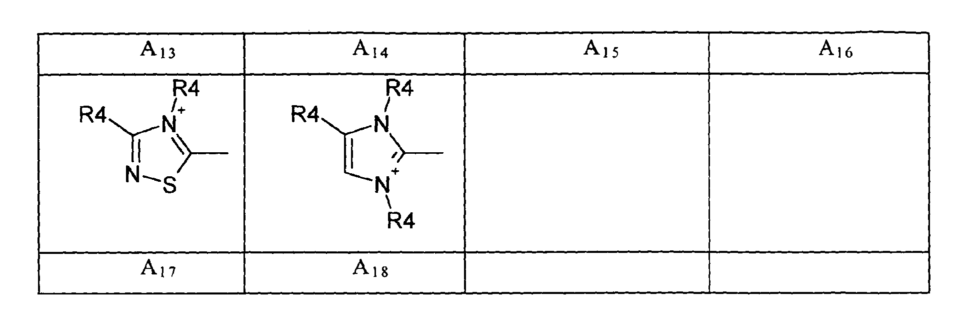
R₄ représente un radical alkyle en C₁-C₄ pouvant être substitué par un radical hydroxyle et R₅ représente un radical alcoxy en C₁-C₄ ;
• Les composés de formule (II) : 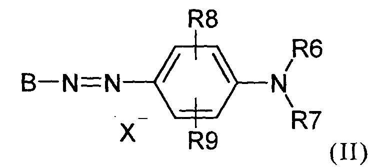 dans laquelle R₆ représente un atome d'hydrogène ou un radical alkyle en C₁-C_{4 ;}
R₇ représente un atome d'hydrogène, un radicale alkyle pouvant être substitué par un radical CN ou par un groupement amino, un radical 4'-aminophényle ou forme avec R₆ un hétérocycle azoté pouvant comporter en plus un atome d'oxygène et/ou un atome d'azote pouvant être substitué par un radical en C₁-C₄ ;
R₈ et R₉, identiques ou différents, représentent un atome d'hydrogène, un atome d'halogène tel que le brome, le chlore, l'iode ou le fluor, un radical alkyle en C₁-C₄ ou alcoxy en C₁-C₄, un radical CN ;
X⁻ représente un anion de préférence choisi parmi le chlorure, le méthylsulfate et l'acétate ;
B représente un groupement choisi parmi les structures B₁ à B₆ suivantes : 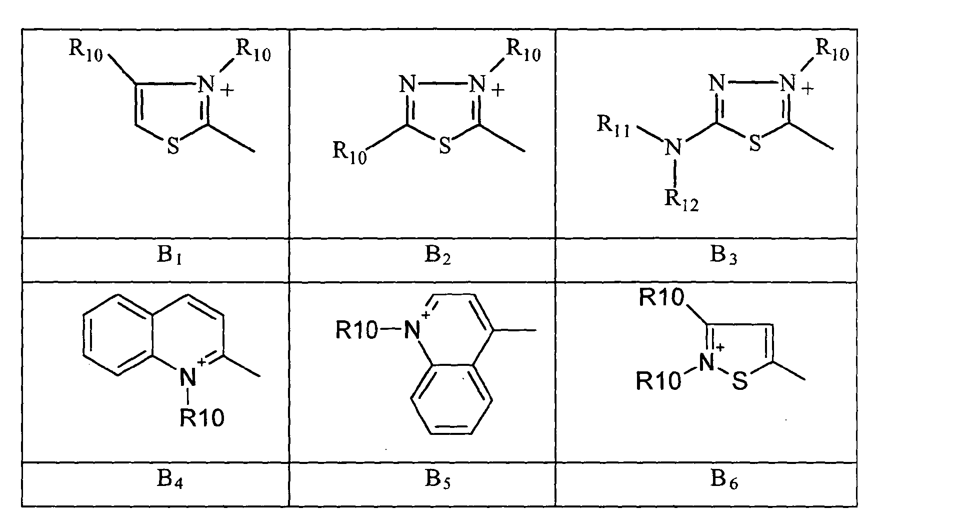 dans lesquelles R₁₀ représente un radical alkyle en C₁-C₄ ;
R₁₁ et R₁₂, identiques ou différents, représentent un atome d'hydrogène ou un radical alkyle en C₁-C₄ ;
• Les composés de formule (III) et (III') : 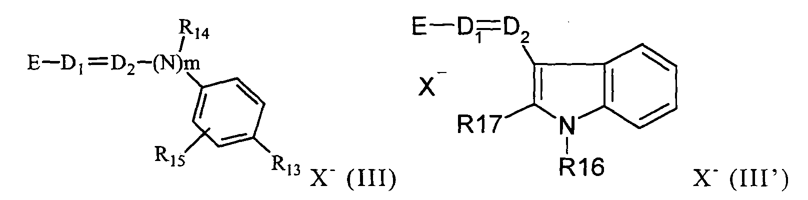 dans lesquelles, R₁₃ représente un atome d'hydrogène, un radical alcoxy en C₁-C₄, un atome d'halogène tel que le brome, le chlore, l'iode ou le fluor ou un radical amino ;
R₁₄ représente un atome d'hydrogène, un radical alkyle en C₁-C₄ ou forme avec un atome de carbone du cycle benzènique un hétérocycle éventuellement oxygéné et/ou azoté substitué par un ou plusieurs groupements en C₁-C₄ ;
R₁₅ représente un atome d'hydrogène ou d'halogène tel que le brome, le chlore, l'iode ou le fluor ;
R₁₆ et R₁₇, identiques ou différents, représentent un atome d'hydrogène ou un radical alkyle en C₁-C₄ ;
D₁ et D₂, identiques ou différents, représentent un atome d'azote ou le groupement CH ;
m est égal à 0 ou 1 ;
étant entendu que lorsque R₁₃ représente un groupement amino non substitué alors D₁ et D₂ représentent simultanément un groupement -CH et m vaut 0 ;
X- représente un anion de préférence choisi parmi le chlorure, le méthyl sulfate et l'acétate ;
E représente un groupement choisi parmi les structures E₁ à E₈ suivantes : 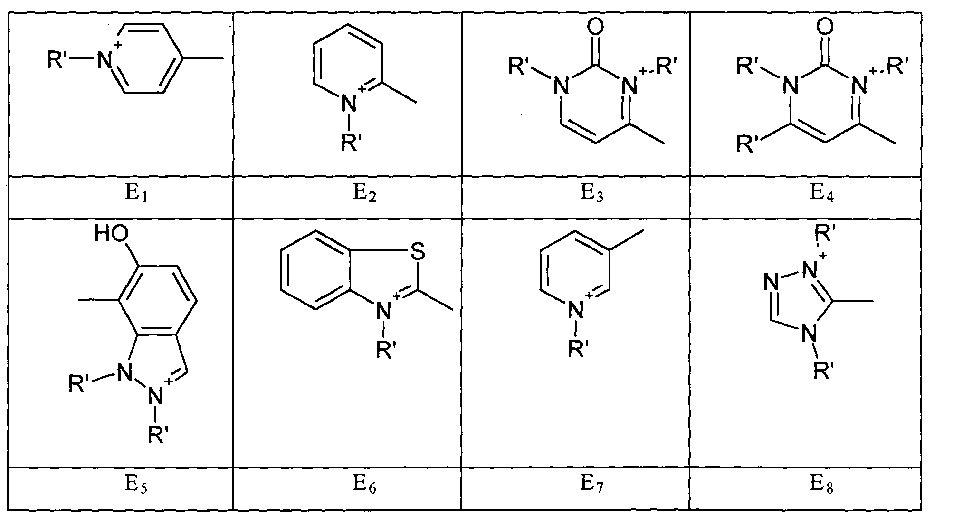 dans lesquelles R' représente un radical alkyle en C₁-C₄,
Lorsque m vaut 0 et que D₁ représente un atome d'azote, alors E peut également désigner un groupement de structure E₉ suivante : 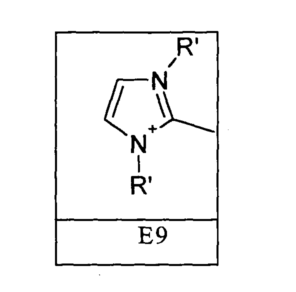 dans laquelle R' représente un radical alkyle en C₁-C₄ ;
• Les composés de formule (IV) :
W₁-W₂-N=N-W₃-L-W₄-N=N-W₅-W₆ (IV)
Dans laquelle W₁ et W₆ représentent, indépendamment l'un de l'autre, un radical NR'₁R'₂ ;
W₂ et W₅ représentent indépendamment l'un de l'autre, un groupement aromatique carboné, pyridinique ou pyridazinyle de formule (IVa) : 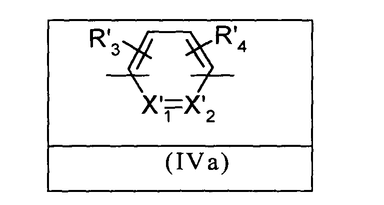
W₃ et W₄ représentent, indépendamment l'un de l'autre, un radical hétéroaromatique représenté par les formules (IVb) et (IVc) suivantes : 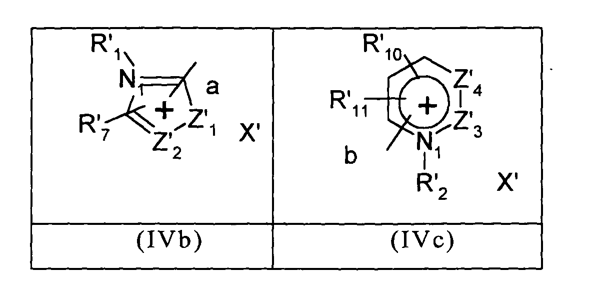 dans lesquelles X'₁ représente un atome d'azote ou un radical CR'₅
X'₂ représente un atome d'azote ou un radical CR'₆
Z'₁ représente un atome d'oxygène, de soufre ou un radical NR'₈
Z'₂ représente un atome d'azote ou un radical CR'₉
Z'₃ représente un atome d'azote ou un radical CR'₁₂
Z'₄ représente un atome d'azote ou un radical CR'₁₃
N₁ du cycle à 5 chaînons de la formule (IVb) est relié au groupement L de la formule (IV) via le groupe R'₁ et la liaison *a* du même cycle à 5 chaînons est reliée au groupement azoïque de la formule (IV) ; la liaison *b* du cycle à 6 chaînons de la formule (IVc) est reliée au groupement azoïque de la formule (IV) et N₁ du cycle à 6 chaînons de la formule (IVc) est reliée au groupe L de la formule (IV) via le groupe R'₂ ;
L, R'₁, R'₂, R'₃, R'₄, R'₅, R'₆, R'₇, R'₉, R'₁₀, R'₁₁, R'₁₂ et R'₁₃ représentent ensemble ou indépendamment les uns des autres une chaîne hydrocarbonée en C₁-C₁₆ linéaire ou ramifiée pouvant former un ou plusieurs cycles carbonés comportant de 3 à 6 chaînons et pouvant être saturés ou insaturés, dont un ou plusieurs carbones de la chaîne carbonée peuvent être remplacés par un atome d'oxygène, d'azote, de soufre ou par un groupement SO₂ et dont les atomes de carbone peuvent être indépendamment les uns des autres substitués par un ou plusieurs atomes d'halogène ;
R'₁, R'₂, R'₃, R'₄, R'₅, R'₆, R'₇, R'₉, R'₁₀, R'₁₁, R'₁₂ et R'₁₃ peuvent représenter l'atome d'hydrogène. L, R'₁, R'₂, R'₃, R'₄, R'₅, R'₆, R'₇, R'₉,
R'₁₀, R'₁₁, R'₁₂ et R'₁₃ ne comportent pas de liaison peroxyde, ni de radicaux diazo ou nitroso et L est un radical divalent ;
R'₈ représente un radical alkyle en C₁-C₈ linéaire ou ramifié, éventuellement substitué par un ou plusieurs radicaux choisis parmi les radicaux hydroxy, alcoxy en C₁-C₂, (poly)-hydroxyalcoxy en C₂-C₄, amino, (di)alkylamino en C₁-C₂, carboxy ou sulfonique, un radical phényle éventuellement substitué ;
R'₇ avec R'₉, R'₁₀ avec R'₁₁, R'₁₂ avec R'₁₃ peuvent former un cycle aromatique carboné tel qu'un phényle ;
X' représente un anion organique ou minéral ;
• Les composés de formule (V) :
W₇-N=N-W₈-N=N-W₉ (V)
dans laquelle :
W₇ et W₉ représentent indépendamment l'un de l'autre un radical hétéroaromatique représenté par les formules Va et Vb suivantes : 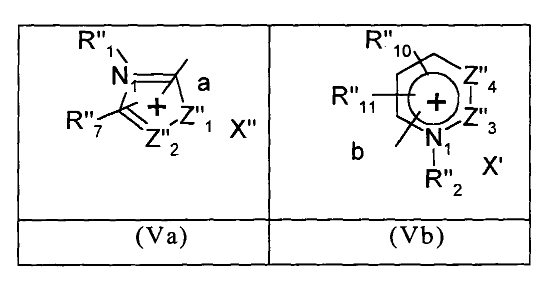
W₈ représente un groupement aromatique carboné pyridinique ou pyridazinyle de formule (Vc) : 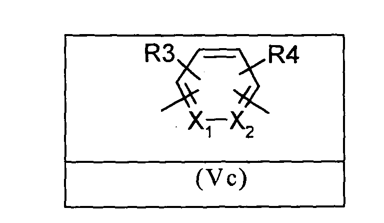
dans lesquelles :
X₁ représente un atome d'azote ou un radical CR''₅
X₂ représente un atome d'azote ou un radical CR''₆
Z''₁ représente un atome d'oxygène, de soufre ou un radical NR''₈
Z''₂ représente un atome d'azote ou un radical CR''₉,
Z''₃ représente un atome d'azote ou un radical CR''₁₂
Z''₄ représente un atome d'azote ou un radical CR''₁₃
la liaison a du cycle cationique à 5 chaînons de la formule (Va) est reliée au groupement azoïque de la formule (V) ;
la liaison b du cycle cationique à 6 chaînons de la formule (Vb) est relié au groupement azoïque de la formule (V) ;
R₃, R₄, R''₁, R''₂, R''₅, R''₆, R''₇, R''₈, R''₉, R''₁₀, R''₁₁, R''₁₂, R''₁₃ représentent, ensemble ou indépendamment l'un de l'autre, un atome d'hydrogène, une chaîne hydrocarbonée en C₁-C₁₆, linéaire ou ramifiée, saturée ou insaturée, pouvant former un ou plusieurs cycles carbonés comportant de 3 à 6 chaînons, dont un ou plusieurs atomes de carbone de la chaîne carbonée peuvent être remplacés par un atome d'oxygène, d'azote ou de soufre ou par un groupement SO₂ et dont les atomes de carbone peuvent être indépendamment les uns des autres substitués par un ou plusieurs atomes d'halogène ;
R₃, R₄, R''₁, R''₂, R''₅, R''₆, R''₇, R''₈, R''₉, R''₁₀, R''₁₁, R''₁₂, R''₁₃ ne comportent pas de liaison peroxyde, ni de radicaux diazo ou nitroso ;
R''₇ et R''₉, R''₁₀ et R''₁₁, R''₁₂ et R''₁₃ peuvent former un cycle carboné tel un phényle ;
X'' est un anion organique ou minéral par exemple choisi parmi un halogénure, un hydroxyde, un sulfate, un hydrogénosulfate, un alkylC₁-C₆sulfate, un acétate, un tartrate, un oxalate, un alkylC₁-C₆sulfonate, un arylsulfonate substitué ou non substitué par un radical alkyl en C₁-C₄ ;
• Les composés de formule (VIa) et (VIb) : 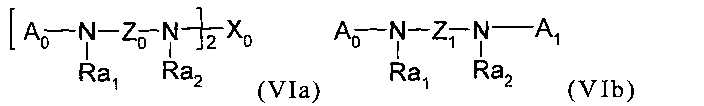 où A₀ et A₁, indépendamment l'un de l'autre désignent : 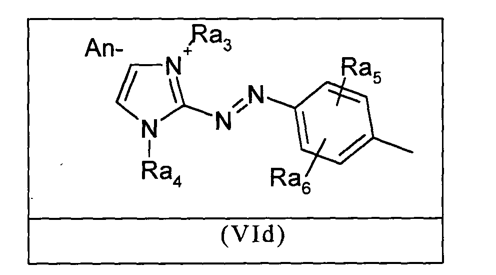
Z₀ désigne un radical aliphatique
Z₁ désigne un radical alkyle
Rₐ₁ et Rₐ₂, indépendamment l'un de l'autre désignent un atome d'hydrogène ou un radical (C₁-C₄)alkyl ou (C₁-C₄)alkyl substitué par un ou plusieurs atomes d'halogène, un radical hydroxyl, carboxyl, cyano, un radical (C₁-C₄)alcoxy, un radical (C₁-C₄)alcoxy substitué par un ou plusieurs radicaux hydroxyl, ou (C₁-C₄)alcoxy, un radical amino, alkylamino, dialkylamino, aminocarbonyl, phényl, phénoxy, ou phénylaminocarbonyl dans lequel le radical phényl est non substitué ou substitué par un radical (C₁-C₄)alkyl, (C₁-C₄)alcoxy ou phénoxy ;
Rₐ₁ et Rₐ₂ peuvent aussi former ensemble avec les deux atomes d'azote qui les portent et le radical Z₀ un radical pipérazinique ;
X₀ est un radical de pontage choisi parmi -CO-, -COCH₂-, -CH₂CO-, -COCO-, 1,4-dicarbonylphényl, -CH₂-CH₂-, ou une triazine de formules suivantes : 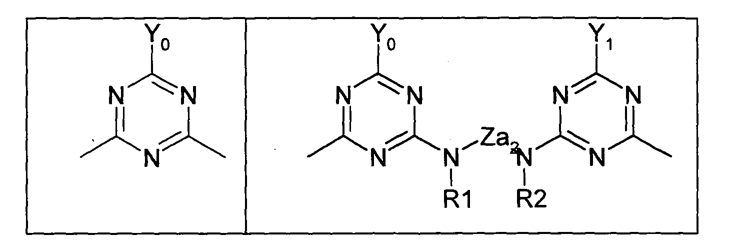 Dans lesquelles Y₀ et Y₁, indépendamment l'un de l'autre, désignent un atome d'halogène ou un radical hydroxyl, ou amino, ou monoalkylamino, ou dialkylamino, ou 1-pipéridino, ou morpholino, ou 1-pipérazino, le radical pipérazino étant non substitué ou substitué sur l'atome d'azote non attaché au cycle triazine par un radical (C₁-C₄)alkyl, lesdits radicaux alkyls étant non substitués ou substitués par hydroxyl, amino, mono-(C₁-C₄)alkylamino ou di(C₁-C₄)alkylamino ;
Zₐ₂ désigne un radical C₂-C₈alkylène ou forme avec les deux atomes d'azote adjacents et les radicaux R₁ et R₂ un cycle pipérazinique ;
Dans le radical de formule (VId) :
Rₐ₃ et Rₐ₄, indépendamment l'un de l'autre, désignent un atome d'hydrogène, ou un radical (C₁-C₄)alkyl ou C₁-C₄alkyl substitué par un ou plusieurs atomes d'halogène, un radical hydroxyl, carboxyl, cyano, un radical (C₁-C₄)alcoxy, un radical (C₁-C₄)alcoxy substitué par un radical hydroxyl ou (C₁-C₄)alcoxy, un radical amino, alkylamino, dialkylamino, aminocarbonyl, phényle, phénoxy ou phénylaminocarbonyl dans lequel le radical phényl est non substitué ou substitué par un radical (C₁-C₄)alkyl, (C₁-C₄)alcoxy ou phénoxy ;
Rₐ₅ et Rₐ₆, indépendamment l'un de l'autre, désignent un atome d'hydrogène, un radical C₁-C₄alkyl ou C₁-C₄alcoxy, éventuellement substitués par un radical hydroxyl, carboxyl, halogène, cyano, (C₁-C₄)alcoxy éventuellement substitué par un radical hydroxyl, ou (C₁-C₄)alcoxy, un radical amino, alkylamino, dialkylamino, aminocarbonyl, phényl, phénoxy, ou phénylaminocarbonyl, dans lequel le radical phényl est non substitué ou substitué par un radical (C₁-C₄)alkyl, (C₁-C₄)alcoxy ou phénoxy ,
An⁻ désigne un anion ;
• Les composés de formule (VIc) : 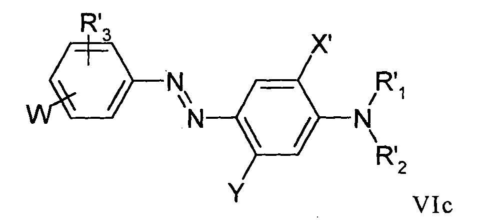 dans laquelle le nombre de charge cationique est de 2 ;
X'et Y, indépendamment l'un de l'autre, désignent un atome d'hydrogène, halogène, (C₁-C₄)alkyl, (C₁-C₄)alcoxy, (C₁-C₄)alkylcarbonylamino, arylcarbonylamino, uréido, ou aryluréido ;
R'₁ désigne un atome d'hydrogène, un radical alkyl ou aryl substitués, un radical alkyl ou aryl non substitués ou la même désignation que R'₂ ;
R'₂ est un radical du type : 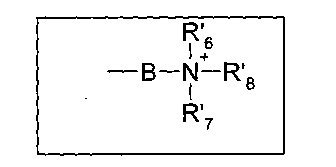 Dans laquelle B désigne un radical alkylène linéaire ou ramifié ;
R'₆ désigne un atome d'hydrogène ou alkyl substitué ou non substitué ;
R'₇ et R'₈, indépendamment l'un de l'autre désignent un radical alkyl substitué ou non substitué ;
R'₆ et R'₇ ensemble avec l'azote, forment un cycle à 5, 6 ou 7 chaînons substitués ou non substitués, pouvant contenir d'autres hétéroatomes ou bien R'₆ et R'₇ et R'₈ forment ensemble un cycle pyridinium ;
R'₃ désigne un atome d'hydrogène, d'halogène (C₁-C₄)alkyl, (C₁-C₄)alcoxy ;
W est un radical de formule : 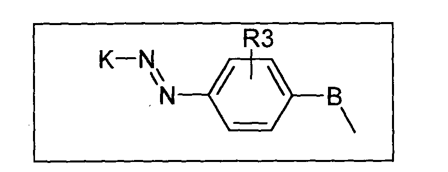 où K désigne un composé de couplage choisi parmi ceux de formule (f), (g) ou (h) suivantes : 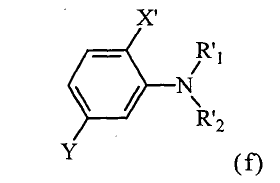 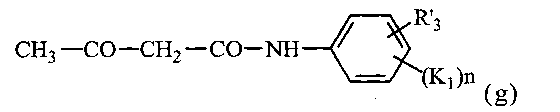 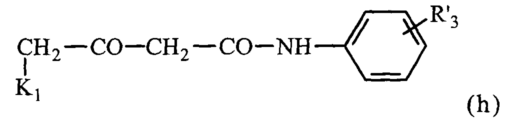 dans lesquelles,
- X' et Y, indépendamment l'un de l'autre, désignent un atome d'hydrogène, un atome d'halogène, un groupe alkyl en C₁-C₄, un alcoxy en C₁-C₄, un alkyl(C₁-C₄)carbonylamino, un arylcarbonylamino, un uréido ou un aryluréido,
- R'₁ désigne un atome d'hydrogène, un radical alkyl substitué, un radical aryl substitué, un radical alkyl non substitué, un radical aryl non substitué, ou la même signification que le substituant R'₂,
- R'₂ est un radical de formule suivante : 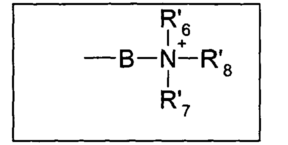 dans laquelle B désigne un radical alkylène linéaire ou ramifié ;
R'₆ désigne un atome d'hydrogène ou alkyl substitué ou non substitué ;
R'₇ et R'₈, indépendamment l'un de l'autre désignent un radical alkyl substitué ou non substitué ;
R'₆ et R'₇ ensemble avec l'azote, forment un cycle à 5, 6 ou 7 chaînons substitués ou non substitués, pouvant contenir d'autres hétéroatomes ou bien R'₆ et R'₇ et R'₈ forment ensemble un cycle pyridinium ;
R'₃ désigne un atome d'hydrogène, d'halogène, (C₁-C₄)alkyl, (C₁-C₄)alcoxy ;
- n est égal à 1 ou 2,
- K₁ désigne le radical de formule -N⁺R'₆R'₇R'₈ ;
R3 a les mêmes significations que Rₐ₃,
B désigne un radical de pontage choisi parmi les radicaux suivants : 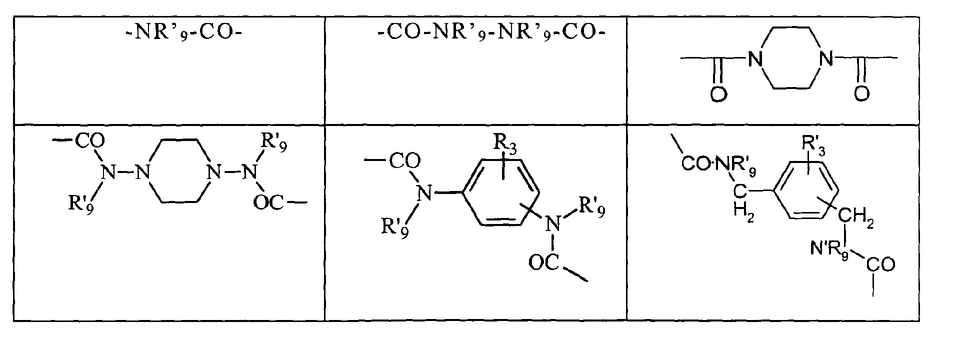 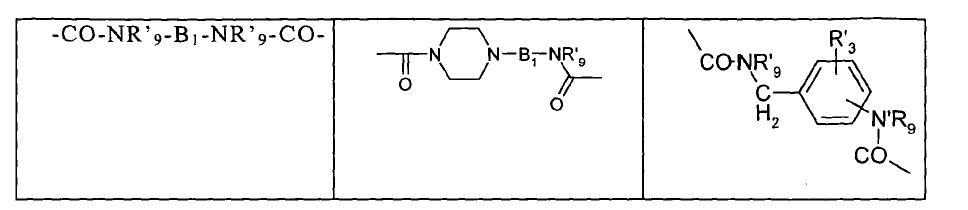
Dans lesquelles R'₉ désigne un atome d'hydrogène, ou un radical alkylène en C₁-C₂ ; B₁ désigne un radical (C₂-C₄)alkylène non substitué
ou substitué, le radical alkylène étant linéaire ou ramifié et pouvant être interrompu par un ou plusieurs groupements choisis parmi -NR'₉-, -O-, -S- ;
• Les composés de formule (VIIIa) :
Z₁-N=N-A₁-(A₃)ₙ-Z₂ (VIIIa) ou Z₁-N=N-A₂ (VIIIb)
Dans lesquelles n est égal à 0 ou 1,
Z₁ représente un radical hétéroaromatique cationique à 5 ou 6 chaînons de formules suivantes : 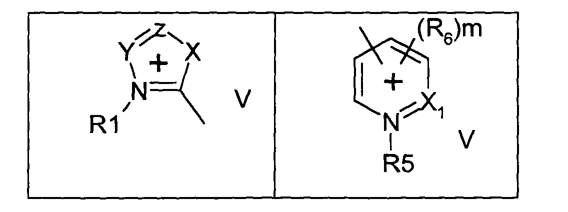
où X représente NR₃, S, ou O, Z représente CR₂ ou N et Y représente CR₄ ou N avec les conditions suivantes :
• lorsque X représente NR₃ ou O et Z représente CR₂ alors Y représente CR₄ ou N,
• lorsque X représente S alors Z représente N ou Y représente N,
• lorsque X représente S et Z représente N alors Y représente CR₄,
X₁ représente CR₆ ou N,
m est un nombre entier égal à 0, 1, 2 ou 3,
R₁, R₃, R₅ représentent, indépendamment l'un de l'autre, une chaîne hydrocarbonée en C₁-C₁₀, saturée ou insaturée, linéaire ou ramifiée pouvant former un cycle carboné ayant de 5 à 7 chaînons éventuellement aromatique, un ou plusieurs atomes de carbone pouvant être remplacée par un atome d'oxygène, d'azote, d'halogène de soufre ou par un groupement SO₂ à l'exception du carbone relié à l'atome d'azote des 2 cycles précédents. Les radicaux R₁ et R₃ ou R₅ ne comportant pas de liaison peroxyde, ni de radicaux diazo, nitro ou nitroso,
R₂, R₄ et R₆ représentent, indépendamment l'un de l'autre, un atome d'hydrogène, une chaîne hydrocarbonée en C₁-C₁₀, saturée ou insaturée, linéaire ou ramifiée pouvant former un cycle carboné ayant de 5 à 7 chaînons, éventuellement aromatique ; un ou plusieurs atomes de carbone pouvant être remplacés par un ou plusieurs atomes d'oxygène, d'azote, de soufre, ou par un groupement SO₂ les radicaux R₂, R₄ ou R₆ ne comportant pas de liaison peroxyde, ni de radicaux diazo, nitro ou nitroso, les radicaux R₂ et R₄ peuvent former ensemble un cycle aromatique carboné,
V représente un anion organique ou minéral.
A₁ et A₃ représentent, indépendamment l'un de l'autre, un radical divalent de formule (VIIIc) ou (VIIId) 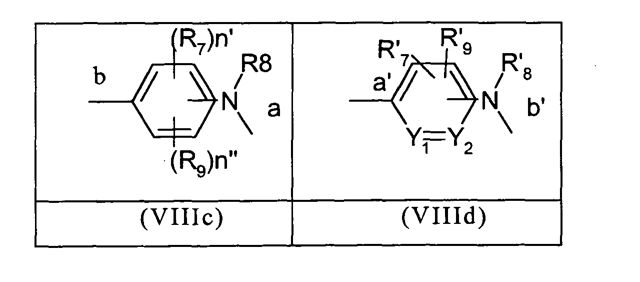
n' est un nombre entier égal à 0, 1, 2 ou 3.
n" est un nombre entier égal à 0 ou 1.
Y₁=Y₂ représente C=N ou N=N ;
lorsque n vaut 0, alors soit la liaison a du groupement A₁ de la formule (VIIIc) est reliée à la fonction Z₂ de la formule (VIIIa), soit la liaison b' du groupement A₁ de la formule (VIIId) est reliée à la fonction Z₂ de la formule (VIIIa)
lorsque n vaut 1, les composés de formule (VIIIa) correspondent aux quatre sous structures :
Z₁-N=N-(VIIIc)-(VIIId)-Z₂
Z₁-N=N-(VIIIc)-(VIIIc)-Z₂
Z₁-N=N-(VIIId)-(VIIIc)-Z₂
Z₁-N=N-(VIIId)-(VIIId)-Z₂
Les radicaux divalents (VIIIc) et (VIIId) étant reliés entre eux ou à Z₂
ou à l'atome d'azote du groupement azoïque via les bras de liaison a, a', b, b'.
R₈ et R'₈ représentent indépendamment l'un de l'autre un groupe non cationique choisi parmi un atome hydrogène, une chaîne hydrocarbonée en C₁-C₁₀, linéaire ou ramifiée pouvant former un cycle carboné ayant de 5 à 7 chaînons, éventuellement aromatique, un ou plusieurs atomes de carbone de la chaîne hydrocarbonée pouvant être remplacés par un ou plusieurs atomes d'oxygène, d'azote, de soufre, ou par un groupement SO₂ à l'exception du carbone relié à l'atome d'azote. Les radicaux R₈ ou R'₈ ne comportant pas de liaison peroxyde, ni de radicaux diazo, nitro ou nitroso ;
R₇, R₉, R'₇ et R'₉ représentent indépendamment l'un de l'autre un groupe non cationique tel que défini pour R₂ ou un groupe cationique Z₃, à la condition qu'un seul des groupes R₇, R₉, R'₇ et R'₉ soit cationique ; R₇ avec R₈, respectivement R'₇ avec R,₈ peuvent former ensemble un hétérocycle à 5 ou 6 chaînons saturé ;
Z₃ est un groupe cationique représenté par la formule (VIIIe) suivante :
-(B)_{n'''}-D (VIIIe)
dans laquelle :
B représente une chaîne hydrocarbonée comportant de 1 à 15 atomes de carbone, linéaire ou ramifiée, pouvant former un ou plusieurs cycles comportant de 3 à 7 chaînons éventuellement aromatiques, et dont un ou plusieurs atomes de carbone peuvent être remplacés par un atome d'oxygène, d'azote, de soufre, ou par un radical SO₂, à l'exception du carbone relié à l'atome d'azote. B ne comportant pas de liaison peroxyde ni de radicaux diazo, nitro ou nitroso ;
le radical B est relié à D par l'un quelconque des atomes du radical D ;
n"' peut prendre la valeur 0 ou 1 ;
D est choisi parmi les groupes cationiques de formules (VIIIf) et (VIIIg) suivantes : 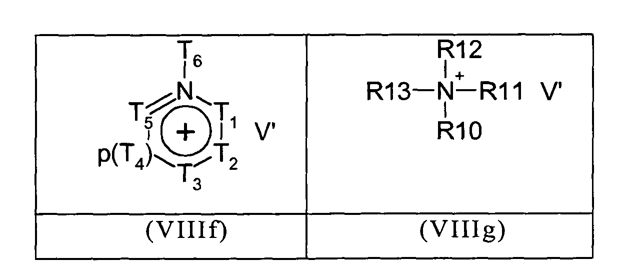
dans lesquelles :
p peut prendre la valeur 0 ou 1 ;
T₁, T₂, T₃ et T₄, indépendamment les uns des autres, représentent un atome d'oxygène, un atome de soufre, un atome d'azote non substitué ou substitué par un radical R₁₄, ou un atome de carbone non substitué ou substitué par un ou deux radicaux R₁₄, identiques ou différents ;
T₅ représente un atome d'azote ou un atome de carbone non substitué ou substitué par un radical R₁₄ ;
T₆ peut prendre les mêmes significations que celles indiquées ci-dessous pour le radical R₁₄, étant entendu que T₆ est différent d'un atome d'hydrogène ;
T₁ ou T₅ peuvent, en outre, former avec T₆ un cycle saturé ou insaturé comportant de 5 à 7 chaînons, chaque chaînon étant non substitué ou substitué par un ou deux radicaux R₁₄ identiques ou différents ;
deux des radicaux adjacents T₁, T₂, T₃, T₄ et T₅ peuvent en outre former un cycle comportant de 5 à 7 chaînons, chaque chaînon étant indépendamment représenté par un atome de carbone non substitué ou substitué par un ou deux radicaux R14 identiques ou différents, un atome d'azote substitué ou non substitué par un radical R₁₄, un atome d'oxygène ou un atome de soufre ;
R₁₀, R₁₁, R₁₂, R₁₃ et R₁₄, identiques ou différents, représentent un atome d'hydrogène, une chaîne hydrocarbonée comportant de 1 à 10 atomes de carbone, linéaire ou ramifiée, éventuellement aromatiques, et dont un ou plusieurs atomes de carbone peuvent être remplacés par un atome d'oxygène, d'azote, de soufre, ou par un groupe SO₂, et dont un ou plusieurs atomes de carbone peuvent, indépendamment les uns des autres, être substitués par un ou plusieurs atomes d'halogène. Ledit radical ne comportant pas de liaison peroxyde ni de radicaux diazo, nitro ou nitroso ;
R₁₀, R₁₁ et R₁₂ peuvent également former, deux à deux avec l'atome d'azote quaternaire auquel ils sont rattachés, un ou plusieurs cycles saturés comportant de 5 à 7 chaînons, chaque chaînon étant indépendamment représenté par un atome de carbone non substitué ou substitué par un ou deux radicaux R₁₄ identiques ou différents, un atome d'azote non substitué ou substitué par un radical R₁₄, un atome d'oxygène ,ou un atome de soufre ;
lorsque n"' vaut 0, alors le groupement de formule (VIIIg) peut être relié au composé de formule (VIIIc) et (VIIId) directement par l'atome d'azote de l'ammonium quaternaire, R₁₃ représentant dans ce cas une simple liaison ;
V' représente un anion organique ou minéral ;
Z₂ représente une chaîne hydrocarbonée en C₁-C₁₀, linéaire ou ramifiée pouvant former un cycle carboné ayant de 5 à 7 chaînons, éventuellement aromatique, un ou plusieurs atomes de carbone pouvant être remplacés par un ou plusieurs atomes d'oxygène, d'azote, de soufre ou par un groupement SO₂ ; ledit radical Z₂ ne comportant pas de liaison peroxyde, ni de radicaux diazo, nitro ou nitroso, un groupe cationique Z₃ tel que défini ci-dessus, avec la réserve que Z₂ n'est pas cationique lorsque R₇, R₉, R_{7'} ou R₉, est cationique ;
A₂ représente un radical de formule (VIIIh) correspondant à un radical aromatique carboné, pyridinique ou pyridazinique substitué par un radical hétéroaromatique cationique à 5 chaînons, éventuellement substitué par un ou plusieurs radicaux R₁₉ de même définition que R₂, un radical de formule (VIIIi) : 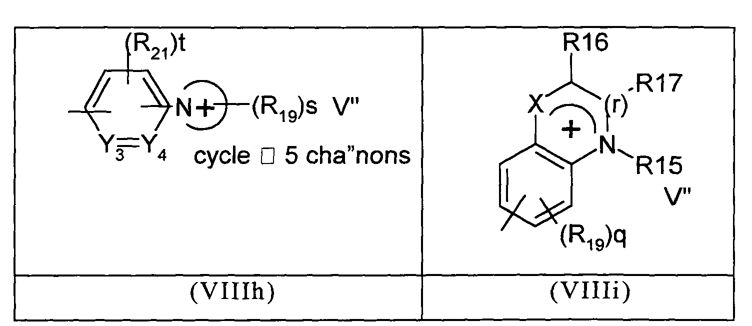 dans lesquelles :
r est un entier égal à 0 ou 1.
q est un entier égal à 0, 1, 2 ou 3.
s est un entier égal à 0, 1, 2, 3 , 4 ou 5.
t est un entier égal à 0, 1 ou 2.
Y₃=Y₄ représente C=C, C=N ou N=N.
si r=0 alors X représente O, S, NR₁₈, CR₂₀.
si r=1 alors X représente CR₂₀.
R₁₅ et R₁₈ ont la même définition que R₁ définie ci-dessus.
R₁₆, R₁₇, R₁₉, R₂₀ et R₂₁ ont la même définition que R₂ définie ci-dessus.
V" représente un anion organique ou minéral avec la condition que dans la formule (VIIIa) un des groupes A₁, Z₂ et A₃ est un groupe cationique ;
• Les composés de formule (IX)
W₁-N=N-W₂-W₃ (IX)
Dans laquelle W₁ représente un hétérocycle aromatique cationique à 5 chaînons de formule (IXa) suivante : 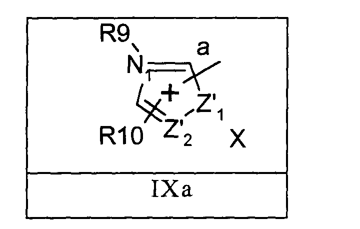
W₂ représente un groupe divalent aromatique carboné ou pyridinique de formules (IXb) ou (IXc) suivantes : 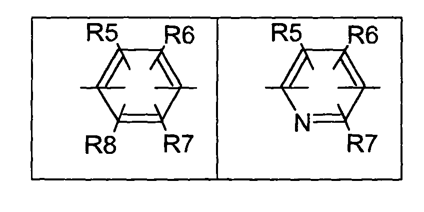 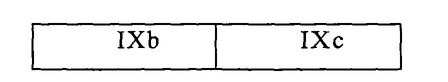
W₃ représente un hétérocycle à 5 ou 6 chaînons de formule (Xd) suivante : 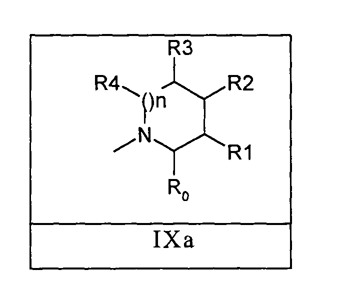 formules dans lesquelles:
Z' ₁ représente un atome d'oxygène, de soufre ou un radical NR₁₂.
Z'₂ représente un atome d'azote ou un radical CR₁₁.
R₉ et R₁₂ représentent, indépendamment l'un de l'autre un, radical alkyle en C₁-C₈, éventuellement substitué par un ou plusieurs radicaux choisis parmi un hydroxy, un alcoxy en C₁-C₂, un radical (poly)-hydroxyalcoxy en C₂-C₄, un amino, un (di)alkylamino en C₁-C₂, un carboxy, un sulfonique ,un radical phényle éventuellement substitué ;
R₁₀ et R₁₁ représentent, indépendamment l'un de l'autre, un atome d'hydrogène, un radical alkyle en C₁-C₄, éventuellement substitué par un ou plusieurs radicaux choisis parmi un hydroxy, un alcoxy en C₁-C₂, un radical (poly)-hydroxyalcoxy en C₂-C₄, un amino, un (di)alkylamino en C₁-C₂, un carboxy, un sulfonique, un radical phényle éventuellement substitué, un radical carboxy, un radical sulfonylamino.
R₅, R₆, R₇ et R₈ représentent, indépendamment les uns des autres un atome d'hydrogène, un atome de chlore, un atome de brome, une chaîne hydrocarbonée en C₁-C₆ linéaire ou ramifiée, pouvant former un ou plusieurs cycles carbonés comportant de 3 à 6 chaînons, et pouvant être saturée ou insaturée, dont un ou plusieurs atomes de carbone de la chaîne carbonée peuvent être remplacés par un atome d'oxygène, d'azote ou de soufre ou par un groupement SO₂ et dont les atomes de carbone peuvent être, indépendamment les uns des autres, substitués par un ou plusieurs atomes d'halogène ; R₅, R₆, R₇ et R₈ ne comportent pas de liaison peroxyde, ni de radicaux diazo ou nitroso ;
n est un nombre entier égale à 0 ou 1 ;
R₀, R₁, R₂, R₃ et R₄ représentent, indépendamment les uns des autres, un atome d'hydrogène, un radical hydroxy, amino, acétoxy, un groupement -NR₁₃R₁₄, R₁₃ et R₁₄ représentant indépendamment l'un de l'autre un atome d'hydrogène, un radical alkyle en C₁-C₄ substitué par un ou plusieurs radicaux choisis parmi un atome d'halogène, un radical hydroxy, alcoxy en C₁-C₂, amino ou amino(di)alkyle en C₁-C₂, un radical sulfonylamino, un radical carboxy, un radical carboxamido, un radical amido, un radical mono ou di alkylamido, un halogène, un radical alkyle en C₁-C₆ substitué par un ou plusieurs radicaux choisis parmi un radical hydroxy, alcoxy en C₁-C₂, (poly)-hydroxyalcoxy en C₂-C₄, amino, (di)alkylamino en C₁-C₂, étant entendu qu'au moins un des groupes R₀, R₁, R₂, R₃ et R₄ est différent de l'hydrogène ,
X est un anion organique ou minéral ;
• Les composés de formule (X)
W'₁-N=N-W'₂-W'₃ (X)
Dans laquelle W'₁ représente un hétérocycle aromatique cationique à 5 chaînons de formule (Xa) suivante : 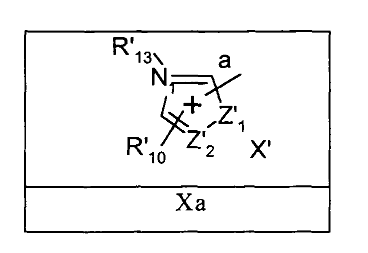
W'₂ représente un groupe divalent aromatique carboné ou pyridinique de formules (Xb) ou (Xc) suivantes : 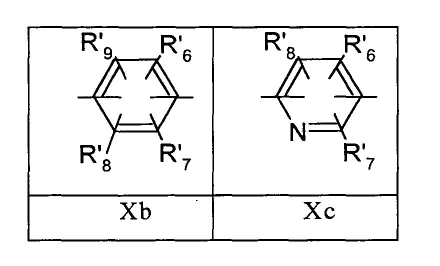
W'₃ représente un hétérocycle à 7 ou 8 chaînons de formule (Xd) suivante : 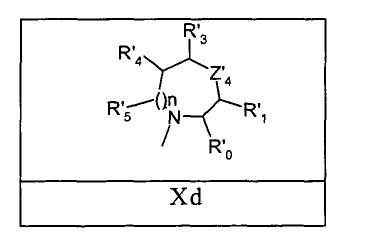 formules dans lesquelles :
Z'₁ représente un atome d'oxygène, de soufre ou un radical NR'₁₂ ;
Z'₂ représente un atome d'azote ou un radical CR'₁₁ ;
R'₁₂ et R'₁₃ représentent, indépendamment l'un de l'autre, un radical alkyle en C₁-C₈, éventuellement substitué par un ou plusieurs radicaux choisis parmi un hydroxy, un alcoxy en C₁-C₂, un radical (poly)-hydroxyalcoxy en C₂-C₄, un amino, un (di)alkylamino en C₁-C₂, un carboxy ou un sulfonique, un radical phényle éventuellement substitué ;
R'₁₀ et R'₁₁ représentent, indépendamment l'un de l'autre, un atome d'hydrogène, un radical alkyle en C₁-C₄, éventuellement substitué par un ou plusieurs radicaux choisis parmi un hydroxy, un alcoxy en C₁-C₂, un radical (poly)-hydroxyalcoxy en C₂-C₄, un amino, un (di)alkylamino en C₁-C₂, un carboxy ou un sulfonique, un radical phényle éventuellement substitué, un radical carboxy, un radical sulfonylamino ;
R'₆, R'₇, R'₈ et R'₉ représentent, indépendamment les uns des autres un atome d'hydrogène, un atome de chlore, un atome de brome, une chaîne hydrocarbonée en C₁-C₆ linéaire ou ramifiée, pouvant former un ou plusieurs cycles carbonés comportant de 3 à 6 chaînons, et pouvant être saturées ou insaturées, dont un ou plusieurs atomes de carbone de la chaîne carbonée peuvent être remplacés par un atome d'oxygène, d'azote ou de soufre ou par un groupement SO₂, et dont les atomes de carbone peuvent être, indépendamment les uns des autres, substitués par un ou plusieurs atomes d'halogène ; R'₆, R'₇, R'₈ et R'₉ ne comportent pas de liaison peroxyde, ni de radicaux diazo ou nitroso ;
n est un nombre entier égale à 1 ou 2 ;
Z'₄ représente un atome d'oxygène, de soufre, un radical NR'₂ ou un radical CR'₂R"₂ ;
R'₀, R'₁, R'₂, R"₂, R'₃, R'₄ et R'₅ représentent, indépendamment les uns des autres, un atome d'hydrogène, un radical alkyle, un radical alcoxy, un radical hydroxy, amino, acétoxy, un groupement -NR₁₄R₁₅, R₁₄ et R₁₅ représentant indépendamment l'un de l'autre un atome d'hydrogène, un radical alkyle en C₁-C₄ substitué par un ou plusieurs radicaux choisis parmi un atome d'halogène, un radical hydroxy, alcoxy en C₁-C₂, amino ou amino(di)alkyle en C₁-C_{2,} un radical sulfonylamino, un radical carboxy, un radical carboxamido, un radical amido, un radical mono ou di alkylamido, un halogène, un radical alkyle en C₁-C₆ substitué par un ou plusieurs radicaux choisis parmi un radical hydroxy, alcoxy en C₁-C₂, (poly)-hydroxyalcoxy en C₂-C₄, amino, (di)alkylamino en C₁-C₂ ,
X' est un anion organique ou minéral ;
• Les composés de formule (XI) 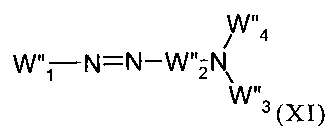 dans laquelle W"₁ représente un hétérocycle aromatique cationique à 5 chaînons de formule (XIa) suivante : 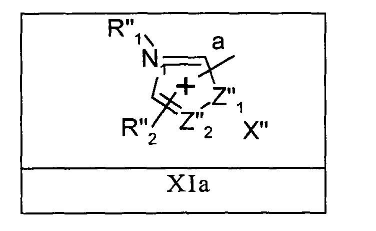
W"₂ représente un groupe divalent aromatique carboné ou pyridinique de formules (XIb) ou (XIc) suivantes : 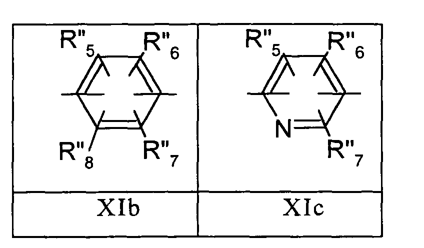 formules dans lesquelles :
Z"₁ représente un atome d'oxygène, de soufre ou un radical NR"_{4.}
Z"₂ représente un atome d'azote ou un radical CR"_{3.}
R"₁ et R"₄ représentent, indépendamment l'un de l'autre, un radical alkyle en C₁-C₈, éventuellement substitué par un ou plusieurs radicaux choisis parmi un hydroxy, un alcoxy en C₁-C₂, un radical (poly)-hydroxyalcoxy en C₂-C₄, un amino, un (di)alkylamino en C₁-C₂, un carboxy, un sulfonique, un radical phényle éventuellement substitué ;
R"₂ et R"₃ représentent, indépendamment l'un de l'autre, un atome d'hydrogène, un radical alkyle en C₁-C₄, éventuellement substitué par un ou plusieurs radicaux choisis parmi un hydroxy, un alcoxy en C₁-C₂, un (poly)-hydroxyalcoxy en C₂-C₄, un amino, un (di)alkylamino en C₁-C₂, un carboxy ou un sulfonique, un radical phényle éventuellement substitué, un radical carboxy, un radical sulfonylamino ;
R"₅, R"₆, R"₇, R"₈ et W"₄ représentent, indépendamment les uns des autres un atome d'hydrogène, un atome de chlore, un atome de brome, une chaîne hydrocarbonée en C₁-C₆ linéaire ou ramifiée, pouvant former un ou plusieurs cycles carbonés comportant de 3 à 6 chaînons, et pouvant être saturées ou insaturées, dont un ou plusieurs atomes de carbone de la chaîne hydrocarbonée peuvent être remplacés par un atome d'oxygène, d'azote ou de soufre ou par un groupement SO₂, et dont les atomes de carbone peuvent être, indépendamment les uns des autres, substitués par un ou plusieurs atomes d'halogènes ; R"₅, R"₆, R"₇, R"₈ et W"₄ ne comportant pas de liaison peroxyde, ni de radicaux diazo ou nitroso, et W"₄ étant un substituant non aromatique ;
W"₃ représente un radical thiényle, pyrazolyle, pyrrolyle, imidazolyle, furyle, triazolyle, thiadiazolyle, isoxazolyle, 5-isothiazolyle, thiazolyle, oxazolyle, pyridyle, pyrimidinyle, triazinyle, pyridazinyle, pyrazinyle, chacun de ces cycles hétéroaromatiques pouvant être substitué par au moins un radical alkyle en C₁-C₄, éventuellement substitué par un ou plusieurs hydroxy, alcoxy en C₁-C₄, (poly)-hydroxyalcoxy, amino, (di )alkylamino en C₁ -C₄, (poly)hydroxyalkylamino en C₂-C₄, carboxy, sulfonyle, alcoxycarbonyle ou thioéther en C₁-C₄, un radical phényle éventuellement substitué par un ou plusieurs radicaux choisis parmi les radicaux alcoxy en C₁-C₂, amino, (di)-alkylamino en C₁-C₂, carboxy, sulfonyle, alkyle en C₁-C₄, halogène ou thioéther en C₁-C₂, un halogène tel qu'un atome de chlore, de fluor ou de brome, un radical amino, un radical alkylamino en C₁-C₄, un radical (poly)hydroxyalkylamino en C₂-C₄, un radical (di)alkylamino en C₁-C₄, un radical alcoxy en C₁-C₂, un radical carboxyle, un radical sulfonylamino ,
X" est un anion organique ou minéral ;
• Les composés de formule (XII)
W⁰ ₁-W⁰ ₂-N=N-W⁰ ₃ (XII)
Dans laquelle W⁰₁ représente un hétérocycle à 5, 6, 7 ou 8 chaînons de formule (XIIa) suivante : 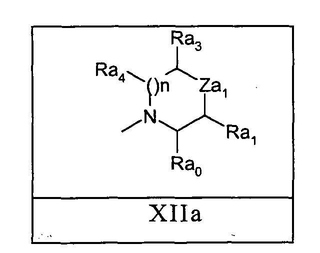
W⁰₂ représente un groupe divalent aromatique carboné, pyridinique ou pyridazinique de formule (XIIb) suivante : 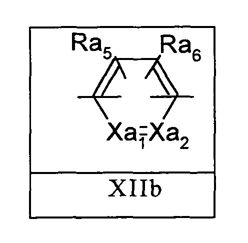
W⁰₃ représente un radical hétéroaromatique cationique représenté par la formule (XIIc) suivante : 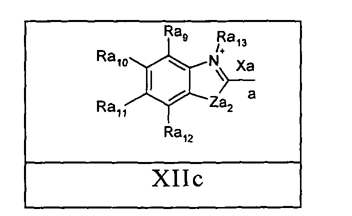 formules (XIIa), (XIIb), (XIIc) dans lesquelles :
n=0, 1, 2 ou 3, étant entendu que lorsque n est supérieur ou égal à 2, alors les radicaux Ra₄ peuvent être identiques ou différents ;
Xa₁ représente un atome d'azote ou un radical CRa₇ ;
Xa₂ représente un atome d'azote ou un radical CRa₈ ;
Za₁ représente un radical CHRa₂, un atome d'oxygène, de soufre ou un radical NRa₁₄ ;
Za₂ représente un atome d'oxygène, de soufre ou un radical NRa₁₅ ;
Ra₀, Ra₁, Ra₂, Ra₃, Ra₄, Ra₅, Ra₆, Ra₇, Ra₈,.Ra₉, Ra₁₀, Ra₁₁, et Ra₁₂ représentent, identiques ou différents, un atome d'hydrogène, une chaîne hydrocarbonée en C₁-C₁₀ linéaire ou ramifiée, pouvant former un ou plusieurs cycles carbonés comportant de 3 à 6 chaînons, et pouvant être saturée ou insaturée, dont un ou plusieurs atomes de carbone de la chaîne carbonée peuvent être remplacés par un atome d'oxygène, d'azote ou de soufre ou par un groupement SO₂, et dont les atomes de carbone peuvent être, indépendamment les uns des autres, substitués par un ou plusieurs atomes d'halogène, Ra₀, Ra₁, Ra₂, Ra₃, Ra₄, Ra₅, Ra₆, Ra₇, Ra₈,.Ra₉, Ra₁₀, Ra₁₁, et Ra₁₂ ne comportant pas de liaison peroxyde, ni de radicaux diazo ou nitroso ;
Ra₁₄ représente un atome d'hydrogène, une chaîne hydrocarbonée en C₁ -C₁₀ linéaire ou ramifiée, pouvant former un ou plusieurs cycles carbonés comportant de 3 à 6 chaînons, et pouvant être saturée ou insaturée, dont un ou plusieurs atomes de carbone de la chaîne carbonée peuvent être remplacés par un atome d'oxygène, d'azote ou de soufre ou par un groupement SO₂, et dont les atomes de carbone peuvent être, indépendamment les uns des autres, substitués par un ou plusieurs atomes d'halogène, Ra₁₄ ne comportant pas de liaison peroxyde, ni de radicaux diazo ou nitroso, étant entendu que lesdits atomes d'oxygène, d'azote et de soufre ne sont pas reliés directement à l'atome d'azote porteur du radical Ra₁₄ ;
Ra₅ et Ra₆ peuvent former un cycle aromatique carboné, tel qu'un phényle ;
Ra13 et Ra15 représentent, identiques ou différents, un radical alkyle en C1-C8,éventuellement substitué par un ou plusieurs radicaux choisis dans le groupe constitué par un hydroxy, un alcoxy en C1-C2, un radical (poly)hydroxyalcoxy en C2-C4, un amino, un (di)-alkylamino en C1-C2, un carboxyle, un sulfonique, un phényl éventuellement substitué ; la liaison a du cycle cationique de la formule (XIIc) est reliée au groupement azoïque de la formule (XIIa) ,
Xa est un anion organique ou minéral ;
• Les composés de formule (XIII)
A-N=N-B (XIII)
Dans laquelle le symbole A représente un groupement choisi parmi les structures A1 à A3 suivantes : 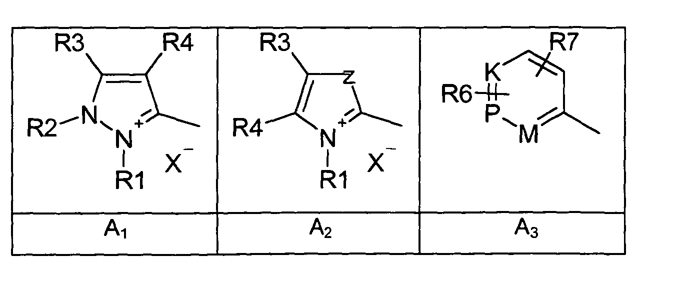 Dans lesquelles,
R₁ désigne un radical alkyle en C₁-C₄, un radical phényle pouvant être substitué par un radical alkyle en C₁-C₄ ou un atome d'halogène choisi parmi le chlore, le brome, l'iode et le fluor ;
R₂ désigne un radical alkyle en C₁-C₄ ou un radical phényle ;
R₃ et R₄, identiques ou différents, représentent un radical alkyle en C₁-C₄, un radical phényle, ou bien, dans le cas de la structure A₁, peuvent former ensemble un cycle benzénique substitué, et dans le cas de la structure A₂, peuvent former ensemble un cycle benzénique éventuellement substitué par un ou plusieurs radicaux alkyle en C₁-C₄, alcoxy en CI-C4, ou NO₂ ;
R₃ peut en outre désigner un atome d'hydrogène ;
Z désigne un atome d'oxygène, de soufre ou un groupement -NR₂ ;
M représente un groupement -CH, -CR (R désignant alkyle en C₁-C₄) ou -NR₅ (X⁻)ᵣ ;
K représente un groupement -CH, -CR (R désignant alkyle en C₁-C₄) ou -NR₅ (X⁻)r ;
P représente un groupement -CH, -CR (R désignant alkyle en C1-C4) ou -NR₅ (X⁻)ᵣ. r désignant 0 ou 1 ;
R₅ représente un atome O⁻, un radical alcoxy en C₁-C₄, ou un radical alkyle en C₁-C₄ ;
R₆ et R₇, identiques ou différents, représentent un atome d'hydrogène ou d'halogène choisi parmi le chlore, le brome, l'iode et le fluor, un radical alkyle en C₁-C₄, alcoxy en C₁-C₄, un radical NO₂ ;
X⁻ représente un anion de préférence choisi parmi le chlorure, l'iodure, le méthyl sulfate, l'éthyl sulfate, l'acétate et le perchlorate, sous réserve que,
• si R₄ désigne un radical alkyle en C₁-C₄ et Z désigne un atome de soufre, R₃ ne désigne pas un atome d'hydrogène.
• si R₅ désigne O⁻, alors r désigne zéro.
• si K ou P ou M désignent -N-alkyle C₁-C₄ X⁻, alors R₆ ou R₇ est différent d'un atome d'hydrogène.
• si. K désigne -NR₅(X⁻)ᵣ, alors M=P=-CH ou -CR
• si M désigne -NR₅(X⁻)ᵣ, alors K=M= -CH ou -CR
• si P désigne -NR₅(X⁻)ᵣ. alors K=M et désignent -CH ou -CR
• si Z désigne -NR₂ et R₂ désigne un radical alkyle en C₁-C₄, alors au moins un des radicaux R₁, R₃ ou R₄ de A₂ est différent d'un radical alkyle en C₁-C₄, le symbole B représente :
a- Un groupement de structure B₁ suivante : 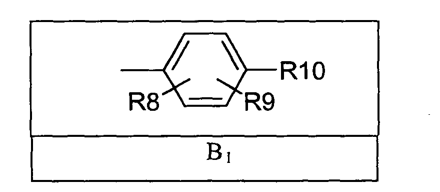 Dans laquelle, R₈ représente un atome d'hydrogène, un atome d'halogène choisi parmi le chlore, le brome, l'iode et le fluor, un radical alkyle en C₁-C₄, alcoxy en C₁-C₄, un radical -OH, -NO₂, -NHR₁₁, -NR₁₂R₁₃, -NHCOalkyle en C₁-C₄, ou forme avec R₉ un cycle à 5 ou 6 chaînons, contenant ou non un ou plusieurs hétéroatomes choisis parmi l'azote, l'oxygène ou le soufre ;
R₉ représente un atome d'hydrogène, un atome d'halogène choisi parmi le chlore, le brome, l'iode et le fluor, un radical alkyle en C₁-C₄, alcoxy en C₁-C_{4,} ou forme avec R₁₀ ou R₁₁ un cycle à 5 ou 6 chaînons contenant ou non un ou plusieurs hétéroatomes choisis parmi l'azote, l'oxygène ou le soufre ;
R₁₀ représente un atome d'hydrogène, un radical -OH, un radical -NHR₁₁, un radical -NR₁₂R₁₃ ;
R₁₁ représente un atome d'hydrogène, un radical alkyle en C₁-C₄, un radical monohydroxyalkyle en C₁-C₄, polyhydroxyalkyle en C₂-C₄, un radical phényle.
R₁₂ et R₁₃, identiques ou différents, représentent un radical alkyle en C₁-C₄, un radical monohydroxyalkyle en C₁-C₄, polyhydroxyalkyle en C₂-C₄ ;
b- un groupement hétérocyclique azoté à 5 ou 6 chaînons susceptible de renfermer d'autres hétéroatomes et/ou des groupements carbonylés et pouvant être substitué par un ou plusieurs radicaux alkyle en C₁-C₄, amino ou phényle et notamment un groupement de structure B₂ suivante : 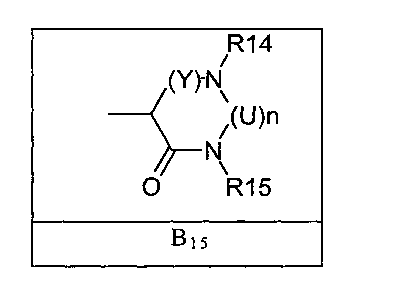 Dans laquelle, R₁₄ et R₁₅ identiques ou différents, représentent un atome d'hydrogéne, un radical alkyle en C₁-C₄, un radical phényle ;
Y désigne le radical -CO- ou le radical 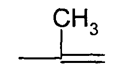
N vaut 0 ou 1, avec, lorsque n désigne 1, U désigne le radical -CO ;
• Les composés de formule (XIV) :
W₁-N=N-W₂-W₃ (XIV)
dans laquelle
- W1 représente un hétérocycle aromatique cationique à 5 chaînons de formule(II) suivante : 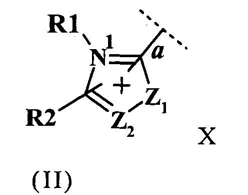
- W2 représente un groupe divalent aromatique carboné ou pyridinique de formules (IV) ou (V) suivantes : 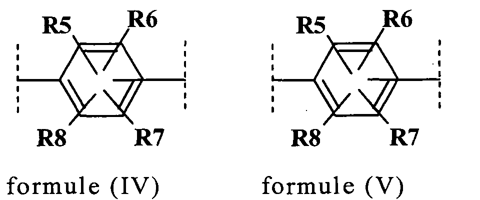
- W3 représente un radical hétéroaromatique azoté à 5 ou 6 chaînons relié à W2 par l'atome d'azote du cycle du radical hétéroaromatique, le radical héréroaromatique étant choisi parmi les radicaux pyrazolyle, pyrrolyle, imidazolyle, triazolyle, thiadiazolyle, pyridazinyle, pyrazinyle, chacun de ces hétéroaromatiques pouvant être éventuellement substitué par un ou plusieurs radicaux choisi parmi un atome d'hydrogène ; un radical alkyle en C1-C6, éventuellement substitué par un ou plusieurs radicaux hydroxy, alcoxy en C1-C4, (poly)-hydroxyalcoxy, amino, (di)alkylamino en C1-C4, (poly)hydroxyalkylamino en C2-C4, carboxy, sulfonyle, alcoxycarbonyle, thioether en C1-C4 ; un radical phényle éventuellement substitué par un ou plusieurs radicaux choisis parmi les radicaux alcoxy en C1-C2, amino, (di)-alkylamino en C1-C2, carboxy, sulfonyle, alkyle en C1-C4, halogène, thioéther en C1-C2,
- Z1 représente un atome d'oxygène, de soufre ou un radical NR4,
- Z2 représente un atome d'azote ou un radical CR3,
- R1 et R4 représentent, indépendamment l'un de l'autre, un radical alkyle en C1-C8, éventuellement substitué par un ou plusieurs radicaux choisis parmi un hydroxy, un alcoxy en C1-C2, un radical (poly)-hydroxyalcoxy en C2-C4, un amino, un (di)alkylamino en C1-C2 , un carboxy, un sulfonique ; un radical phényle éventuellement substitué,
- R2 et R3 représentent, indépendamment l'un de l'autre, un atome d'hydrogène ; un radical alkyle en C1-C6, éventuellement substitué par un ou plusieurs radicaux choisis parmi un hydroxy, un alcoxy en C1-C2, un radical (poly)-hydroxyalcoxy en C2-C4, un amino, un (di)alkylamino en C1-C2 , un carboxy, un sulfonique; un radical phényle éventuellement substitué ; un radical carboxy ; un radical sulfonylamino ;
- R5, R6, R7 et R8 représentent, ensemble ou indépendamment l'un de l'autre un atome d'hydrogène ; un atome de chlore ; un atome de brome ; une chaîne hydrocarbonée en C1-C6 linéaire ou ramifiée, pouvant former un ou plusieurs cycles carbonés comportant de 3 à 6 chaînons, et pouvant être saturées ou insaturées, dont un ou plusieurs atomes de carbone de la chaîne carbonée peuvent être remplacés par un atome d'oxygène, d'azote ou de soufre ou par un groupement SO2, et dont les atomes de carbone peuvent être, indépendamment les uns des autres, substitués par un ou plusieurs atomes d'halogènes ; R5, R6, R7 et R8 ne comportant pas de liaison peroxyde, ni de radicaux diazo ou nitroso,
- X représente un anion organique ou minéral ;
• Les composés de formule (XV) :
W₁-N=N-W₂-(W₃)n-W₄-N=N-W₅ (XV)
dans laquelle
n représente 0 ou 1,
- W1 et W5, indépendamment l'un de l'autre, représentent un radical hétéroaromatique de formules (II) et (III) suivantes : 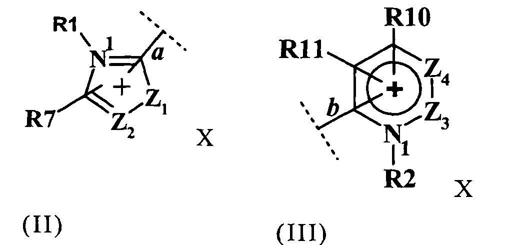
- W3 représente un atome d'oxygène, un radical NR14, un groupe -NR15-W6-NR16-, un groupe -NR16-W6-O-, un groupe -O-W6-O-, un radical W6, un radical carbonyle,
- W2 et W4 représentent, indépendamment l'un de l'autre, un groupement aromatique carboné, pyridinique ou pyridazinyle de formule (IV) 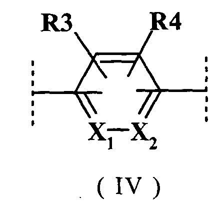
- W6 représente un groupement aromatique ou hétéroaromatique à 5 ou 6 chaînons de formule (V) : 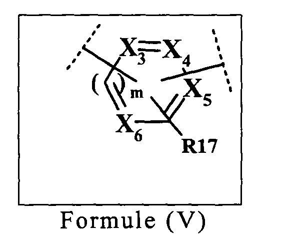 dans lesquelles
m représente 0 ou 1,
X1 représente un atome d'azote ou un radical CR5,
X2 représente un atome d'azote ou un radical CR6,
X3 représente un atome d'azote, de carbone ou un radical CR18,
X4 représente un atome d'azote, de carbone ou un radical CR19,
X5 représente un atome d'azote, de carbone ou un radical CR20,
X6 représente un atome d'azote, de carbone ou un radical CR21,
Z1 représente un atome d'oxygène, de soufre ou un radical NR8,
Z2 représente un atome d'azote ou un radical CR9,
Z3 représente un atome d'azote ou un radical CR12,
Z4 représente un atome d'azote ou un radical CR13,
la liaison *a* du cycle cationique à 5 chaînons de la formule (II) est reliée au groupement azoïque de la formule (XV),
la liaison *b* du cycle cationique à 6 chaînons de la formule (III) est reliée au groupement azoïque de la formule (XV),
étant entendu que lorsque X3 à X6 représentent un atome de carbone, alors ils sont reliés à W2 ou W4,
étant entendu que la formule (V) ne contient pas plus de trois atomes d'azote,
étant entendu que lorsque la formule (V) contient trois atomes d'azote, ils sont non contigus,
R1, R2 et R8 représentent indépendamment l'un de l'autre un radical alkyle en C1-C8, linéaire ou ramifié, éventuellement substitué par un ou plusieurs radicaux choisis parmi les radicaux hydroxy, alcoxy en C1-C2, (poly)-hydroxyalcoxy en C2-C4, amino, (di)alkylamino en C1-C2 , carboxy ou sulfonique ; un radical phényle éventuellement substitué par un ou plusieurs radicaux choisis parmi les radicaux hydroxy, alcoxy en C1-C2, (poly)-hydroxyalcoxy en C2-C4, amino, (di)alkylamino en C 1-C2 , carboxy, sulfonique ou un atome d'halogène tel que chlore, fluor ou brome,
R3, R4, R5, R6, R7, R9, R10, R11, R12, R13 , R14, R15 , R16, R17, R18 , R19 , R20 et R21 représentent, indépendamment l'un de l'autre, un atome d'hydrogène, une chaîne hydrocarbonée en C1-C16 linéaire ou ramifiée, pouvant être saturée ou insaturée, dont un ou plusieurs atomes de carbone de la chaîne carbonée peuvent être remplacés par un atome d'oxygène, d'azote ou de soufre ou par un groupement SO2, et dont les atomes de carbone peuvent être, indépendamment les uns des autres, substitués par un ou plusieurs atomes d'halogènes ; R3, R4, R5, R6, R7, R9, R10, R11, R12, R13 , R14, R15, R16, R17, R18 , R19 , R20 et R21 ne comportant pas de liaison peroxyde, ni de radicaux diazo ou nitroso, R7 avec R9, R10 avec R11 et R12 avec R13 peuvent former un cycle aromatique carboné, tel qu'un phényle,
X est un anion organique ou minéral ;
• Les composés de formule (XVI) :
[W₁-N=N-W₂-W₃-(W₄-W₅)ₚ]₂-L (XVI)
dans laquelle
p vaut 0 ou 1.
- W1 représente un radical hétéroaromatique de formules (II) et (III) suivantes : 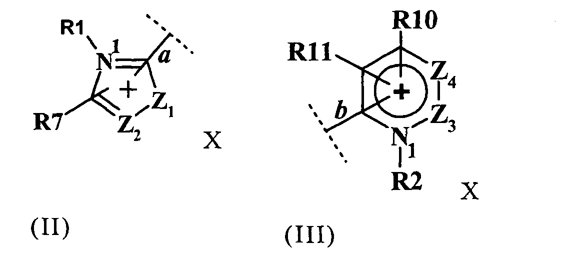
- W2 représente un groupement aromatique carboné, pyridinique ou pyridazinyle de formule (IV) suivante : 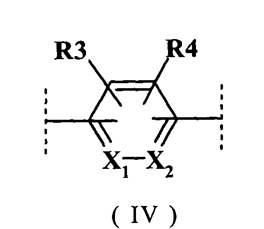
- W3 et W5 représentent indépendamment l'un de l'autre un radical -NR14- ou un atome d'oxygène -O- , ou un atome d'azote,
W4 représente une chaîne hydrocarbonée en C1-C16 linéaire ou ramifiée, pouvant former un ou plusieurs cycles carbonés comportant de 3 à 6 chaînons, et pouvant être saturée ou insaturée, dont un ou plusieurs atomes de carbone de la chaîne carbonée peuvent être remplacés par un atome d'oxygène, d'azote ou de soufre ou par un groupement SO2, et dont les atomes de carbone peuvent être, indépendamment les uns des autres, substitués par un ou plusieurs atomes d'halogènes, W4 ne comportant pas de liaison peroxyde, ni de radicaux diazo ou nitroso; un cycle pipérazine ; un groupe de formule (V) suivante : 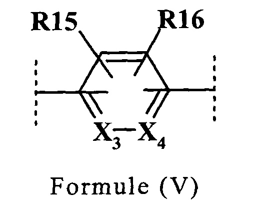 Formules (II), (III), (IV), (V) dans lesquelles :
X1 représente un atome d'azote ou un radical CR5
X2 représente un atome d'azote ou un radical CR6
X3 représente un atome d'azote ou un radical CR17
X4 représente un atome d'azote ou un radical CR18
Z1 représente un atome d'oxygène, de soufre ou un radical NR8,
Z2 représente un atome d'azote ou un radical CR9,
étant entendu que (Z1, Z2) est différent de (NR8, CR9),
Z3 représente un atome d'azote ou un radical CR12,
Z4 représente un atome d'azote ou un radical CR13,
la liaison *a* du cycle cationique à 5 chaînons de la formule (II) est reliée au groupement azoïque de la formule (XVI),
la liaison *b* du cycle cationique à 6 chaînons de la formule (III) est reliée au groupement azoïque de la formule (XVI),
lorsque p vaut 0, alors L représente une chaîne hydrocarbonée en C1-C16 linéaire ou ramifiée, pouvant former un ou plusieurs cycles carbonés comportant de 3 à 6 chaînons dont un ou plusieurs atomes de carbone de la chaîne carbonée peuvent être remplacés par un atome d'oxygène, d'azote ou de soufre ou par un groupement SO2, et dont les atomes de carbone peuvent être, indépendamment les uns des autres, substitués par un ou plusieurs atomes d'halogènes ; L ne comportant pas de liaison peroxyde, ni de radicaux diazo ou nitroso,
lorsque p vaut 1, alors L représente une chaîne hydrocarbonée en C1-C16 linéaire ou ramifiée, pouvant former un ou plusieurs cycles carbonés comportant de 3 à 6 chaînons, et pouvant être saturée ou insaturée, dont un ou plusieurs atomes de carbone de la chaîne carbonée peuvent être remplacés par un atome d'oxygène, d'azote ou de soufre ou par un groupement SO2 et ne comportant pas de liaison peroxyde, ni de radicaux diazo ou nitroso,
R3, R4, R5, R6, R7, R9, R10, R11, R12, R13, R14, R15, R16, R17 et R18 représentent, ensemble ou indépendamment l'un de l'autre , un atome d'hydrogène, une chaîne hydrocarbonée en C1-C16 linéaire ou ramifiée, pouvant former un ou plusieurs cycles carbonés comportant de 3 à 6 chaînons, et pouvant être saturée ou insaturée, dont un ou plusieurs atomes de carbone de la chaîne carbonée peuvent être remplacés par un atome d'oxygène, d'azote ou de soufre ou par un groupement SO2, et dont les atomes de carbone peuvent être, indépendamment les uns des autres, substitués par un ou plusieurs atomes d'halogènes ; R3, R4, R5, R6, R7, R9, R10, R11, R12 , R13 , R14, R15, R16, R17 et R18 comportant pas de liaison peroxyde, ni de radicaux diazo ou nitroso,
R3 avec R4, R7 avec R9, R10 avec R11,R12 avec R13 et R15 avec R16 peuvent former un cycle aromatique carboné, tel qu'un phényle,
X est un anion organique ou minéral ;
• Les composés de formule (XVI) :
W₁-N=N-W₂-NW₃-W₄-W₅ (XVI)
dans laquelle
- W1 représente un hétérocycle aromatique cationique à 5 ou 6 chaînons de formules (II) et (III) suivantes : 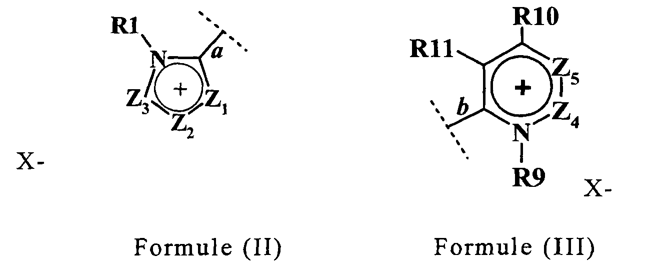 dans lesquelles :
Z1 représente un atome d'oxygène, un atome de soufre, un radical NR2, ou un radical CR3,
Z2 représente un atome d'azote ou un radical CR4,
Z3 représente un radical NR12 ou un radical CR13,
Z4 représente un atome d'azote ou un radical CR14,
Z5 représente un atome d'azote ou un radical CR15,
la liaison *a* relie le cycle cationique à 5 chaînons de formule (II) à la fonction azoïque de la formule (XVI).
la liaison *b* du cycle cationique à 6 chaînons de la formule (III) est reliée à la fonction azoïque de la formule (XVI).
X- est un anion organique ou minéral ;
- W2 et W4 (formule XVI) représentent, identiques ou différents, un groupe divalent aromatique carboné ou pyridinique de formules (IV) ou (V) suivantes : 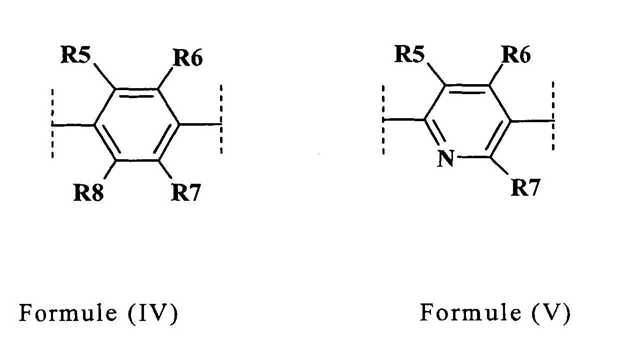
- W3 représente un atome d'hydrogène ou un radical alkyle en C1-C6 éventuellement substitué par un ou plusieurs radicaux hydroxy, un ou plusieurs radicaux alcoxy, un ou plusieurs radicaux amino, un ou plusieurs radicaux monoalkylamino, un ou plusieurs radicaux dialkylamino,
- W5 représente un radical hétéroaromatique azoté à 5 chaînons relié à W4 par l'atome d'azote du cycle dudit radical hétéroaromatique, ce radical héréroaromatique étant choisi parmi les radicaux pyrazolyle, pyrrolyle, imidazolyle, triazolyle, thiadiazolyle, chacun de ces hétéroaromatiques pouvant être substitué par un ou plusieurs atomes d'hydrogène, de chlore ou de fluor, ou par un ou plusieurs radicaux alkyle en C1-C6, éventuellement substitués par un ou plusieurs radicaux hydroxy, alcoxy en C1-C4, (poly)-hydroxyalcoxy, amino, (di)alkylamino en C1-C4, (poly)hydroxyalkylamino en C2-C4, carboxyle, sulfonyle, alcoxycarbonyle, thioether en C1-C4 ; ou par un ou plusieurs radicaux phényle éventuellement substitués par un ou plusieurs radicaux choisis parmi les radicaux hydroxy, alcoxy en C1-C2, amino, (di)-alkylamino en C1-C2, carboxy, sulfonyle, alkyle en C1-C4, halogène, thioéther en C1-C2,
- R1, R2, R9, R12 représentent, identiques ou différents, un radical phényle éventuellement substitué ou un radical alkyle en C1-C8, éventuellement substitué par un ou plusieurs radicaux choisis dans le groupe formé par les radicaux hydroxy, alcoxy en C1-C2, (poly)-hydroxyalcoxy en C2-C4, amino, (di)alkylamino en C1-C2 ,
- R5, R6, R7 et R8 représentent, identiques ou différents, un atome d'hydrogène ; un atome de chlore ; un atome de brome ; une chaîne hydrocarbonée en C1-C8 linéaire ou ramifiée, pouvant former un ou plusieurs cycles carbonés comportant de 3 à 6 chaînons, et pouvant être saturée ou insaturée, dont un ou plusieurs atomes de carbone de la chaîne carbonée peuvent être remplacés par un atome d'oxygène, d'azote ou de soufre ou par un groupement SO2, et dont les atomes de carbone peuvent être, indépendamment les uns des autres, substitués par un ou plusieurs atomes d'halogène ; R5, R6, R7 et R8 ne comportant pas de liaison peroxyde, ni de radicaux diazo ou nitroso,
- R3, R4, R10, R11, R13, R14 et R15 représentent, identiques ou différents, un atome d'hydrogène, une chaîne hydrocarbonée en C1-C16 linéaire ou ramifiée, pouvant former un ou plusieurs cycles carbonés comportant de 3 à 6 chaînons, et pouvant être saturée ou insaturée, dont un ou plusieurs atomes de carbone de la chaîne carbonée peuvent être remplacés par un atome d'oxygène, d'azote ou de soufre ou par un groupement SO2, et dont les atomes de carbone peuvent être, indépendamment les uns des autres, substitués par un ou plusieurs atomes d'halogène ; R3, R4, R10, R11, R13, R14 et R15 ne comportant pas de liaison peroxyde, ni de radicaux diazo ou nitroso,
R4 avec R13 et R14 avec R15 peuvent former un cycle aromatique carboné, tel qu'un phényle.

3. Composition selon la revendication 2, **caractérisée en ce que** les colorants directs cationiques azoïques, méthiniques ou azométhiniques utilisés de préférence sont les composés de formule I, II, III et III' .

4. Composition selon l'une quelconque des revendications précédentes, **caractérisée en ce que** les colorants directs cationiques représentent de préférence de 0,0005 à 15% en poids environ du poids total de la composition et encore plus préférentiellement, de 0,005 à 10% en poids environ.

5. Composition selon l'une quelconque des revendications précédentes, **caractérisée en ce que** l'enzyme alcool oxydase appartient à la classe E.C.1.1.3.

6. Composition selon la revendication 6, **caractérisée en ce que** l'enzyme alcool oxydase est choisie parmi les alcool primaire oxydases (EC1.1.3.13), les alcool secondaire oxydases (EC 1.1.3.18), les alcool à longue chaîne hydrocarbonée oxydases (EC 1.1.3.20), les alcool polyvinylique oxydases ( EC 1.1.3.30), l'alcool vanillique oxydase (EC 1.1.3.38), les alcool aromatique oxydases (EC 1.1.3.7).

7. Composition selon la revendication 7, **caractérisée en ce que** l'enzyme alcool oxydase est issue d'une des espèces suivantes : *Rhodococcus erythropolis, Pseudomonas pseudoalcaligenes, Aspergillus niger, Kamagataella pastoris, Phanerochaete chrysosporium, Polyporus obtusus, Hansenula polymorpha, Poria contigua, Penicillium simplicissimum, Pleurotus pulmonarius, Pichia sp. (pastoris, methanolica, angusta)* et *Candida sp. (boidinii, albicans, tropicalis) , Pinus strobus, Gastropode mollusc, Manduca sexta*.

8. Composition selon la revendication 8, **caractérisée en ce que** l'enzyme alcool oxydase est la *Pichia pastoris*

9. Composition selon l'une quelconque des revendications précédentes, **caractérisée en ce que** la concentration en enzyme alcool oxydase est comprise entre 0,05 et 20%, de préférence entre 0,1 et 10%, et plus particulièrement entre 0,5 et 8% en poids par rapport au poids total de ladite composition et de préférence comprise entre en poids par rapport au poids total de ladite composition.

10. Composition selon l'une quelconque des revendications précédentes, **caractérisée en ce que** la concentration en enzyme alcool oxydase est comprise entre 10³U et 10⁵U, de préférence entre 2.10³U et 5.10⁴U, pour 100g de la composition tinctoriale.

11. Composition selon l'une des revendications précédentes, **caractérisée en ce que** le substrat pour l'enzyme est un alcool choisi parmi les alcools primaires, secondaires, ramifiés ou non, saturés ou non, substitués ou non, les alcools à longue chaîne hydrocarbonée et les alcools aromatiques.

12. Composition selon la revendication 11, **caractérisée en ce que** la concentration du ou des substrats pour l'enzyme est comprise entre 0,01% et 60% en poids par rapport au poids total de la composition et de préférence comprise entre 0,05% et 30% en poids par rapport au poids total de la composition.

13. Composition selon l'une des revendications précédentes, **caractérisée en ce qu'**elle comprend en tant que précurseur de colorant d'oxydation une base d'oxydation classique choisie parmi les paraphénylènediamines, les bis-phénylalkylènediamines, les para-aminophénols, les ortho-aminophénols, les bases hétérocycliques et leurs sels d'addition.

14. Composition selon la revendication 13, **caractérisée en ce que** la concentration de la ou des bases d'oxydation est comprise entre 0,0001 et 20% en poids par rapport au poids total de la composition.

15. Composition selon l'une des revendications précédentes, **caractérisée en ce que** la composition comprend en tant que précurseur de colorant d'oxydation un coupleur d'oxydation classique choisi parmi les métaphénylènediamines, les métaaminophénols, les métadiphénols, les coupleurs naphtaléniques, les coupleurs hétérocycliques ainsi que leurs sels d'addition.

16. Composition selon la revendication 15, **caractérisée en ce que** la concentration du ou des coupleurs est comprise entre 0,0001 et 20 % en poids par rapport au poids total de la composition.

17. Composition selon l'une des revendications précédentes **caractérisée par le fait qu'**elle renferme un ou plusieurs colorants directs autres que des colorants directs cationiques azoïques, méthiniques ou azométhiniques.

18. Procédé de teinture des fibres kératiniques et en particulier des fibres kératiniques humaines telles que les cheveux, **caractérisé par le fait qu'**on applique sur lesdites fibres au moins une composition selon l'une des revendications 1 à 17, pendant une durée suffisante pour développer la coloration désirée.

19. Procédé selon la revendication 18, **caractérisé par le fait que** la composition est une composition prête à l'emploi comprenant, une composition selon l'une des revendications 1 à 17, l'ensemble étant stocké sous forme anaérobie, exempte d'oxygène gazeux.

20. Procédé selon la revendication 18, **caractérisé par le fait qu'**il comporte une étape préliminaire consistant à stocker sous forme séparée, d'une part une composition (A) comprenant, dans un milieu approprié pour la teinture, au moins un précurseur de colorant d'oxydation et d'autre part, une composition (B) renfermant, dans un milieu approprié pour les fibres kératiniques, au moins une enzyme alcool oxydase, la composition (A) et/ou la composition (B) contenant au moins un substrat pour ladite enzyme et la composition (A) et/ou la composition (B) contenant au moins un colorant direct cationique azoïque, méthinique ou azométhinique, puis à procéder au mélange des compositions (A) et (B) au moment de l'emploi avant d'appliquer ce mélange sur les fibres kératiniques.

21. Procédé selon la revendication 18, **caractérisé par le fait qu'**il comporte une étape préliminaire consistant à stocker sous forme séparée, d'une part une composition (A) comprenant, dans un milieu approprié pour la teinture, au moins un précurseur de colorant d'oxydation, au moins un substrat pour l'enzyme alcool axydase et au moins un colorant direct cationique azoïque, méthinique ou azométhinique et d'autre part, une composition (B) renfermant, dans un milieu approprié pour les fibres kératiniques, au moins une enzyme alcool oxydase, puis à procéder au mélange des compositions (A) et (B) au moment de l'emploi avant d'appliquer ce mélange sur les fibres kératiniques.

22. Dispositif à plusieurs compartiments ou « Kit » de teinture, **caractérisé par le fait qu'**il comporte un premier compartiment renfermant la composition (A) telle que définie à l'une des revendications 20 ou 21 et un second compartiment renfermant la composition (B) telle que définie à l'une des revendications 20 ou 21.
